# EUROPEAN PATENT APPLICATION

(11) **EP 2 295 977 A1**
(43) Date of publication of application: **16.03.2011**
(21) Application number: 09179125.1
(22) Date of filing: 14.12.2009
(51) Int. Cl.: G01N 33/574

(54) **Novel tumor markers**

(30) Priority: 03.09.2009 EP 09169424
(71) Applicant: Koninklijke Philips Electronics N.V., 5621 BA Eindhoven (NL)
(72) Inventor: The designation of the inventor has not yet been filed
(74) Representative: Jungen, Carmen

(57) **Abstract**

The present invention relates to a tumor marker or group of tumor markers associated with the progression of a cancer disease from a less progressed stage to a more progressed stage, wherein the expression of the tumor markers is modified when comparing the expression in the less progressed stage and in the more progressed stage. The present invention further relates to a composition for diagnosing, detecting, graduating, monitoring or prognosticating a cancer disease associated with a progression from a less progressed cancer stage to a more progressed cancer stage comprising affinity ligands for the expression products of the tumor markers, to corresponding methods, and to the use of said tumor markers for detecting, diagnosing, graduating, monitoring or prognosticating a cancer disease associated with a progression from a less progressed cancer stage to a more progressed cancer stage. The present invention further relates to a corresponding immunoassay as well as to a pharmaceutical composition based on the inhibition of the expression of said tumor markers.

## Description

### FIELD OF THE INVENTION

The present invention relates to a tumor marker or group of tumor markers associated with the progression of a cancer disease from a less progressed stage to a more progressed stage, wherein the expression of the tumor markers is modified when comparing the expression in the less progressed stage and in the more progressed stage. The present invention further relates to a composition for diagnosing, detecting, graduating, monitoring or prognosticating a cancer disease associated with a progression from a less progressed cancer stage to a more progressed cancer stage comprising affinity ligands for the expression products of the tumor markers, to corresponding methods, and to the use of said tumor markers for detecting, diagnosing, graduating, monitoring or prognosticating a cancer disease associated with a progression from a less progressed cancer stage to a more progressed cancer stage. The present invention further relates to a corresponding immunoassay as well as to a pharmaceutical composition based on the inhibition and/or activation of the expression of said tumor markers.

### BACKGROUND OF THE INVENTION

Cancer is a class of diseases in which a group of cells display uncontrolled growth, invasion and sometimes metastasis. These three malignant properties of cancers differentiate them from benign tumors, which are self-limited and do not invade or metastasize. Among men, the three most commonly diagnosed cancers are prostate, lung and colorectal cancer in developed countries. Particularly prostate cancer is the most common malignancy in European males. In 2002 in Europe, an estimated 225,000 men were newly diagnosed with prostate cancer and about 83,000 died from this disease.

Several different proteins or peptides have been associated with cancer development. For instance, phosphodiesterase PDE7 has been shown to be linked to chronic lymphocytic leukemia. Yet, for many cancer types or cancer progression forms there is no adequate marker molecule available.

Prostate cancer, for example, is traditionally diagnosed via the serum level of prostate-specific antigen (PSA). However, PSA is not prostate cancer-specific and can be raised due to other circumstances, leading to a large number of false-positives (cancer is not found in around 70% of men with raised PSA levels who undergo biopsy). Furthermore, there will be an unpredictable number of false-negatives who later develop prostate cancer in the presence of a "normal" PSA test.

Therefore, there is a need for the provision of a new and effective, alternative diagnosis perspective for the detection, monitoring and prognostication of cancer, in particular of prostate cancer. There is in particular a need for tumor markers that can increase the diagnostic specificity and differentiate between harmless and aggressive neoplastic diseases.

### SUMMARY OF THE INVENTION

The present invention addresses this need and provides means and methods which allow the diagnosis and detection of cancer, in particular prostate cancer.

The above objective is accomplished by the identification of novel tumor markers from samples of cancer patients. Using tissue or bodily fluid samples of patients representing different progression stages of cancer novel tumor markers could be identified which were shown to be differentially expressed, depending on the stage of progression of cancer. In particular, it was demonstrated by the present inventors that the novel tumor markers are significantly or up-regulated when comparing samples from patients having a less progressed stage of cancer to samples from patients having a more progressed stage of the disease. Therefore, these markers are considered as markers for cancer prediction and a decision tool for the stratification of certain cancer surveillance regimes, as well as for the prognosis and monitoring of cancer progression. Diagnostic methods and uses based on the tumor markers of the present invention can thus advantageously be employed for (i) detecting and diagnosing cancer forms, in particular prostate cancer forms, (ii) prognosticating cancer forms, in particular prostate cancer forms, (iii) monitoring of cancer progression towards more progressed cancer stages, in particular of prostate cancer, and (iv) distinguishing between less and more progressed cancer forms, in particular less and more progressed prostate cancer forms ,and most advantageously less progressed prostate cancer forms wherein the prostate cancer is of stage ≤ T2 (UICC 2002 classification) and Gleason score < 6 and more progressed prostate cancer forms wherein the prostate cancer is of stage >T2 (UICC 2002 classification) and Gleason score ≥7. Finally, pharmaceutical compositions based on these tumor markers will provide novel therapeutic avenues in the treatment of cancer, in particular prostate cancer.

The present invention, thus relates in a first aspect to a tumor marker or group of tumor markers associated with the progression of a cancer disease from a less progressed stage to a more progressed stage, wherein the expression of the tumor marker or group of tumor markers is modified when comparing the expression of the tumor marker or group of tumor markers in the less progressed stage to the expression in the more progressed stage, wherein said tumor marker or group of tumor markers comprises at least one tumor marker selected from Table 1.

In a preferred embodiment of the present invention the expression of said marker(s) is increased (up-regulated) when comparing the expression in the more progressed stage to the expression in the less progressed stage, as indicated in section I) of Table 1.

In yet another preferred embodiment of the present invention said group of tumor markers comprises at least one marker with a decreased expression in said more progressed stage and at least one marker with an increased expression in said more progressed stage.

In a further preferred embodiment of the present invention said group of tumor markers comprises at least 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200 or all of the tumor markers of Table 1.

In another preferred embodiment of the present invention the p-value of the expression modification of said group of tumor markers is 0.014 or lower.

In a further embodiment of the present invention the group of tumor markers comprises at least 5 tumor markers corresponding to tumor marker #1 to #5 of Table 1, at least 10 tumor markers corresponding to tumor marker #1 to #10 of Table 1, at least 15 tumor markers corresponding to tumor marker #1 to #15 of Table 1, at least 20 tumor markers corresponding to tumor marker #1 to #20 of Table 1, at least 25 tumor markers corresponding to tumor marker #1 to #25 of Table 1, at least 30 tumor markers corresponding to tumor marker #1 to #30 of Table 1, at least 35 tumor markers corresponding to tumor marker #1 to #35 of Table 1, at least 40 tumor markers corresponding to tumor marker #1 to #40 of Table 1, at least 45 tumor markers corresponding to tumor marker #1 to #45 of Table 1 or at least 50 tumor markers corresponding to tumor marker #1 to #50 of Table 1.

In a further aspect the present invention relates to a composition for diagnosing, detecting, graduating, monitoring or prognosticating a cancer disease associated with a progression from a less progressed cancer stage to a more progressed cancer stage, or the progression from a less progressed cancer stage to a more progressed cancer stage, comprising a nucleic acid affinity ligand and/or a peptide affinity ligand for the expression product(s) or protein(s) of said tumor marker or group of tumor markers as defined above.

In a preferred embodiment of the present invention said peptide affinity ligand is a patient serum autoantibody.

In a preferred embodiment of the present invention said nucleic acid affinity ligand or peptide affinity ligand is modified to function as an imaging contrast agent.

In yet another aspect the present invention relates to a method for detecting, diagnosing, monitoring or prognosticating a cancer disease associated with a progression from a less progressed cancer stage to a more progressed cancer stage, or the progression from a less progressed cancer stage to a more progressed cancer stage comprising at least the step of determining the level of a tumor marker or group of tumor markers as defined above, in a sample.

In a preferred embodiment the determining step of said method is accomplished by the measurement of nucleic acid or protein level(s) or by the determination of the biological activity of the tumor marker or group of tumor markers as defined above.

In another preferred embodiment of the present invention said method comprises the additional step of comparing the measured nucleic acid or protein level(s) or the measured biological activity to a control level, wherein said control level is the expression level of the tumor marker or the group of tumor markers in one or more samples of a less progressed stage of the same cancer.

In yet another preferred embodiment of the present said method is a method of graduating cancer, comprising the steps of:
(a) determining the level of a tumor marker or group of tumor markers as defined above in a sample by the measurement of nucleic acid or protein level(s) or by the determination of the biological activity of said tumor marker or group of tumor markers, and
(b) comparing the measured nucleic acid or protein level(s) or the measured biological activity to a control level, wherein said control level is the expression level of the tumor marker or the group of tumor markers in one or more samples of a less progressed stage of the same cancer; and
(c) deciding on the stage or developmental status of cancer based on the results obtained in step (b).

In a further aspect the present invention relates to the use of said tumor marker or a group of tumor markers as defined above as a marker for detecting, diagnosing, graduating, monitoring or prognosticating a cancer disease associated with a progression from a less progressed cancer stage to a more progressed cancer stage, or the progression from a less progressed cancer stage to a more progressed cancer stage.

In yet another aspect the present invention relates to an immunoassay for detecting, diagnosing, graduating, monitoring or prognosticating a cancer disease associated with a progression from a less progressed cancer stage to a more progressed cancer stage, or for detecting, diagnosing, monitoring or prognosticating the progression from a less progressed cancer stage to a more progressed cancer stage comprising at least the steps of
(a) testing in a sample obtained from an individual for the expression of a tumor marker or a group of tumor markers as defined above;
(b) testing in a control sample for the expression of the same tumor marker or group of tumor markers as in (a);
(c) determining the difference in expression of the tumor marker or group of tumor markers of steps (a) and (b); and
(d) deciding on the presence, stage or risk of recurrence of cancer or the progression of cancer based on the results obtained in step (c),
wherein said testing steps are based on the use of an antibody specifically binding (a) protein(s) of a tumor marker or group of tumor markers as defined above.

In an additional aspect the present invention relates to a pharmaceutical composition for the treatment or prevention of a cancer disease associated with a progression from a less progressed cancer stage to a more progressed cancer stage, wherein said cancer disease implies the increased (up-regulated) expression of a tumor marker or group of tumor markers as defined above, comprising at least one element selected from the group of:
(b) a compound directly inhibiting the activity of a tumor marker as defined above, preferably an antagonist of said tumor marker enzymatic activity;
(c) a compound indirectly inhibiting the activity of a tumor marker as defined above;
(d) a dominant negative form of a protein of a tumor marker as defined above or a biologically active equivalent thereof;
(e) a nucleic acid encoding and expressing a dominant negative form of a protein of a tumor marker as defined above;
(f) a miRNA specific for a tumor marker as defined above;
(g) an antisense molecule of a tumor marker as defined above;
(h) a siRNA specific for a tumor marker as defined above;
(i) an aptamer specific for the expression product of a tumor marker as defined above or for the protein of a tumor marker as defined above;
(j) a small molecule or peptidomimetic capable of specifically binding to the protein of a tumor marker as defined above; and
(k) an antibody specific for the protein of a tumor marker as defined above and/or an antibody variant specific for the protein of a tumor marker as defined above.

In another aspect the present invention relates to a pharmaceutical composition for the treatment or prevention of a cancer disease associated with a progression from a less progressed cancer stage to a more progressed cancer stage, wherein said cancer disease implies the decreased (downregulated) expression of a tumor marker or group of tumor markers as defined above, comprising at least one element selected from the group of:
(a) a compound directly stimulating or modulating the activity of a tumor marker as defined above, preferably an agonist of said tumor marker enzymatic activity;
(b) a compound indirectly stimulating or modulating the activity of a tumor marker as defined above;
(c) a protein of a tumor marker as defined above or a biologically active equivalent thereof;
(d) a nucleic acid encoding and expressing a protein of a tumor marker as defined above; and
(e) a miRNA inhibitor specific for a miRNA of a tumor marker as defined above.

An especially preferred embodiment of the present invention relates to the abovementioned tumor marker or group of tumor markers, the abovementioned compositions, the abovementioned methods, the abovementioned use, the abovementioned immunoassay, or the abovementioned pharmaceutical compositions, wherein said cancer is prostate cancer.

In a particularly preferred embodiment of the present invention said less progressed stage of prostate cancer as mentioned in the context of the above defined tumor marker or group of tumor markers, the above defined compositions, the above defined methods, the above defined uses, the above defined immunoassays, or the above defined pharmaceutical compositions is of stage ≤ T2 (UICC 2002 classification), Gleason score ≤6, and said more progressed stage of prostate cancer is of stage >T2 (UICC 2002 classification), Gleason score ≥6.

### BRIEF DESCRIPTION OF THE DRAWINGS

- Fig. 1: depicts a list of clinical samples (and corresponding clinical groups) used for the experiments described in the present application. The group no in the following Figurs refer to the group numbers in Fig. 1.
- Fig. 2: shows the statistical distribution of sample collection dates (A) and age distribution (B) of clinical samples.
- Fig. 3: indicates the statistical distribution of PSA values of the clinical samples with maximally 100 ng/ml.
- Fig. 4: indicates the statistical distribution of prostate volumes (A) or total size of cancer tissue in the biopsy core (B) of the clinical samples.
- Fig. 5: shows the statistical distribution of total size of non-cancerous tissue in the biopsy core (A) or primary Gleason score groups 3 and 4 (B) and secondary Gleason score groups 3 and 4 (C) of the clinical samples.
- Fig. 6: shows a corresponding Bradford standard curve using bovine serum albumin (BSA) separation of peptides.
- Fig. 7: (A-N) gives an illustrative overview over the AUC under a ROC of tumor markers according to the present invention for discrimination between clinical insignificant from significant prostate cancer.

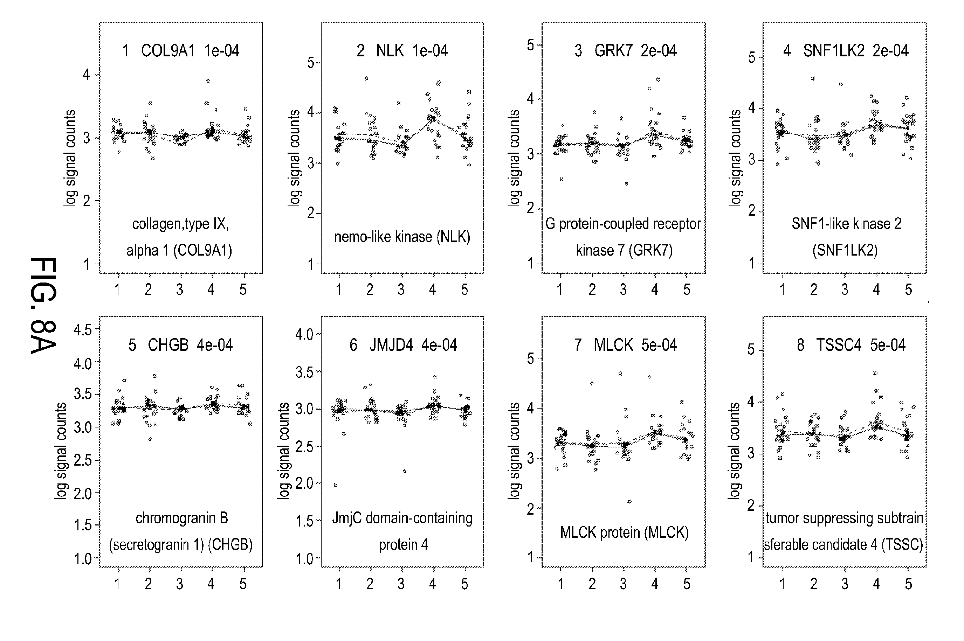

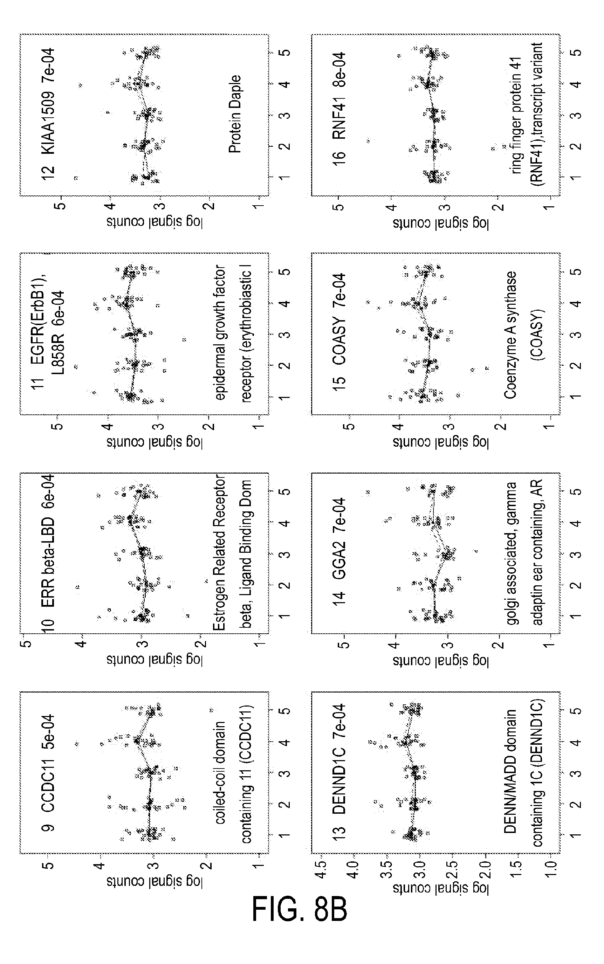

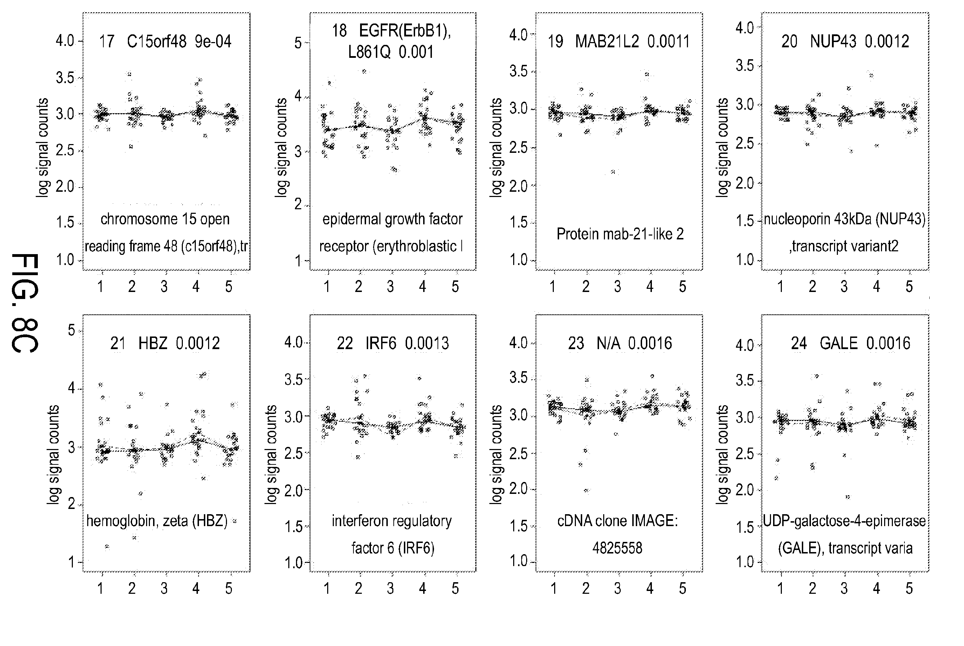

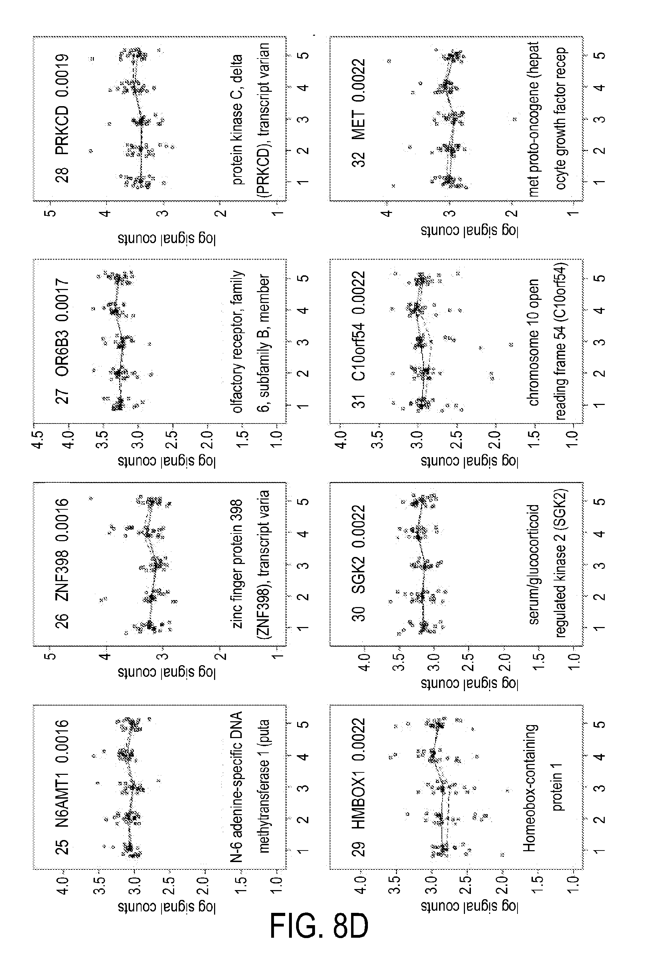

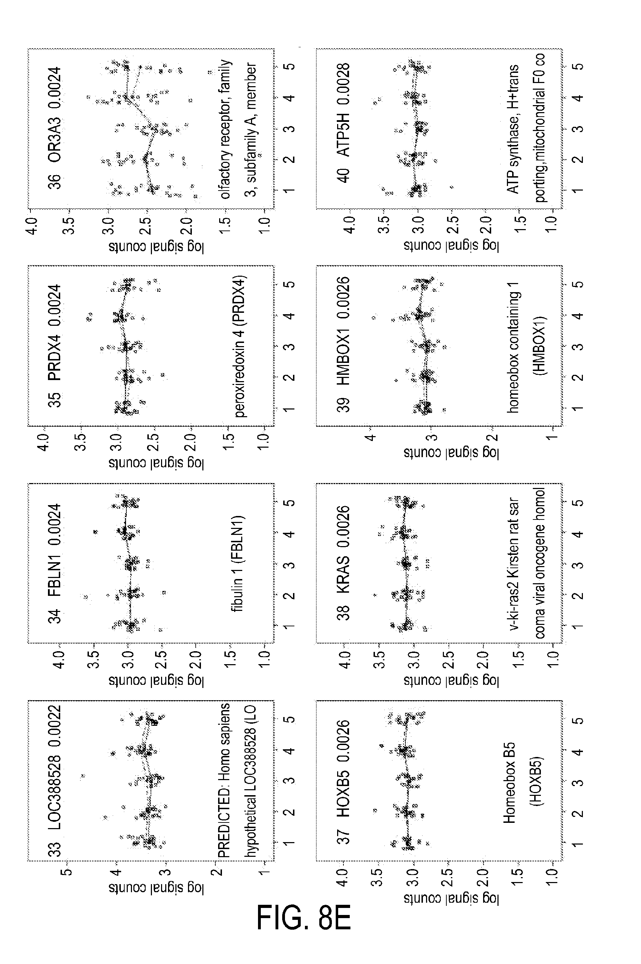

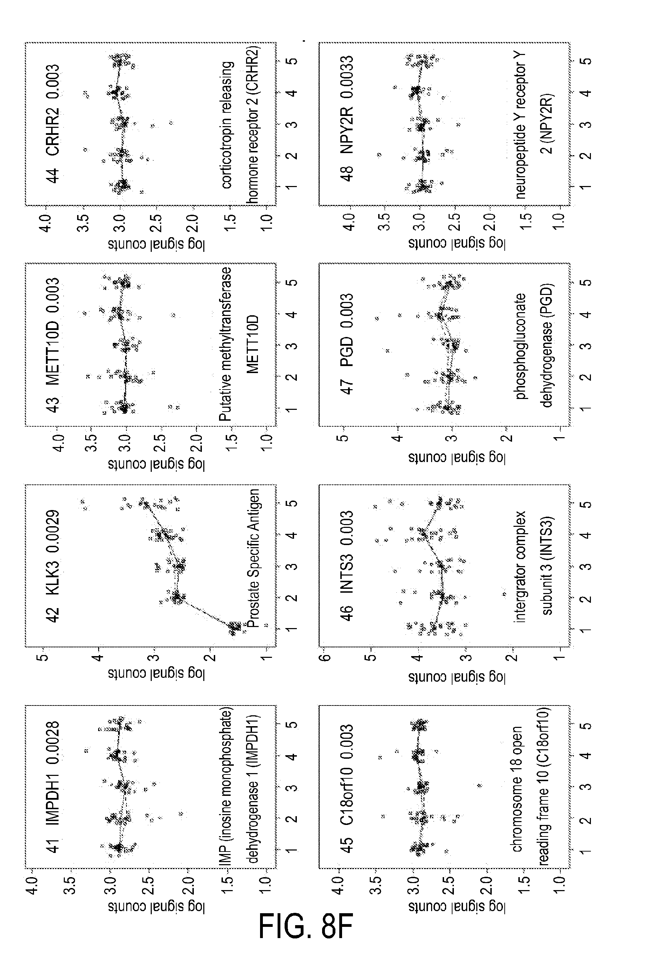

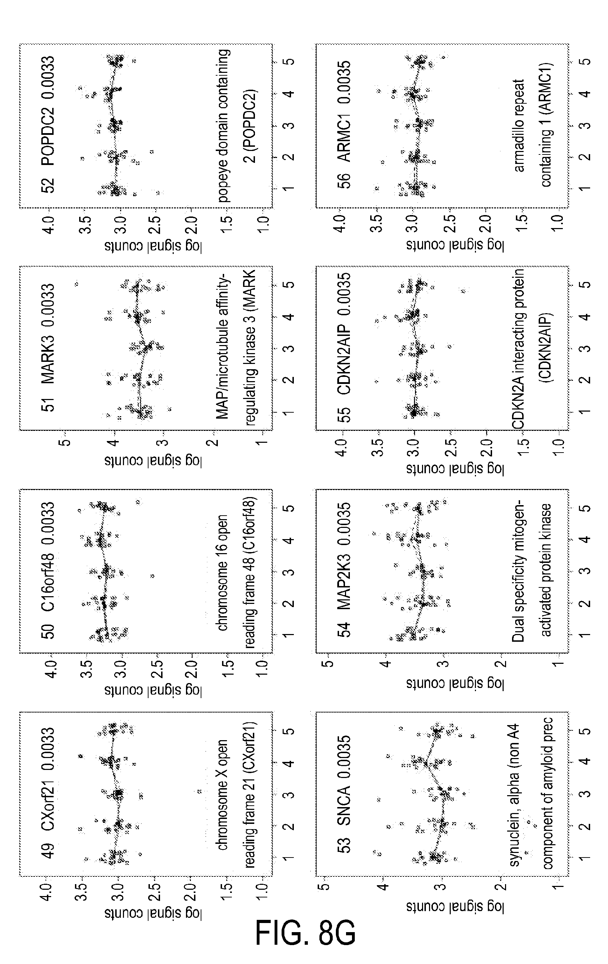

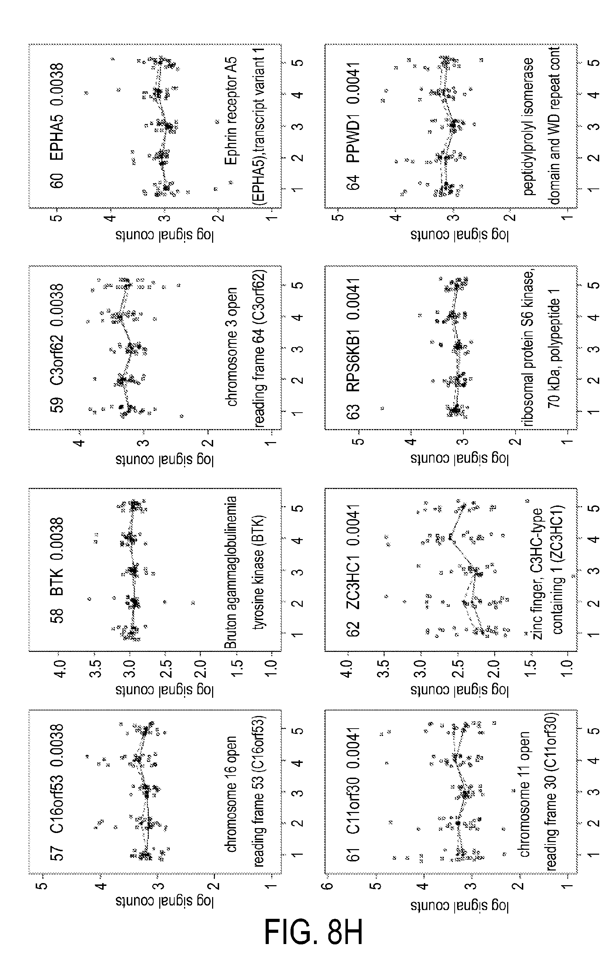

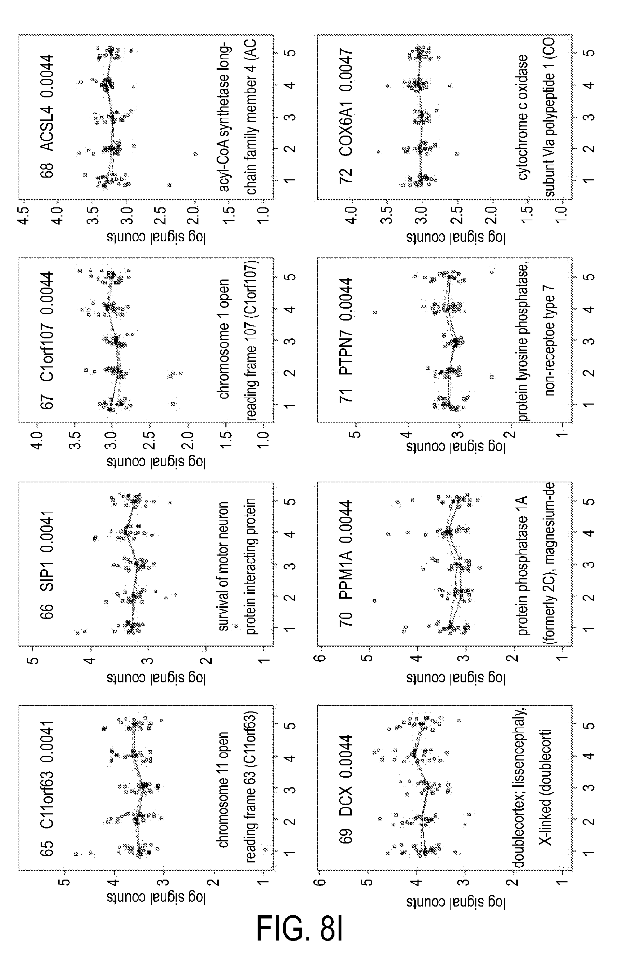

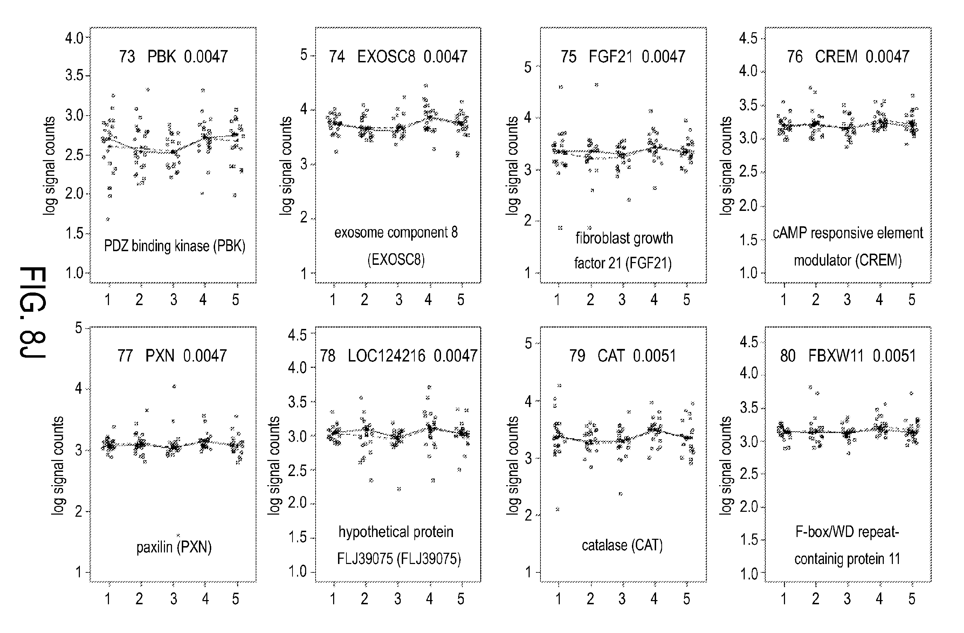

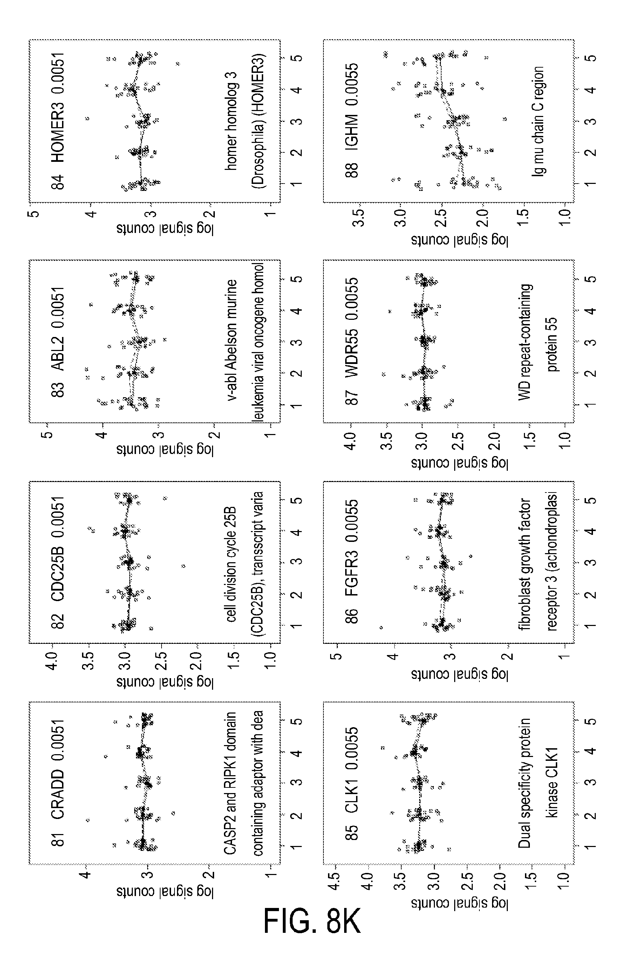

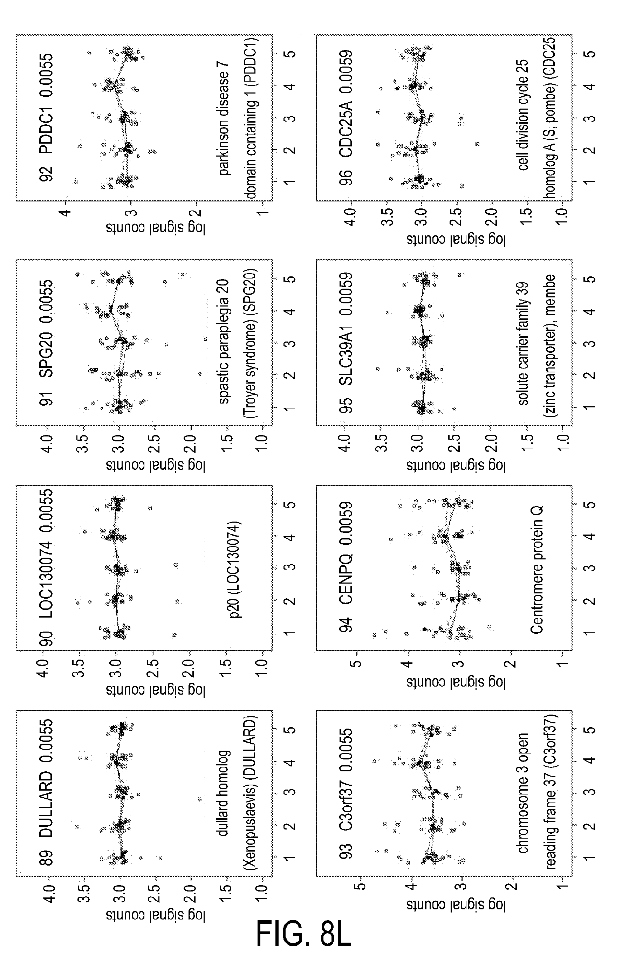

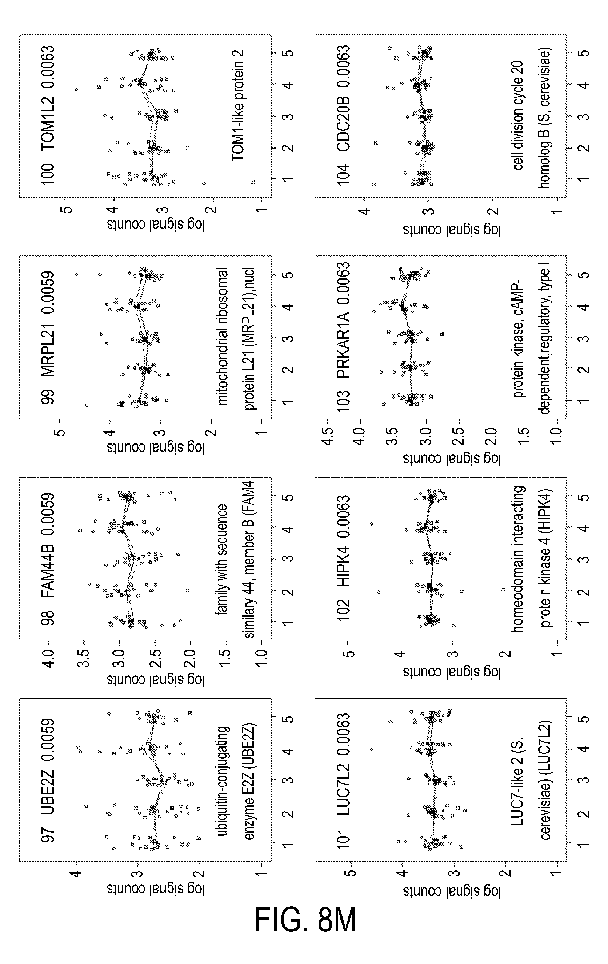

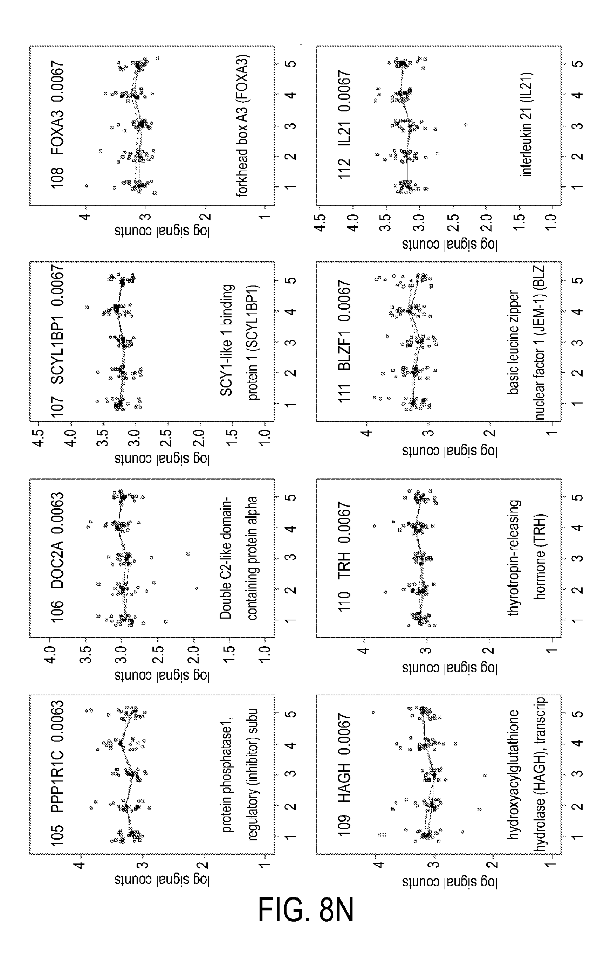

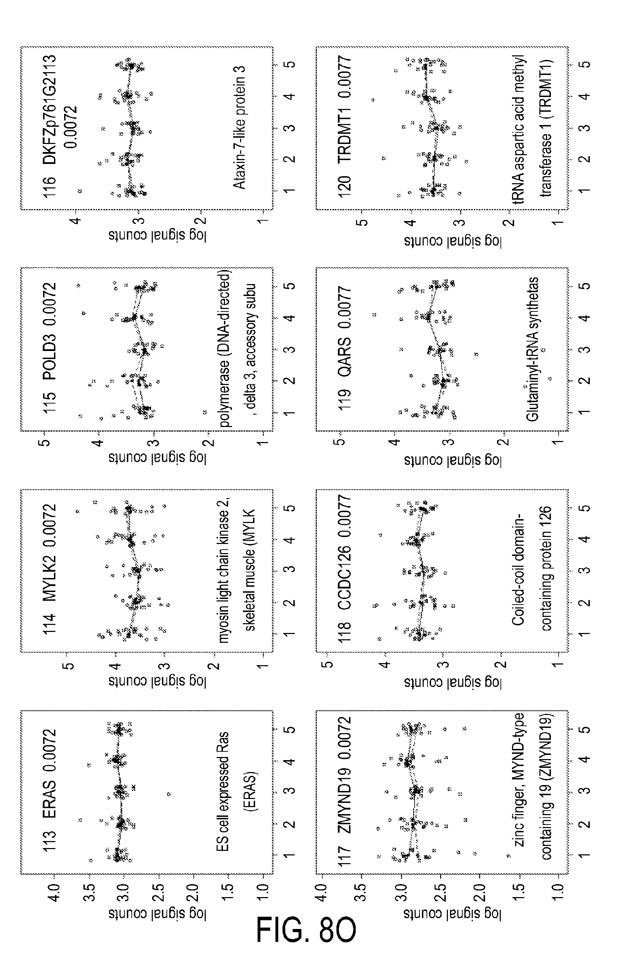

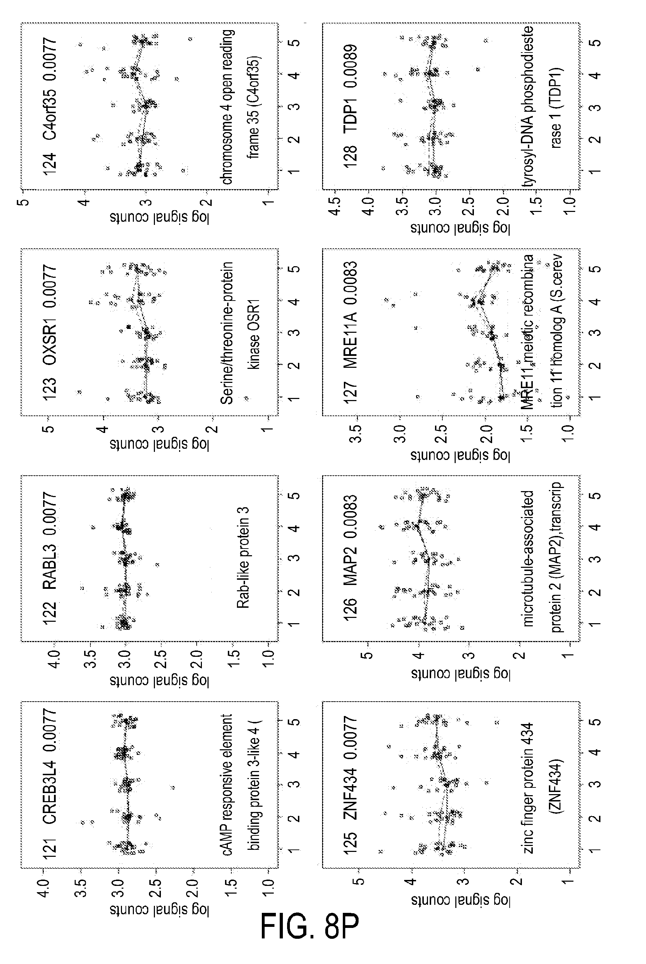

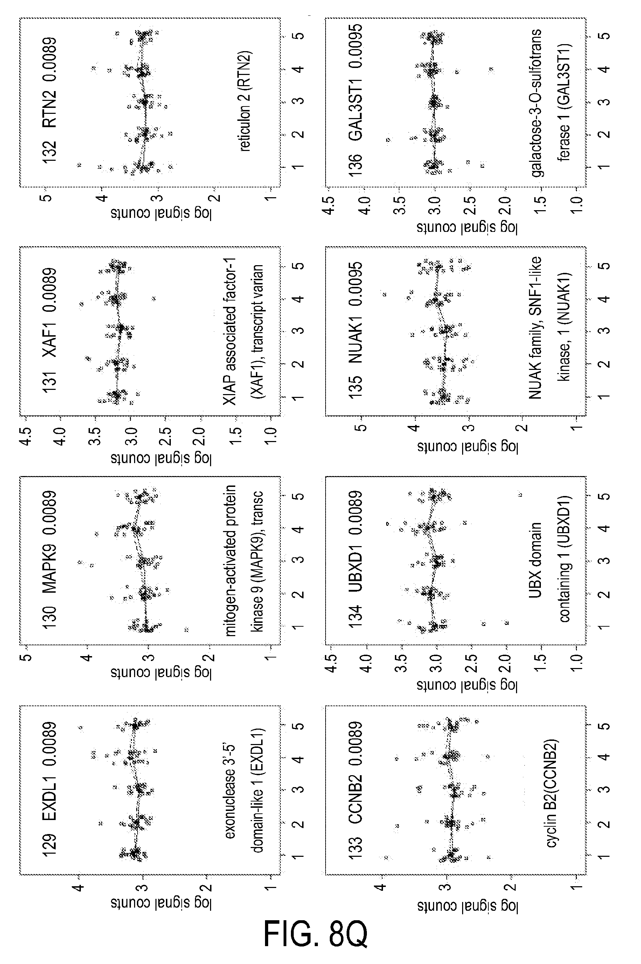

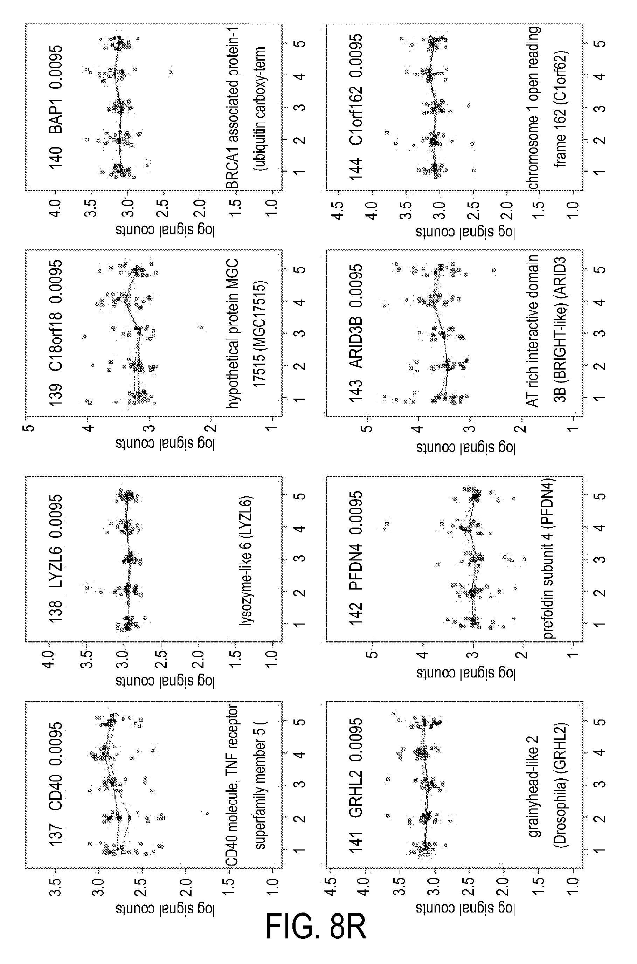

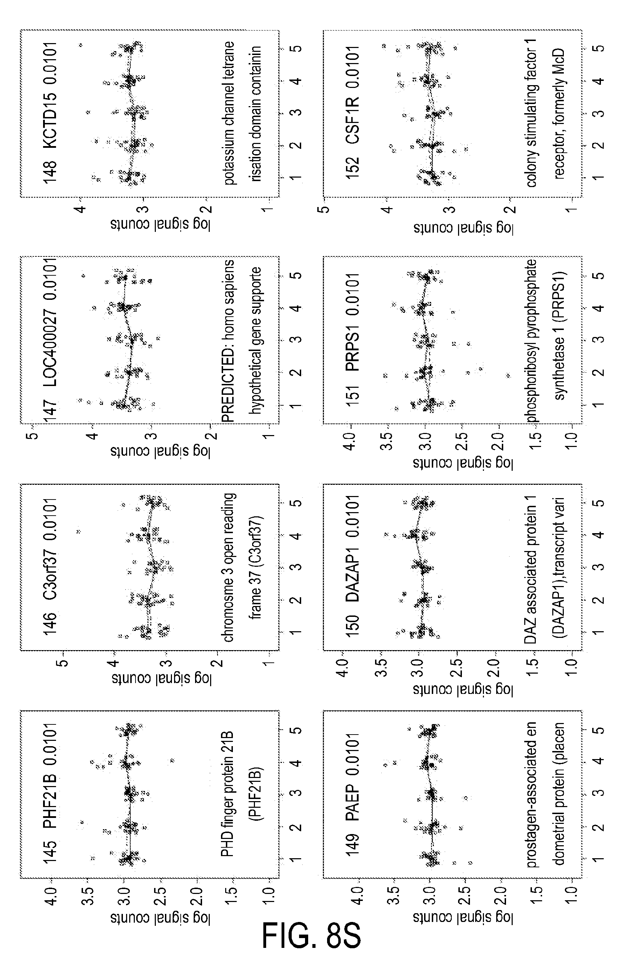

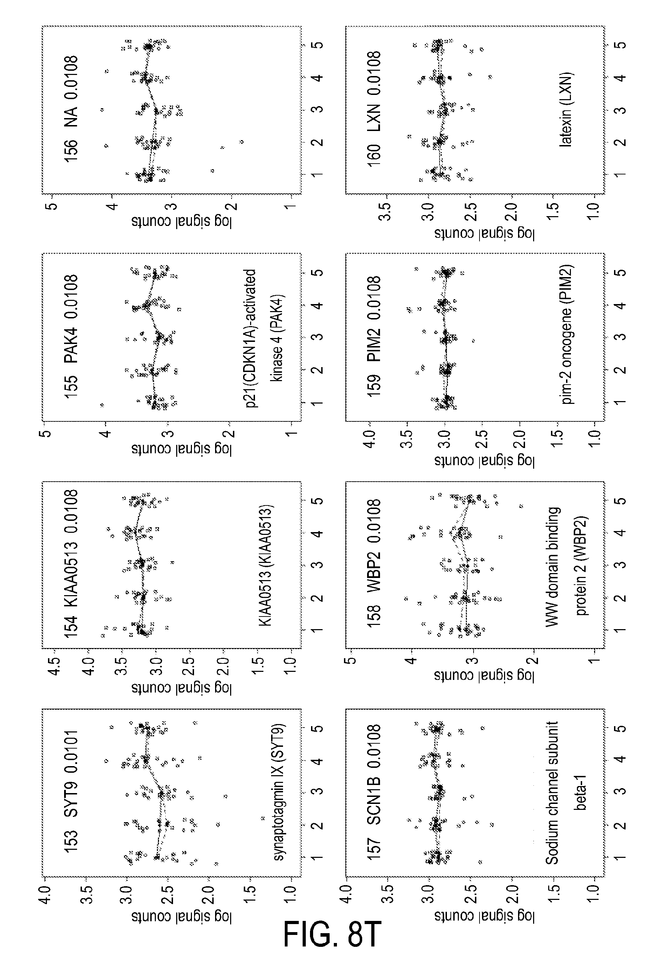

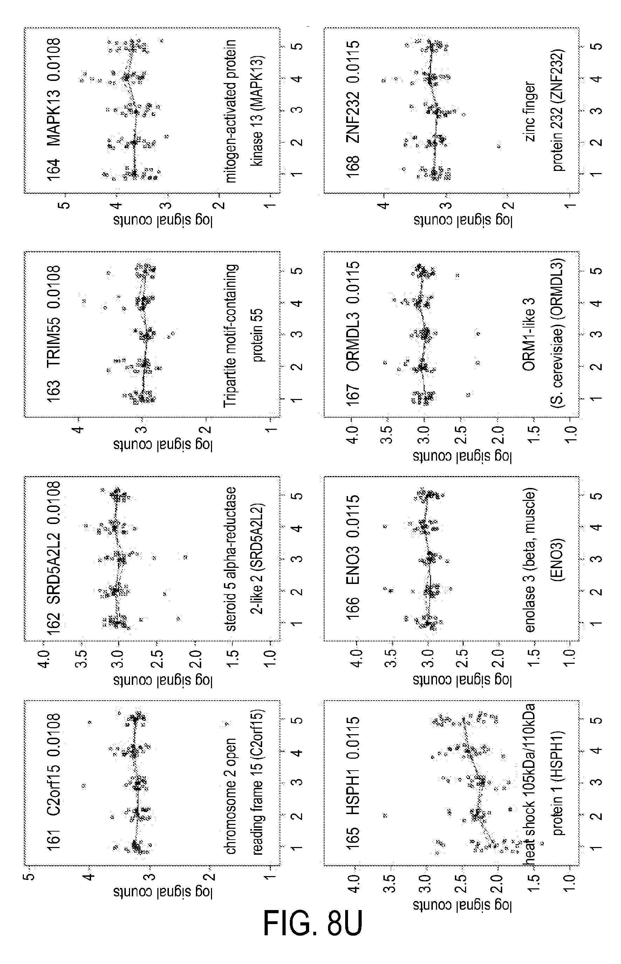

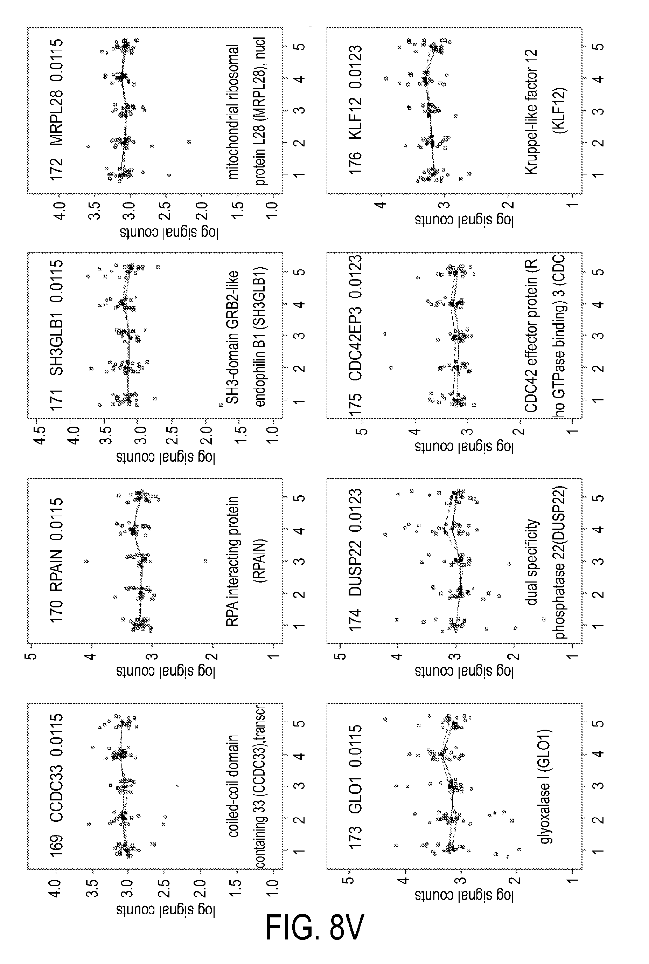

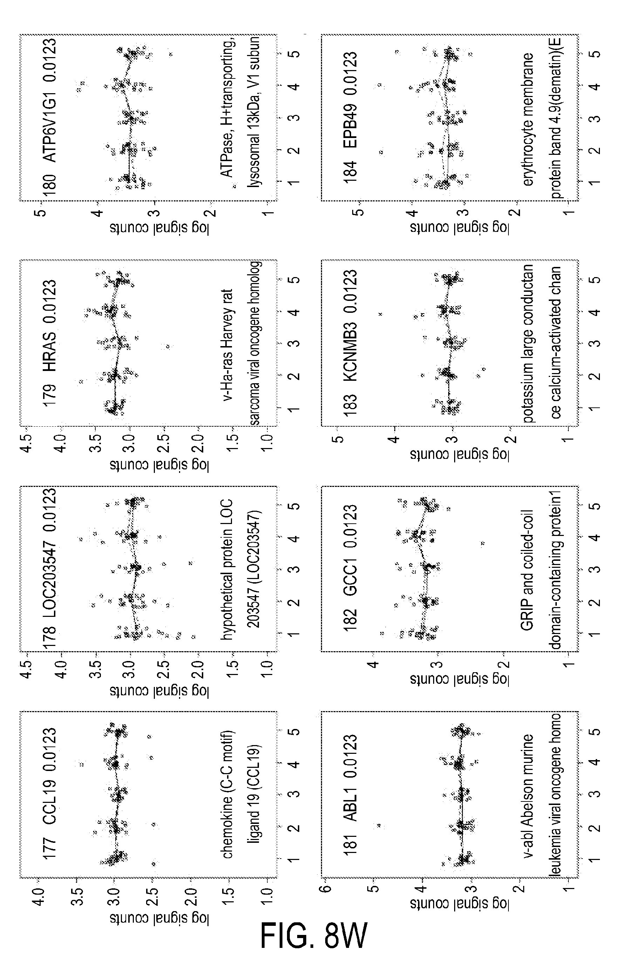

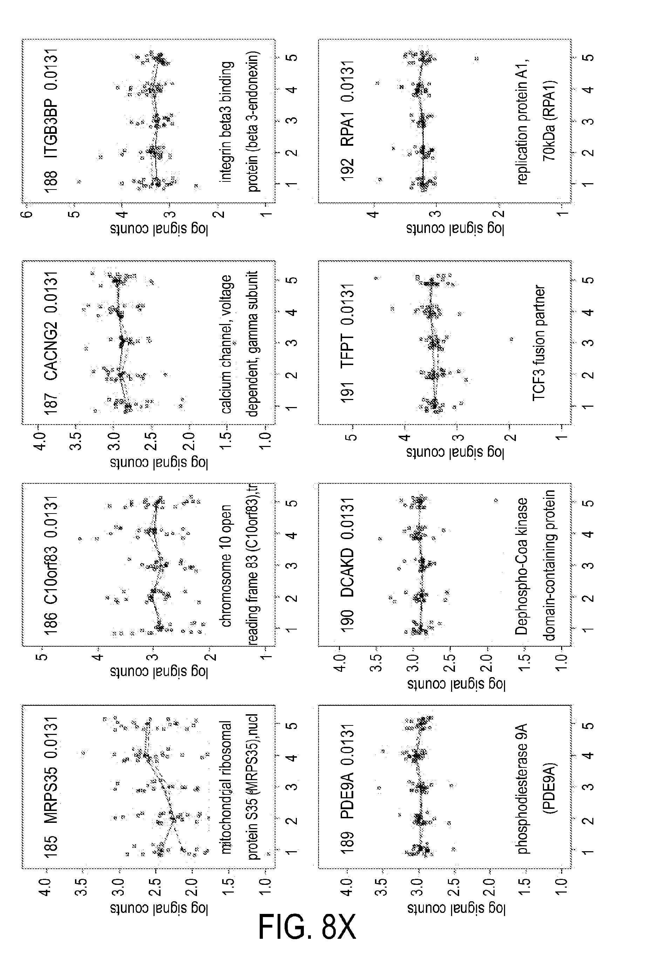

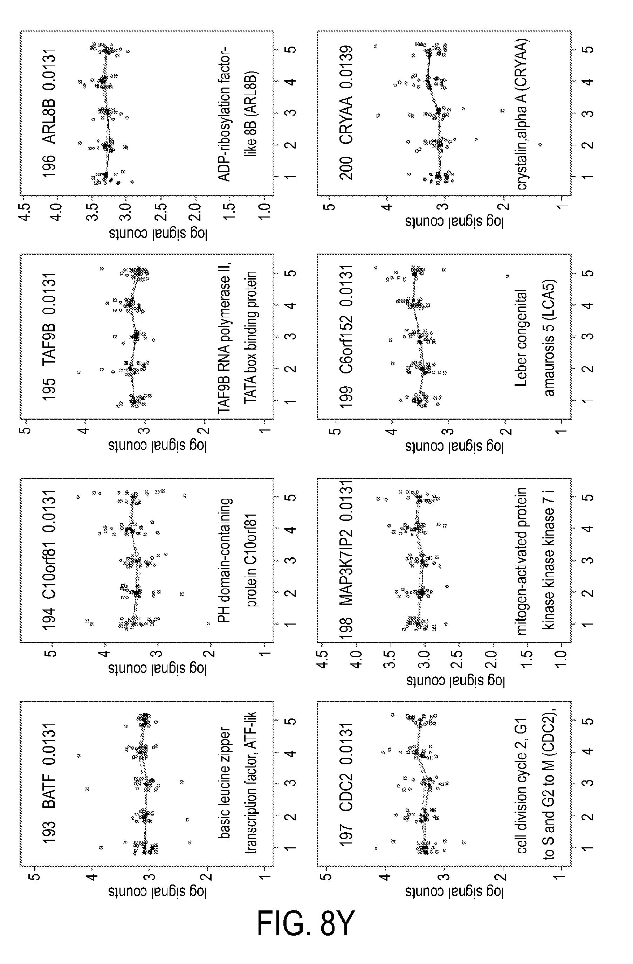

- Fig. 8: (A-X) gives an illustrative overview over the expression values of tumor markers according to the present invention over the five clinical groups studied (see Fig 1 for details).

### DETAILED DESCRIPTION OF EMBODIMENTS

It is shown that tumor markers as depicted in Table 1 are differentially expressed, depending on the stage of progression of cancer when testing samples of patients. In particular, it was demonstrated by the present inventors that tumor markers are statistically significantly up-regulated when comparing samples from a less progressed stage to a more progressed stage of a cancer disease. Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" is considered to be a preferred embodiment of the term "comprising of".

If hereinafter a group is defined to comprise at least a certain number of embodiments, this is meant to also encompass a group which preferably consists of these embodiments only.

Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)" etc. relate to steps of a method or use there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

It is to be understood that this invention is not limited to the particular methodology, protocols, reagents etc. described herein as these may vary. It is also to be understood that the terminology used herein is for the purpose of describing particular embodiments only, and is not intended to limit the scope of the present invention that will be limited only by the appended claims.

Unless defined otherwise, all technical and scientific terms used herein have the same meanings as commonly understood by one of ordinary skill in the art.

As has been set out above, a first aspect of the present invention pertains to a tumor marker or group of tumor markers associated with the progression of a cancer disease from a less progressed stage to a more progressed stage, wherein the expression of the tumor marker or group of tumor markers is modified when comparing the expression of the tumor marker or group of tumor markers in the less progressed stage to the expression in the more progressed stage, wherein said tumor marker or group of tumor markers comprises at least one tumor marker selected from the following Table 1.

**Table 1**

| **A) Marker #** | **B) Uniprot Accessio n Code** | **C) Marker Function** | **D) SEQ ID NO. nucleotide sequence** | **E) SEQ ID NO. amino acid sequence** | **F) Entrez Gene ID** | **G) Protein Sequence (Immune Response Fragment)** | **H) P value of expression difference between groups 3 & 4** |
|---|---|---|---|---|---|---|---|
| **1** | CO9A1_ HUMAN | Collagen, type IX, alpha 1 (COL9A1) | **1** | **201** | 1297 | **401** | 0.00010065 |
| **2** | NLK_HU MAN | Nemo-like kinase (NLK) | **2** | **202** | 51701 | **402** | 0.00012478 |
| **3** | GRK7_H UMAN | G protein-coupled receptor kinase 7 | **3** | **203** | 131890 | **403** | 0.00023222 |
| **4** | SIK2_HU MAN | SNF1-like kinase 2 (SIK2) | **4** | **204** | 23235 | **404** | 0.00023222 |
| **5** | SCG1_H UMAN | Chromogranin B (secretogranin 1) | **5** | **205** | 1114 | **405** | 0.0004179 |
| **6** | JMJD4_H UMAN | JmjC domain-containing protein 4 | **6** | **206** | 65094 | **406** | 0.0004179 |
| **7** | MYLK_ HUMAN | MLCK protein | **7** | **207** | 4638 | **407** | 0.00045946 |
| **8** | TSSC4_H UMAN | Tumor suppressing subtransferable candidate 4 | **8** | **208** | 10078 | **408** | 0.00050472 |
| **9** | CCD11_ HUMAN | Coiled-coil domain containing 11 | **9** | **209** | 220136 | **409** | 0.00050472 |
| **10** | ERR2_H UMAN | Estrogen Related Receptor beta, Ligand Binding Domain | **10** | **210** | 2103 | **410** | 0.00060748 |
| **11** | EGFR_H UMAN | Epidermal growth factor receptor (L858R) | **11** | **211** | 1956 | **411** | 0.00060748 |
| **12** | DAPLE_ HUMAN | Protein Daple | **12** | **212** | 440193 | **412** | 0.00066561 |
| **13** | DEN1C_ HUMAN | DENN/MADD domain containing 1C | **13** | **213** | 79958 | **413** | 0.00066561 |
| **14** | GGA2_H UMAN | Golgi associated, gamma adaptin ear containing, ARF binding protein 2 | **14** | **214** | 23062 | **414** | 0.00066561 |
| **15** | COASY_ HUMAN | Coenzyme A synthase | **15** | **215** | 80347 | **415** | 0.0007287 |
| **16** | RNF41_ HUMAN | Ring finger protein 41 | **16** | **216** | 10193 | **416** | 0.00079711 |
| **17** | NMES1_ HUMAN | Chromosome 15 open reading frame 48 | **17** | **217** | 84419 | **417** | 0.00087123 |
| **18** | EGFR_H UMAN | Epidermal growth factor receptor (L861Q) | **18** | **218** | 1956 | **418** | 0.00095147 |
| **19** | MB212_ HUMAN | Protein mab-21-like 2 | **19** | **219** | 10586 | **419** | 0.00113207 |
| **20** | NUP43_ HUMAN | nucleoporin 43kDa | **20** | **220** | 348995 | **420** | 0.00123337 |
| **21** | HBAZ_H UMAN | Hemoglobin, zeta | **21** | **221** | 3050 | **421** | 0.00123337 |
| **22** | IRF6_HU MAN | interferon regulatory factor 6 | **22** | **222** | 3664 | **422** | 0.00134269 |
| **23** | N/A | cDNA clone IMAGE:4825558 | **23** | **223** | 100287114 | **423** | 0.00158756 |
| **24** | GALE_H UMAN | UDP-galactose-4-epimerase | **24** | **224** | 2582 | **424** | 0.00158756 |
| **25** | N6MT1_ HUMAN | N-6 adenine-specific DNA methyltransferase 1 | **25** | **225** | 29104 | **425** | 0.00158756 |
| **26** | ZN398_H UMAN | Zinc finger protein 398 | **26** | **226** | 57541 | **426** | 0.00158756 |
| **27** | OR6B3_ HUMAN | Olfactory receptor, family 6, subfamily B, member 3 | **27** | **227** | 150681 | **427** | 0.00172428 |
| **28** | KPCD_H UMAN | Protein kinase C, delta | **28** | **228** | 5580 | **428** | 0.00187136 |
| **29** | HMBX1_ HUMAN | Homeobox-containing protein 1 | **29** | **229** | 79618 | **429** | 0.00219926 |
| **30** | SGK2_H UMAN | serum/glucocorticoid regulated kinase 2 | **30** | **230** | 10110 | **430** | 0.00219926 |
| **31** | GI24_HU MAN | Chromosome 10 open reading frame 54 | **31** | **231** | 64115 | **431** | 0.00219926 |
| **32** | MET_HU MAN | met proto-oncogene | **32** | **232** | 4233 | **432** | 0.00219926 |
| **33** | N/A | PREDICTED: Homo sapiens hypothetical LOC388528 | **33** | **233** | 388528 | **433** | 0.00219926 |
| **34** | FBLN1_ HUMAN | fibulin 1 | **34** | **234** | 2192 | **434** | 0.00238152 |
| **35** | PRDX4_ HUMAN | peroxiredoxin 4 | **35** | **235** | 10549 | **435** | 0.00238152 |
| **36** | OR3A3_ HUMAN | Olfactory receptor, family 3, subfamily A, member 3 | **36** | **236** | 8392 | **436** | 0.00238152 |
| **37** | HXB5_H UMAN | Homeobox B5 | **37** | **237** | 3215 | **437** | 0.00257699 |
| **38** | RASK_H UMAN | v-Ki-ras2 Kirsten rat sarcoma viral oncogene homolog | **38** | **238** | 3845 | **438** | 0.00257699 |
| **39** | HMBX1_ HUMAN | Homeobox containing 1 | **39** | **239** | 79618 | **439** | 0.00257699 |
| **40** | ATP5H_ HUMAN | ATP synthase, H+ transporting, mitochondrial F0 complex, subunit d | **40** | **240** | 10476 | **440** | 0.00278648 |
| **41** | IMDH1_ HUMAN | IMP (inosine monophosphate) dehydrogenase 1 | **41** | **241** | 3614 | **441** | 0.00278648 |
| **42** | KLK3_H UMAN | Prostate Specific Antigen | **42** | **242** | 354 | **442** | 0.00286423 |
| **43** | MET10_ HUMAN | Putative methyltransferase | **43** | **243** | 79066 | **443** | 0.00301082 |
| **44** | CRFR2_ HUMAN | corticotropin releasing hormone receptor 2 | **44** | **244** | 1395 | **444** | 0.00301082 |
| **45** | TPGS2_ HUMAN | Chromosome 18 open reading frame 1 | **45** | **245** | 25941 | **445** | 0.00301082 |
| **46** | INT3_HU MAN | Integrator complex subunit 3 | **46** | **246** | 65123 | **446** | 0.00301082 |
| **47** | 6PGD_H UMAN | Phosphogluconate dehydrogenase | **47** | **247** | 5226 | **447** | 0.00301082 |
| **48** | NPY2R_ HUMAN | Neuropeptide Y receptor Y2 | **48** | **248** | 4887 | **448** | 0.00325089 |
| **49** | CX021_H UMAN | Chromosome X open reading frame 21 | **49** | **249** | 80231 | **449** | 0.00325089 |
| **50** | CP048_H UMAN | Chromosome 16 open reading frame 48 | **50** | **250** | 84080 | **450** | 0.00325089 |
| **51** | MARK3_ HUMAN | MAP/microtubule affinity-regulating kinase 3 | **51** | **251** | 4140 | **451** | 0.00325089 |
| **52** | POPD2_ HUMAN | Popeye domain containing 2 | **52** | **252** | 64091 | **452** | 0.00325089 |
| **53** | SYUA_H UMAN | Synuclein, alpha | **53** | **253** | 6622 | **453** | 0.0035076 |
| **54** | MP2K3_ HUMAN | Dual specificity mitogen-activated protein kinase kinase 3 | **54** | **254** | 5606 | **454** | 0.0035076 |
| **55** | CARF_H UMAN | CDKN2A interacting protein | **55** | **255** | 55602 | **455** | 0.0035076 |
| **56** | ARMC1_ HUMAN | Armadillo repeat containing 1 | **56** | **256** | 55156 | **456** | 0.0035076 |
| **57** | PA1_HU MAN | Chromosome 16 open reading frame 53 | **57** | **257** | 79447 | **457** | 0.00378192 |
| **58** | BTK_HU MAN | Bruton agammaglobulinemia tyrosine kinase | **58** | **258** | 695 | **458** | 0.00378192 |
| **59** | CC062_H UMAN | Chromosome 3 open reading frame 62 | **59** | **259** | 375341 | **459** | 0.00378192 |
| **60** | EPHA5_ HUMAN | Ephrin receptor A5 | **60** | **260** | 2044 | **460** | 0.00378192 |
| **61** | EMSY_H UMAN | Chromosome 11 open reading frame 30 | **61** | **261** | 56946 | **461** | 0.00407484 |
| **62** | NIPA_H UMAN | Zinc finger, C3HC-type containing 1 | **62** | **262** | 51530 | **462** | 0.00407484 |
| **63** | KS6B1_ HUMAN | Ribosomal protein S6 kinase, 70kDa, polypeptide 1 | **63** | **263** | 6198 | **463** | 0.00407484 |
| **64** | PPWD1_ HUMAN | Peptidylprolyl isomerase domain and WD repeat containing 1 | **64** | **264** | 23398 | **464** | 0.00407484 |
| **65** | CK063_H UMAN | Chromosome 11 open reading frame 63 | **65** | **265** | 79864 | **465** | 0.00407484 |
| **66** | GEMI2_ HUMAN | Survival of motor neuron protein interacting protein 1 | **66** | **266** | 8487 | **466** | 0.00407484 |
| **67** | DEF_HU MAN | Chromosome 1 open reading frame 107 | **67** | **267** | 27042 | **467** | 0.0043874 |
| **68** | ACSL4_ HUMAN | Acyl-CoA synthetase long-chain family member 4 | **68** | **268** | 2182 | **468** | 0.0043874 |
| **69** | DCX_HU MAN | Doublecortex; lissencephaly, X-linked (doublecortin) | **69** | **269** | 1641 | **469** | 0.0043874 |
| **70** | PPM1A_ HUMAN | Protein phosphatase 1A (formerly 2C), magnesium-dependent | **70** | **270** | 5494 | **470** | 0.0043874 |
| **71** | PTN7_H UMAN | Protein tyrosine phosphatase, non-receptor type 7 | **71** | **271** | 5778 | **471** | 0.0043874 |
| **72** | CX6A1_ HUMAN | Cytochrome c oxidase subunit VIa polypeptide 1 | **72** | **272** | 1337 | **472** | 0.00472067 |
| **73** | TOPK_H UMAN | PDZ binding kinase | **73** | **273** | 55872 | **473** | 0.00472067 |
| **74** | EXOS8_ HUMAN | Exosome component 8 | **74** | **274** | 11340 | **474** | 0.00472067 |
| **75** | FGF21_H UMAN | Fibroblast growth factor 21 | **75** | **275** | 26291 | **475** | 0.00472067 |
| **76** | CREM_H UMAN | cAMP responsive element modulator | **76** | **276** | 1390 | **476** | 0.00472067 |
| **77** | PAXI_H UMAN | Paxillin | **77** | **277** | 5829 | **477** | 0.00472067 |
| **78** | N/A | Hypothetical protein FLJ39075 | **78** | **278** | 124216 | **478** | 0.00472067 |
| **79** | CATA_H UMAN | Catalase F-box/WD | **79** | **279** | 847 | **479** | 0.00507577 |
| **80** | FBW1B_ HUMAN | repeat-containing protein 11 | **80** | **280** | 23291 | **480** | 0.00507577 |
| **81** | CRADD_ HUMAN | CASP2 and RIPK1 domain containing adaptor with death domain | **81** | **281** | 8738 | **481** | 0.00507577 |
| **82** | MPIP2_H UMAN | Cell division cycle 25B | **82** | **282** | 994 | **482** | 0.00507577 |
| **83** | ABL2_H UMAN | v-abl Abelson murine leukemia viral oncogene homolog 2 | **83** | **283** | 27 | **483** | 0.00507577 |
| **84** | HOME3_ HUMAN | Homer homolog 3 | **84** | **284** | 9454 | **484** | 0.00507577 |
| **85** | CLK1_H UMAN | Dual specificity protein kinase | **85** | **285** | 1195 | **485** | 0.00545388 |
| **86** | FGFR3_ HUMAN | Fibroblast growth factor receptor 3 | **86** | **286** | 2261 | **486** | 0.00545388 |
| **87** | WDR55_ HUMAN | WD repeat-containing protein 55 | **87** | **287** | 54853 | **487** | 0.00545388 |
| **88** | IGHM_H UMAN | Ig mu chain C region | **88** | **288** | 3507 | **488** | 0.00545388 |
| **89** | DULRD_ HUMAN | Dullard homolog | **89** | **289** | 23399 | **489** | 0.00545388 |
| **90** | N/A | p20 (LOC130074) | **90** | **290** | 130074 | **490** | 0.00545388 |
| **91** | SPG20_H UMAN | Spastic paraplegia 20 | **91** | **291** | 23111 | **491** | 0.00545388 |
| **92** | PDDC1_ HUMAN | Parkinson disease 7 domain containing 1 | **92** | **292** | 347862 | **492** | 0.00545388 |
| **93** | N/A | Chromosome 3 open reading frame 37 | **93** | **293** | 56941 | **493** | 0.00545388 |
| **94** | CENPQ_ HUMAN | Centromere protein Q | **94** | **294** | 55166 | **494** | 0.00585619 |
| **95** | S39A1_H UMAN | Solute carrier family 39 (zinc transporter) | **95** | **295** | 27173 | **495** | 0.00585619 |
| **96** | MPIP1_H UMAN | Sell division cycle 25 homolog A | **96** | **296** | 993 | **496** | 0.00585619 |
| **97** | UBE2Z_ HUMAN | Ubiquitin-conjugating enzyme E2Z | **97** | **297** | 65264 | **497** | 0.00585619 |
| **98** | BOD1_H UMAN | Family with sequence similarity 44 | **98** | **298** | 91272 | **498** | 0.00585619 |
| **99** | RM21_H UMAN | Mitochondrial ribosomal protein L21 | **99** | **299** | 219927 | **499** | 0.00585619 |
| **100** | TM1L2_ HUMAN | TOM1-like protein 2 | **100** | **300** | 146691 | **500** | 0.00628397 |
| **101** | LC7L2_H UMAN | LUC7-like 2 | **101** | **301** | 51631 | **501** | 0.00628397 |
| **102** | HIPK4_H UMAN | Homeodomain interacting protein kinase 4 | **102** | **302** | 147746 | **502** | 0.00628397 |
| **103** | KAP0_H UMAN | Protein kinase, cAMP-dependent, regulatory, | **103** | **303** | 5573 | **503** | 0.00628397 |
| **104** | CD20B_ HUMAN | type I, alpha Cell division cycle 20 homolog B | **104** | **304** | 166979 | **504** | 0.00628397 |
| **105** | PPR1C_ HUMAN | Protein phosphatase 1, regulatory (inhibitor) subunit 1C | **105** | **305** | 151242 | **505** | 0.00628397 |
| **106** | DOC2A_ HUMAN | Double C2-like domain-containing protein alpha | **106** | **306** | 8448 | **506** | 0.00628397 |
| **107** | GORAB_ HUMAN | SCY1-like 1 binding protein 1 | **107** | **307** | 92344 | **507** | 0.00673851 |
| **108** | FOXA3_ HUMAN | Forkhead box A3 | **108** | **308** | 3171 | **508** | 0.00673851 |
| **109** | GLO2_H UMAN | Hydroxyacylglutathion e hydrolase | **109** | **309** | 3029 | **509** | 0.00673851 |
| **110** | TRH_HU MAN | Thyrotropin-releasing hormone | **110** | **310** | 7200 | **510** | 0.00673851 |
| **111** | GO45_H UMAN | Basic leucine zipper nuclear factor 1 | **111** | **311** | 8548 | **511** | 0.00673851 |
| **112** | IL21_HU MAN | Interleukin 21 | **112** | **312** | 59067 | **512** | 0.00673851 |
| **113** | RASE_H UMAN | ES cell expressed Ras | **113** | **313** | 3266 | **513** | 0.00722115 |
| **114** | MYLK2_ HUMAN | Myosin light chain kinase 2, skeletal muscle | **114** | **314** | 85366 | **514** | 0.00722115 |
| **115** | DPOD3_ HUMAN | Polymerase (DNA-directed), delta 3, accessory subunit | **115** | **315** | 10714 | **515** | 0.00722115 |
| **116** | N/A | Ataxin-7-like protein 3 | **116** | **316** | 56970 | **516** | 0.00722115 |
| **117** | ZMY19_ HUMAN | Zinc finger, MYND-type containing 19 ( | **117** | **317** | 116225 | **517** | 0.00722115 |
| **11** | CC126_H UMAN | Coiled-coil domain-containing protein 126 | **11** | **318** | 90693 | **518** | 0.00773328 |
| **119** | SYQ_HU MAN | Glutaminyl-tRNA synthetase | **119** | **319** | 5859 | **519** | 0.00773328 |
| **120** | TRDMT_ HUMAN | tRNA aspartic acid methyltransferase 1 | **120** | **320** | 1787 | **520** | 0.00773328 |
| **121** | CR3L4_ HUMAN | cAMP responsive element binding protein 3-like 4 | **121** | **321** | 148327 | **521** | 0.00773328 |
| **122** | RABL3_ HUMAN | Rab-like protein 3 | **122** | **322** | 285282 | **522** | 0.00773328 |
| **123** | OXSR1_ HUMAN | Serine/threonine-protein kinase OSR1 Chromosome 4 | **123** | **323** | 9943 | **523** | 0.00773328 |
| **124** | CLPH_H UMAN | open reading frame 3 | **124** | **324** | 85438 | **524** | 0.00773328 |
| **125** | ZN434_H UMAN | Zinc finger protein 434 Microtubule-associated | **125** | **325** | 54925 | **525** | 0.00773328 |
| **126** | MAP2_H UMAN | protein 2 MRE11 meiotic | **126** | **326** | 4133 | **526** | 0.00827633 |
| **127** | MRE11_ HUMAN | recombination 11 homolog A | **127** | **327** | 4361 | **527** | 0.00827633 |
| **128** | TYDP1_ HUMAN | Tyrosyl-DNA phosphodiesterase 1 Exonuclease 3'-5' | **128** | **328** | 55775 | **528** | 0.00885178 |
| **129** | EXD1_H UMAN | domain-like 1 Mitogen-activated | **129** | **329** | 161829 | **529** | 0.00885178 |
| **130** | MK09_H UMAN | protein kinase 9 (MAPK9) XIAP associated factor- | **130** | **330** | 5601 | **530** | 0.00885178 |
| **131** | XAF1_H UMAN | 1 | **131** | **331** | 54739 | **531** | 0.00885178 |
| **132** | RTN2_H UMAN | Reticulon 2 | **132** | **332** | 6253 | **532** | 0.00885178 |
| **133** | CCNB2_ HUMAN | Cyclin B2 UBX domain | **133** | **333** | 9133 | **533** | 0.00885178 |
| **134** | UBXN6_ HUMAN | containing 1 NUAK SNF1- | **134** | **334** | 80700 | **534** | 0.00885178 |
| **135** | NUAK1_ HUMAN | family, like kinase, 1 Galactose-3-O- | **135** | **335** | 9891 | **535** | 0.00946116 |
| **136** | G3ST1_H UMAN | sulfotransferase 1 | **136** | **336** | 9514 | **536** | 0.00946116 |
| **137** | TNR5_H UMAN | CD40 molecule, TNF receptor superfamily member 5 | **137** | **337** | 958 | **537** | 0.00946116 |
| **138** | LYZL6_ HUMAN | Lysozyme-like 6 | **138** | **338** | 57151 | **538** | 0.00946116 |
| **139** | CR018_H UMAN | Hypothetical protein MGC17515 | **139** | **339** | 147525 | **539** | 0.00946116 |
| **140** | BAP1_H UMAN | BRCA1 associated protein-1 | **140** | **340** | 8314 | **540** | 0.00946116 |
| **141** | GRHL2_ HUMAN | Grainyhead-like 2 | **141** | **341** | 79977 | **540** | 0.00946116 |
| **142** | PFD4_H UMAN | Prefoldin subunit 4 | **142** | **342** | 5203 | **542** | 0.00946116 |
| **143** | ARI3B_H UMAN | AT rich interactive domain 3B | **143** | **343** | 10620 | **543** | 0.00946116 |
| **144** | CA162_H UMAN | Chromosome 1 open reading frame 162 | **144** | **344** | 128346 | **544** | 0.00946116 |
| **145** | PF21B_H UMAN | PHD finger protein 21B | **145** | **345** | 112885 | **545** | 0.01010602 |
| **146** | N/A | Chromosome 3 open reading frame 37 | **146** | **346** | 56941 | **546** | 0.01010602 |
| **147** | N/A | PREDICTED: Homo sapiens hypothetical gene supported by BC047417 | **147** | **347** | 400027 | **547** | 0.01010602 |
| **148** | KCD15_ HUMAN | Potassium channel tetramerisation domain | **148** | **348** | 79047 | **548** | 0.01010602 |
| **149** | PAEP_H UMAN | containing 15 Progestagen-associated endometrial protein | **149** | **349** | 5047 | **549** | 0.01010602 |
| **150** | DAZP1_ HUMAN | DAZ associated protein 1 | **150** | **350** | 26528 | **550** | 0.01010602 |
| **151** | PRPS1_H UMAN | Phosphoribosyl pyrophosphate synthetase 1 | **151** | **351** | 5631 | **551** | 0.01010602 |
| **152** | CSF1R_ HUMAN | Colony stimulating factor 1 receptor | **152** | **352** | 1436 | **552** | 0.01010602 |
| **153** | SYT9_H UMAN | Synaptotagmin IX | **153** | **353** | 143425 | **553** | 0.01010602 |
| **154** | K0513_H UMAN | KIAA0513 | **154** | **354** | 9764 | **554** | 0.01078799 |
| **155** | PAK4_H UMAN | p21(CDKN1A)-activated kinase 4 Tripartite motif- | **155** | **355** | 10298 | **555** | 0.01078799 |
| **156** | R052_H UMAN | containing protein Sodium channel | **156** | **356** | 6737 | **556** | 0.01078799 |
| **157** | SCN1B_ HUMAN | subunit beta-1 WW domain | **157** | **357** | 6324 | **557** | 0.01078799 |
| **158** | WBP2_H UMAN | binding protein 2 | **158** | **358** | 23558 | **558** | 0.01078799 |
| **159** | PIM2_H UMAN | Pim-2 oncogene | **159** | **359** | 11040 | **559** | 0.01078799 |
| **160** | LXN_HU MAN | Latexin Chromosome 2 | **160** | **360** | 56925 | **560** | 0.01078799 |
| **161** | CB015_H UMAN | open reading frame 15 Steroid 5 | **161** | **361** | 150590 | **561** | 0.01078799 |
| **162** | TECRL_ HUMAN | alpha-reductase 2-like 2 motif- | **162** | **362** | 253017 | **562** | 0.01078799 |
| **163** | TRI55_H UMAN | Tripartite containing protein 55 | **163** | **363** | 84675 | **563** | 0.01078799 |
| **164** | MK13_H UMAN | Mitogen-activated protein kinase 13 | **164** | **364** | 5603 | **564** | 0.01078799 |
| **165** | HS105_H UMAN | Heat shock 105kDa/110kDa protein 1 | **165** | **365** | 10808 | **565** | 0.01150872 |
| **166** | ENOB_H UMAN | Enolase 3 | **166** | **366** | 2027 | **566** | 0.01150872 |
| **167** | ORML3_ HUMAN | ORM1-like 3 | **167** | **367** | 94103 | **567** | 0.01150872 |
| **168** | ZN232_H UMAN | Zinc finger protein 232 Coiled-coil domain | **168** | **368** | 7775 | **568** | 0.01150872 |
| **169** | CCD33_ HUMAN | containing 33 | **169** | **369** | 80125 | **569** | 0.01150872 |
| **170** | RIP_HU MAN | RPA interacting protein SH3-domain GRB2- | **170** | **370** | 84268 | **570** | 0.01150872 |
| **171** | SHLB1_ HUMAN | like endophilin B1 Mitochondrial | **171** | **371** | 51100 | **571** | 0.01150872 |
| **172** | RM28_H UMAN | ribosomal protein L28 | **172** | **372** | 10573 | **572** | 0.01150872 |
| **173** | LGUL_H UMAN | Glyoxalase I Dual | **173** | **373** | 2739 | **573** | 0.01150872 |
| **174** | DUS22_ HUMAN | specificity phosphatase 22 | **174** | **374** | 56940 | **574** | 0.01226991 |
| **175** | BORG2_ HUMAN | CDC42 effector protein | **175** | **375** | 10602 | **575** | 0.01226991 |
| **176** | KLF12_H UMAN | Kruppel-like factor 12 Chemokine | **176** | **376** | 11278 | **576** | 0.01226991 |
| **177** | CCL19_ HUMAN | (C-C motif) ligand 19 | **177** | **377** | 6363 | **577** | 0.01226991 |
| **178** | N/A | Hypothetical protein LOC203547 | **178** | **378** | 203547 | **578** | 0.01226991 |
| **179** | RASH_H UMAN | v-Ha-ras Harvey rat sarcoma viral oncogene | **179** | **379** | 3265 | **579** | 0.01226991 |
| **180** | VATG1_ HUMAN | ATPase, H+ transporting, lysosomal 13kDa, V1 subunit G1 | **180** | **380** | 9550 | **580** | 0.01226991 |
| **181** | ABL1_H UMAN | v-ab1 Abelson murine leukemia viral oncogene homolog 1 | **181** | **381** | 25 | **581** | 0.01226991 |
| **182** | GCC1_H UMAN | GRIP and coiled-coil domain-containing protein 1 | **182** | **382** | 79571 | **582** | 0.01226991 |
| **183** | KCMB3_ HUMAN | Potassium large conductance calcium-activated channel, subfamily M beta member 3 | **183** | **383** | 27094 | **583** | 0.01226991 |
| **184** | DEMA_ HUMAN | Erythrocyte membrane protein band 4.9 | **184** | **384** | 2039 | **584** | 0.01226991 |
| **185** | RT35_H UMAN | Mitochondrial ribosomal protein S35 | **185** | **385** | 60488 | **585** | 0.01307331 |
| **186** | MORN4_ HUMAN | Chromosome 10 open reading frame 83 | **186** | **386** | 118812 | **586** | 0.01307331 |
| **187** | CCG2_H UMAN | Calcium channel, voltage-dependent, gamma subunit 2 | **187** | **387** | 10369 | **587** | 0.01307331 |
| **188** | CENPR_ HUMAN | Integrin beta 3 binding protein | **188** | **388** | 23421 | **588** | 0.01307331 |
| **189** | PDE9A_ HUMAN | Phosphodiesterase 9A (PDE9A) | **189** | **389** | 5152 | **589** | 0.01307331 |
| **190** | DCAKD_ HUMAN | Dephospho-CoA kinase domain-containing protein | **190** | **390** | 79877 | **590** | 0.01307331 |
| **191** | TFPT_H UMAN | TCF3 fusion partner | **191** | **391** | 29844 | **591** | 0.01307331 |
| **192** | RFA1_H UMAN | Replication protein A1, 70kDa | **192** | **392** | 6117 | **592** | 0.01307331 |
| **193** | BATF_H UMAN | Basic leucine zipper transcription factor, ATF-like | **193** | **393** | 10538 | **593** | 0.01307331 |
| **194** | CJ081_H UMAN | PH domain-containing protein C10orf81 | **194** | **394** | 79949 | **594** | 0.01307331 |
| **195** | TAF9B_ HUMAN | TAF9B RNA polymerase II, TATA box binding protein (TBP)-associated factor, 31kDa | **195** | **395** | 51616 | **595** | 0.01307331 |
| **196** | ARL8B_ HUMAN | ADP-ribosylation factor-like 8B | **196** | **396** | 55207 | **596** | 0.01307331 |
| **197** | CDC2_H UMAN | Cell division cycle 2, G1 to S and G2 to M | **197** | **397** | 983 | **597** | 0.01307331 |
| **198** | TAB2_H UMAN | Mitogen-activated protein kinasekinasekinase 7 interacting protein 2 | **198** | **398** | 23118 | **598** | 0.01307331 |
| **199** | LCA5_H UMAN | Leber congenital amaurosis 5 | **199** | **399** | 167691 | **599** | 0.01307331 |
| **200** | CRYAA_ HUMAN | Crystallin, alpha A | **200** | **400** | 1409 | **600** | 0.0139207 |

| | | | | | | | |
|---|---|---|---|---|---|---|---|
| NB: c1 = local prostate cancer, insignificant disease corresponding to a clinical tumor stage ≤ T2 (UICC 2002 classification) and Gleason Score ≤ 6 and Prostate Cancer Volume ≤ 0.5 ml c2 = local prostate cancer, significant disease corresponding to a clinical tumor stage > T2 (UICC 2002 classification); and/or Gleason Score > 6; and/or Prostate Cancer Volume > 0.5 ml | | | | | | | |

The term "marker" or "tumor marker", as used herein, relates to a gene, genetic unit, antibody, antigen or sequence (a nucleotide sequence or amino acid or protein sequence) as defined in Table 1, whose expression level is modified, i.e. increased, in a cancerous cell, or in a cancerous tissue or in any type of sample comprising cancerous cells or cancerous tissues or portions or fragments thereof, in comparison to a control level or state.

The term also refers to any expression product of said genetic unit or sequence or variants or fragments thereof, as well as homologues or derivatives thereof. The term specifically refers to the genes, genetic units, sequences, proteins, protein sequences, antibodies, antigens, homologues and/or derivatives thereof indicated as marker #1 to #200 in Table 1, having the nucleotide or amino acid sequences of SEQ ID NOs: 1 to 600, respectively, wherein the numbers 1-200 indicate the nucleotide sequences and the following numbers 201-600 the corresponding amino acid sequences, or derivatives or fragments thereof. The term also comprises corresponding genomic sequences as indicated in section F) of Table 1. Importantly, the term additionally comprises any known or yet unknown isoform (either as mRNA molecule or transcript or in the form of a polypeptide or protein), splice variant or corresponding derivative which can be derived from said genomic sequence. Corresponding information on the isoforms or splice variants would either be known by the person skilled in the art or could be retrieved with the help of suitable techniques, software tools etc. from databases or information depositories. The presence, size, form and/or identity of isoforms or splice variants may additionally be detected, determined and/or calculated with suitable tools known to the person skilled in the art. Furthermore, the size, identity, location etc. of intron and/or exon sequences and/or boundaries within said genomic sequences would also be known to the person skilled in the art. It is envisaged by the present invention that correspondingly identified intron and exon boundaries may be respected or used during the course of marker detection etc. as described herein.

A "marker" or "tumor marker" according to the present invention may also comprises nucleotide sequences showing a high degree of homology to a marker molecule as indicated in Table 1, in particular to the nucleotide sequence indicated in section D) of Table 1. Furthermore, a "marker" or "tumor marker" according to the present invention may comprise amino acid sequences or protein sequences showing a high degree of homology to a marker molecule as indicated in Table 1, in particular to the amino acid sequence indicated in section E) and section G) of Table 1.

Nucleic acid sequences according to the present invention may be, for example, at least 75%, 80%, 85%, 90%, 91%, 92%, 93%, 94%, 95%, 96%, 97%, 98% or 99% identical to the sequence as set forth in section D) of Table 1, amino acid sequences may be at least 75%, 80%, 85%, 90%, 91%, 92%, 93%, 94%, 95%, 96%, 97%, 98% or 99% identical to the sequence as set forth in section E) and section G) of Table 1, nucleic acid sequences encoding amino acid sequences may be at least 75%, 80%, 85%, 90%, 91%, 92%, 93%, 94%, 95%, 96%, 97%, 98% or 99% identical to the sequence as set forth in section E) and section G) of Table, or amino acid sequences may be encoded by nucleic acid sequences being at least 75%, 80%, 85%, 90%, 91%, 92%, 93%, 94%, 95%, 96%, 97%, 98% or 99% identical to the sequence as set forth in section D) of Table 1. Further, any variants, mutants and functional domains of the nucleic acid molecules, proteins and polypeptides as mentioned above may be encompassed. The term "tumor marker gene" or "marker gene" as used herein thus relates to the gene encoding the tumor marker mentioned in Table 1. Preferably, the term relates to a gene expressing a tumor marker protein as indicated in Table 1, e.g. specific exon combinations derivable from the indicated genomic sequence information of Table or as set forth in the sequences of section D) of Table 1. The term also relates to DNA molecules derived from mRNA transcripts encoding a tumor marker as indicated in Table 1, preferably cDNA molecules.

A "gene", "genetic unit" or a "nucleotide sequence" is a nucleic acid sequence which may be transcribed under certain physiological or biochemical conditions. The transcribed nucleic acid may further (but must not necessarily) be translated under certain physiological or biochemical conditions into a polypeptide, e.g. when placed under the control of appropriate regulatory sequences. The boundaries of the coding sequence may be determined by a start codon at the 5' (amino) terminus and a translation stop codon at the 3' (carboxy) terminus.

The terms "protein" or "polypeptide" are used herein to designate a produced or naturally occurring polypeptide or a recombinant polypeptide corresponding to the tumor marker as mentioned in Table 1. The term "protein" according to the present invention is to be seen as being interchangeably with the term "polypeptide". The polypeptides or proteins may be encoded by any of the abovementioned nucleic acid molecules. The polypeptides or proteins may further be glycosylated or may be non-glycosylated or may otherwise by modified. In addition, polypeptides or proteins may also include an initial modified methionine residue, in some cases as a result of host-mediated processes.

The term "cancer" as used in the context of the present invention refers to any of a number of diseases that are characterized by uncontrolled, abnormal proliferation of cells, as well as any of a number of characteristic structural and/or molecular features. A "cancerous cell" is accordingly understood as a cell having specific structural properties, lacking differentiation and in many instances, being capable of invasion and metastasis.

The term "cancerous" relates in the context of the present invention to a cancerous disease state as defined herein above. The term "non-cancerous" relates in the context of the present invention to a condition in which neither benign nor malign proliferation can be detected. Suitable means for said detection are known in the art.

The term "progression of cancer" as used herein relates to a switch between different stages of cancer development and principally refers to a situation in which the cancer disease becomes worse and/or spreads in the body. Any changes that are associated with a worsening of the disease, i.e. be it the aggressiveness of the cancer, the nature of the transformation (benign to malignant), the localization of the tumor and or /cancerous cells, the occurrence of metastases, presentation of additional or more profound clinical symptoms, recurrence of a tumor after treatment, decreased survival rate, are typically translated into a progression of stages of a given cancer disease.

Any worsening of the disease can thus be translated into a switch into the next stage of a given cancer disease, e.g. stages 0 and I to IV of the TNM classification, preferably the TNM classification system for prostate cancer as defined herein below, or any other stage or sub-stage of any suitable staging or scoring system, starting from a healthy condition up to a terminal cancer scenario. Typically such switches are accompanied by a modification of the expression level of the tumor marker or group of tumor markers according to Table 1, preferably an increase or decrease of the expression level in a test sample in comparison to a previous test sample from the same individual, a test sample from an individual having been diagnosed with a certain cancer type and/or tumor or cancer stage or state, or a value derivable from an information depository on expression data etc.

Progression of cancer may further be determined, checked, crosschecked or independently be diagnosed etc. according to the "Gleason score". To determine the Gleason score, typically a grade is assigned to the most common tumor pattern, and a second grade to the next most common tumor pattern. The two grades are added together to get a Gleason score. The Gleason grade is also known as the Gleason pattern or Gleason sum. The Gleason grade may range from 1 to 5, with 5 having the worst prognosis. The Gleason score typically ranges from 2 to 10, with 10 having the worst prognosis. Methods to apply the Gleason score system, corresponding assessment techniques etc. would be known to the person skilled in the art. The scoring system may be used, for example, in order to verify, check or fine-tune the diagnosis, detection, indication of stages or monitoring according to the present invention.

The term "less progressed stage" as used herein, relates to an expression level which may be determined at the same time and/or under similar or comparable conditions as the test sample by using (a) sample(s) previously collected and stored from a subject/subjects whose disease state, is/are known or from the same subject at an earlier point in time, e.g. 1, 2, 3,4, 5, 6, 7, 8, 9, 10, 11, 12 months, 2 years, 3 years, 4 years, 5 years, 10 years before etc. ("expression level of a less progressed stage"). The term may also refer to an expression level corresponding to a cancer stage or cancer form, whose disease state or stage is known. The term "disease state" relates to any state or type of cellular or molecular condition between a non-cancerous cell state and/or healthy and (including) a terminal cancerous cell state. The term, thus, includes benign tumor forms as well as malignant tumor forms. Preferably, the term includes different cancerous proliferation/developmental stages or levels of tumor development in the organism between (and excluding) a non-cancerous cell state and (including) a terminal cancerous cell state. For example, these stages may include all stages of the histological grading as per the guidelines of the American Joint Commission on Cancer. As per their standards, one grading possibility is:

| | |
|---|---|
| GX | Grade cannot be assessed |
| G1 | Well differentiated (Low grade) |
| G2 | Moderately differentiated (Intermediate grade) |
| G3 | Poorly differentiated (High grade) |
| G4 | Undifferentiated (High grade) |

Further envisaged is the alternative four-tier grading scheme:

| | |
|---|---|
| Grade 1 | Low grade; Well-differentiated |
| Grade 2 | Intermediate grade; Moderately-differentiated |
| Grade 3 | High grade; Poorly-differentiated |
| Grade 4 | Anaplastic; Anaplastic |

Also envisaged is the alternative three-tier grading scheme:

| | |
|---|---|
| Grade 1 | Low grade; Well-differentiated |
| Grade 2 | Intermediate grade; |
| Grade 3 | High grade; Poorly-differentiated; |

or the two-tier grading scheme

| | |
|---|---|
| Grade 1 | Low grade; Well-differentiated |
| Grade 2 | High grade; Poorly-differentiated. |

Such developmental stages may also include all stages of the TNM (Tumor, Node, Metastasis) classification system of malignant tumors as defined by the UICC, e.g. stages 0 and I to IV. The stages may in particular include all TNM stages of prostate cancer as define herein below. The term also includes stages before TNM stage 0, e.g. developmental stages in which cancer biomarkers known to the person skilled in the art show a modified expression or expression pattern. In a specific embodiment of the present invention a less progressed stage may be a healthy state. In a further specific embodiment of the present invention a less progressed sate may also be a stage of benign tumor development, e.g. benign prostate tumors. Particularly preferred are less progressed stages of malignant cancers, e.g. malignant prostate cancer.

Corresponding expression levels or information about the expression level(s) of (a) less progressed stage(s) may be derived, for example, from experimental approaches or from a database of expression patterns or expression levels from previously tested subjects, tissues or cells or from any suitable source of information known to the person skilled in the art. In a specific embodiment of the present invention, the expression level of a less progressed stage can be determined from a reference sample derived from a subject who has been diagnosed to suffer from a certain cancer, and wherein the stage and development has been determined. Correspondingly obtained values and information may also be combined, normalized and statistically processed according to any suitable technique or method known to the person skilled in the art.

The term "more progressed stage" as used herein, relates to the reflection of any changes that are associated with a worsening of the disease as defined herein above, e.g. the aggressiveness of the cancer, the nature of the transformation (benign to malignant), the localization of the tumor and or /cancerous cells, the occurrence of metastases, the presentation of additional or more profound clinical symptoms, recurrence of a tumor after treatment, decreased survival rate, the modification of the expression of bio- or tumor markers, e.g. of tumor marker known to the person skilled in the art like PSA in comparison to a corresponding less progressed stage as defined herein above. Preferably, the term relates to a worsened disease state of a tumor or cancer in comparison to a less progressed stage as defined herein above. For example, if the less progressed stage is a stage 0 or healthy state, the more progressed stage may be any higher or more advanced stage, e.g. a stage of any one of stage I, II, III or IV. Preferably, a "more progressed stage" as used herein may be the next worse stage if starting from a less progressed stage as defined herein above. For example, if the less progressed stage is a stage I, the more progressed stage may be a stage II. The "next worse stage" may be reflected by any of the known staging and/or grading systems known to the person skilled in the art. Preferably, the "next worse stage" refers to the staging system provided by the UICC 2002 classification, more preferably the TNM classification for prostate cancer provided herein below. In a specific embodiment of the present invention the staging or grading of a tissue may optionally or additionally be determined, checked, crosschecked or independently be diagnosed by classical staging methods known to the person skilled in the art, e.g. via histological approaches, imaging methods etc.

In a specific embodiment of the present invention a more progressed stage may be determined by a comparison of the expression level of a tumor marker as indicated in Table 1 to a control level or control state of the same tumor marker. The term "control level" (or "control state"), as used herein, relates to an expression level which may be determined at the same time and/or under similar or comparable conditions as the test sample by using (a) sample(s) previously collected and stored from a subject/subjects whose disease state, e.g. cancerous, non-cancerous, having a tumor, having no tumor, and whose disease stage(s) as defined herein above is/are known. Alternatively, the control level may be determined by a statistical method based on the results obtained by analyzing previously determined expression level(s) of the gene(s) of the tumor marker or group of tumor markers according to Table 1 in samples from subjects whose disease state is known. Furthermore, the control level can be derived from a database of expression levels or patterns from previously tested subjects or cells. Moreover, the control level may be multiple control levels whose control levels are determined from multiple reference samples. The control level may accordingly be derived from experimental approaches or from a database of expression levels from previously tested subjects, tissues or cells or from any suitable source of information known to the person skilled in the art. In a specific embodiment of the present invention, the control level can be determined from a reference sample derived from a subject who has been diagnosed to suffer from a certain cancer, and wherein the stage and development of the cancer or tumor disease has been determined, as well as from healthy individuals.

In the context of the present invention, a control level determined from a biological sample that is known not to be cancerous is called "normal control level". In another embodiment of the present invention, the control level can be from a cancerous biological sample, e.g. a sample from a subject for which cancer, in prostate cancer was diagnosed independently, it may be designated as "cancerous control level". Alternatively, reference samples may comprise material derived from cell lines, e.g. immortalized cancer cell lines, or be derived from tissue xenografts. Preferably, material derived from prostate cancer cell lines or material derived from tissue xenografts with human prostate tissue, in particular with benign and tumor-derived human prostate tissue, may be comprised in a reference sample according to the present invention. Examples of cancer cell lines to be used comprise cells lines PC346P, PC346B, LNCaP, VCaP, DuCaP, PC346C, PC3, DU145, PC346CDD, PC346Flul, PC346Flu2. Examples of xenografts which may be used comprise PC295, PC310, PC-EW, PC82, PC133, PC135, PC324 and PC374. Preferably an entire panel of cell lines and xenografts may be used, e.g. the human PC346 panel. Correspondingly obtained values and information may also be combined, normalized and statistically processed according to any suitable technique or method known to the person skilled in the art. By comparing a control level to a measured expression level a modification of the expression may be registered, which may accordingly be used for the determination of the more progressed stage of a cancer disease. For such comparison processes 1, 2, 3, 4, 5, 6, 7, 8, 9, 10, 11, 12, 13, 14, 15, 20, 25 or more different control levels may be determined or assessed. Accordingly, the less progressed stage of a cancer diseased may be determined by a assessing the outcome of such comparison process.

In a preferred embodiment of the invention said comparison process comprises the determination of control levels in a sample of an individual. In an even more preferred embodiment of the invention said comparison process comprises the determination of control levels in a sample of an individual afflicted with a neoplastic disease, e.g. cancer. In an even more preferred embodiment of the invention said comparison process comprises the determination of control levels obtained from a sample of an individual afflicted with a neoplastic disease, e.g. cancer, wherein the sample is representative of stages of the histological four-, three-, two-layer grading as per the guidelines of the American Joint Commission on Cancer, as defined herein above.

The comparison processes may further be combined with a comparison with the indications in sections H), and I) of Table 1. Particularly preferred are control levels determined at the same time and/or under similar or comparable conditions as the test sample by using (a) sample(s) previously collected and stored from a subject/subjects whose disease state is/are known to be prostate cancer, more preferably prostate cancer of stage >T2 (UICC 2002 classification), even more preferably prostate cancer of stage >T2 (UICC 2002 classification) and Gleason score >6.

The term "expression level" as used herein refers to the amount of any transcript and/or protein derivable from a defined number of cells or a defined tissue portion, preferably to the amount of a transcript and/or protein obtainable in a standard nucleic acid (e.g. RNA) or protein extraction procedure. Suitable extraction methods are known to the person skilled in the art.

The term "modified" or "modified expression level" in the context of the present invention thus denotes a change in the expression level. Expression levels are deemed to be "changed" when the gene expression of the tumor marker or group of tumor markers according to Table 1, e.g. in a sample to be analyzed, differs by, for example, 5%, 6%, 7%, 8%, 9%, 10%, 15%, 20%, 25%, 30%, 40%, 50%, or more than 50% from a control level or the expression level of a less progressed stage, as defined herein above, or at least 0.1 fold, at least 0.2 fold, at least 1 fold, at least 2 fold, at least 5 fold, or at least 10 fold or more in comparison to a control level or the expression level of a less progressed stage as defined herein above.

The term "modified" as used throughout the specification relates preferably to an increase or up-regulation of the expression level of the tumor marker or group of tumor markers according to Table 1 if a test sample is compared to a control level or the expression level of a less progressed stage as defined herein above.

In a preferred embodiment of the present invention the expression of the tumor marker(s) or group of tumor markers is increased (up-regulated) when comparing the expression in the more progressed stage to the expression in the less progressed stage, as indicated in section I) of Table 1. The term "increased" or "increased expression level" or "up-regulated expression level" or "increase of expression level" (which may be used synonymously) in the context of the present invention thus denotes a raise in the expression level of the tumor marker or group of tumor markers according to Table 1 between a situation to be analyzed, e.g. a situation derivable from a patient's sample, and a reference point, which could either be a control level derivable from any suitable prostate tumor or cancer stage known to the person skilled in the art, e.g. a healthy state, a benign tumor stage or the expression of a less progressed stage as defined herein above. Expression levels are deemed to be "increased" when the gene expression of the tumor marker or group of tumor markers according to Table 1, e.g. in a sample to be analyzed, differs by, for example, 2%, 3%, 4%, 5%, 6%, 7%, 8%, 9%, 10%, 15%, 20%, 25%, 30%, 40%, 50%, or more than 50% from a control level, or at least 0.1 fold, at least 0.2 fold, at least 1 fold, at least 2 fold, at least 5 fold, or at least 10 fold or more in comparison to a control level or the expression level of a less progressed stage as defined herein above. In a particularly preferred embodiment of the present invention the tumor marker or group of tumor markers comprises at least 1, 2, 3, 4, 5, 6, 7, 8, 9, 10, 11, 12, 13, 14, 15, 16, 17, 18, 19, 20, 21, 22, 23, 24, 25, 27, 29, 30, 32, 35, 37, 40, 42, 45, 46, 47, 48, 49, 50 or all tumor markers indicated in Table 1.

In another preferred embodiment of the present invention the group of tumor markers comprises at least 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190,195 or all of the tumor markers of Table 1. Also preferred is a group of tumor markers comprising at least 1, 2, 3, 4, 6, 7, 8, 9, 11, 1,2, 13, 14, 16, 17, 18, 19, 21, 22, 23, 24, 26, 27, 28, 29, 31, 32, 33, 34, 36, 37, 38, 39, 41, 42, 43, 44, 46, 47, 48, 49, 51, 52, 53, 55, 56, 57, 58, 59,61,62,63,64,66,67,68,69,71,72,73,74,76,77,78,79,81,82,83,84,86,87,88,89, 91 92, 93, 94, 95, 96, 97, 98, 99, or 100 of the tumor markers indicated in Table 1. The group may further comprise any sub-grouping or combinations of these markers.

In yet another embodiment of the present invention the group of tumor markers comprises those tumor markers which show a p-value of the expression modification of 0.0001, 0.0002, 0.0003, 0.0004, 0.0005, 0.006, 0.0007, 0.0008, 0.0009, 0.001, 0.0011, 0.0012, 0.0013, 0.0014, 0.005, 0.06, 0.007, 0.008, 0.009, 0.01, 0.012, 0.013, 0.014, or lower as indicated in section H) of Table 1. Preferred are groups of markers which show p-values of the expression of between about 0.0001 and about 0.001, particularly preferred are groups of markers having a p-value of expression of 0.0033 or lower, as indicated in section H) of Table 1. The term "p-value" is a measure of the probability that a variant would assume a value greater than or equal to the observed value strictly by chance and is expressed by the following term: P (z ≥ z_{observed}). Thus, in the context of the present invention, the p-value may be seen as a measure of statistical significance.

In another embodiment of the present invention the group of tumor markers comprises at least 5 tumor markers corresponding to tumor marker #1 to #5 of Table 1, at least 10 tumor markers corresponding to tumor marker #1 to #10 of Table 1, at least 15 tumor markers corresponding to tumor marker #1 to #15 of Table 1, at least 20 tumor markers corresponding to tumor marker #1 to #20 of Table 1, at least 25 tumor markers corresponding to tumor marker #1 to #25 of Table 1, at least 30 tumor markers corresponding to tumor marker #1 to #30 of Table 1, at least 35 tumor markers corresponding to tumor marker #1 to #35 of Table 1, at least 40 tumor markers corresponding to tumor marker #1 to #40 of Table 1, at least 45 tumor markers corresponding to tumor marker #1 to #45 of Table 1 or at least 50 tumor markers corresponding to tumor marker #1 to #50 of Table 1. Furthermore, the group of tumor markers may comprise tumor markers #1 to #55, tumor markers #1 to #60, tumor markers #1 to #65, tumor markers #1 to #70, tumor markers #1 to #75, tumor markers #1 to #80, tumor markers #1 to #85, tumor markers #1 to #90, tumor markers #1 to #95, tumor markers #1 to #100, tumor markers #1 to #105, tumor markers #1 to #110, tumor markers #1 to #115, tumor markers #1 to #120, tumor markers #1 to #125, tumor markers #1 to #130, tumor markers #1 to #135, tumor markers #1 to #140, tumor markers #1 to #145, tumor markers #1 to #150, tumor markers #1 to #155, tumor markers #1 to #160, tumor markers #1 to #165, tumor markers #1 to #170, tumor markers #1 to #175, tumor markers #1 to #180, tumor markers #1 to #185, tumor markers #1 to #190, tumor markers #1 to #195, tumor markers #1 to #200 of Table 1. The group of tumor marker according to the present invention may also comprise tumor markers #1, #3, #5, #7 and #9 of Table 1, #2, #4, #6, #8, and #10 of Table 1, #3, #5, #7, #9 and #11 of Table 1, #4, #6, #8, #10, and #12 of Table 1, #5, #7, #9, #11 and #13 of Table 1, #6, #8, #10, #12 and #14 of Table 1, #7, #9, #11, #13 and #15 of Table 1, #8, #10, #12, #14 and #16 of Table 1, #9, #11, #13, #15 and #17 of Table 1, #10, #12, #14, #16 and #18 of Table 1, #11, #13, #15, #17 and #19 of Table 1, #12, #14, #16, #18 and #20 of Table 1, #13, #15, #17, #19 and #21 of Table 1 etc.

In a further aspect the present invention relates to the use of a tumor marker or group of tumor markers as defined herein above as a marker for diagnosing, detecting, monitoring or prognosticating a cancer disease associated with a progression from a less progressed cancer stage to a more progressed cancer stage, or the progression from a less progressed cancer stage to a more progressed cancer stage.

The term "diagnosing a cancer disease associated with a progression from a less progressed cancer stage to a more progressed cancer stage" as used herein means that a subject or individual may be considered to be suffering from a more progressed cancer stage, e.g. a more progressed prostate cancer, when the expression level of a tumor marker or the group of tumor markers of the present invention is modified, e.g. increased/up-regulated compared to the expression level of a less progressed disease state as defined herein above, or compared to a control level as defined herein above. The term "diagnosing" also refers to the conclusion reached through that comparison process. An expression level may be deemed to be modified, when the expression level of a tumor marker or group of tumor markers as defined herein above differs by, for example, between about 1% and 100%, e.g. 1%, 2%, 3%, 4%, 5%, 6%, 7%, 8%, 9%, 10%, 15%, 20%, 25%, 30%, 40%, 50% or more from the expression level of a less progressed disease state or from a control level as defined herein above, or at least 0.1 fold, at least 0.2 fold, at least 1 fold, at least 2 fold, at least 5 fold, or at least 10 fold or more in comparison to such a less progressed disease state or control level. The modification may be an increase of said expression level.

In a further embodiment, an additional similarity in the overall gene expression pattern of a group of tumor markers according to the present invention between a sample obtained from a subject and a control sample or a sample corresponding to a less progressed cancer disease state as described herein above, may indicate that the subject is suffering from a more progressed cancer disease stage. In another embodiment of the present invention, the diagnosis may be combined with the elucidation of additional cancer biomarker expression levels, in particular prostate cancer biomarkers. Suitable biomarkers, in particular prostate cancer biomarkers, would be known to the person skilled in the art. For example, the expression of biomarkers like PSA may be tested. X90

The term "detecting a cancer disease associated with a progression from a less progressed cancer stage to a more progressed cancer stage" as used herein means that the presence of a cancer disease or disorder in an organism, which is associated with a more progressed cancer stage may be determined or that such a disease or disorder may be identified in an organism. The determination or identification of a more progressed cancer disease or disorder may be accomplished by a comparison of the expression level of the tumor marker or group of tumor markers of the present invention in a sample from a patient or individual to be analyzed and the expression level of a control level as defined herein above, wherein said control level corresponds to the expression level of said more progressed cancer disease or disorder. In a preferred embodiment of the present invention a more progressed cancer stage may be detected if the expression level the tumor marker or group of tumor markers is similar to an expression level of a more progressed cancer stage. The expression level of the more progressed cancer stage may be independently established, e.g. from sample depositories, value databases etc. as mentioned herein above.

The term "graduating a cancer disease associated with a progression from a less progressed cancer stage to a more progressed cancer stage" as used herein means that the clinical stage, phase, grade or any other suitable sub-step of cancer disease related features such as the transition from a benign to a malignant tumor, the grade of malignancy, the grade of tissue damage to non-cancerous tissue, the grade of the extent of tumor growth, the grade of aggressiveness of a tumor, the grade of metastasizing and all other useful and suitable parameters of a cancerous disease or disorder in an organism may be determined in an organism.

In a preferred embodiment the graduating of a more progressed cancer stage may be accomplished by a comparison of the expression level of the tumor marker or group of tumor markers according to Table 1 of the present invention in a sample from a patient or individual to be analyzed and a control level as defined herein above, or the expression level of a less progressed cancer stage as defined herein above. In a further preferred embodiment the graduating of a more progressed cancer stage may be accomplished by a comparison of the expression level of the tumor marker or group of tumor markers according to Table 1 of the present invention in a sample from a patient or individual to be analyzed and any of the cancer cells, cancer tissues, tumor biopsies, or the cancerous control levels mentioned above. In further preferred embodiment the graduating of a more progressed cancer stage may be accomplished by a comparison of the expression level of the tumor marker or group of tumor markers according to Table 1 of the present invention in a sample from a patient or individual to be analyzed and the above cancer cells, cancer tissues, tumor biopsies, or the cancerous control levels being derived from or representative for a less progressed stage of the cancer. In further preferred embodiment the graduating of a more progressed cancer stage may be accomplished by a comparison of the expression level of the tumor marker or group of tumor markers according to Table 1 of the present invention in a sample from a patient or individual to be analyzed and the above cancer cells, cancer tissues, tumor biopsies, or the cancerous control levels being derived from or representative for a more progressed stage of the cancer. In a further preferred embodiment the determination of the graduating cancer may be accomplished by a comparison of the expression level of the tumor marker or group of tumor markers according to Table 1 of the present invention in a sample from a patient or individual to be analyzed and the above cancer cells, cancer tissues, tumor biopsies, or the cancerous control levels being derived from or representative for an identical stage of the cancer.

The term "monitoring a cancer disease associated with a progression from a less progressed cancer stage to a more progressed cancer stage" as used herein relates to the accompaniment of a diagnosed or detected, more progressed cancer disease or disorder, e.g. during a treatment procedure or during a certain period of time, typically during 6 months, 1 year, 2 years, 3 years, 5 years, 10 years, or any other period of time. The term "accompaniment" means that states of disease as defined herein above and, in particular, changes of these states of disease may be detected by comparing the expression level of the tumor marker or group of tumor markers of the present invention in a sample to a control level as defined herein above or to the expression level of an established, e.g. independently established cancer cell or cell line, or to corresponding database values in any type of periodical time segment, e.g. every week, every 2 weeks, every month, every 2, 3, 4, 5, 6, 7, 8, 9, 10, 11 or 12 month, every 1.5 year, every 2, 3, 4, 5, 6, 7, 8,9 or 10 years, during any period of time, e.g. during 2 weeks, 3 weeks, 1, 2, 3, 4, 5, 6, 7, 8, 9, 10, 11, 12 months, 1.5, 2, 3, 4, 5, 6, 7, 8, 9, 10, 15 or 20 years, respectively. The established, e.g. independently established, cancer cell or cell line giving rise to a control level may be derived from samples corresponding to different stages of cancer development, e.g. the stages as mentioned herein above. In a preferred embodiment of the present invention the term relates to the accompaniment of a diagnosed more progressed prostate cancer.

The term "prognosticating a cancer disease associated with a progression form a less progressed cancer stage to a more progressed cancer stage " as used herein refers to the prediction of the course or outcome of a diagnosed or detected more progressed cancer stage, e.g. during a certain period of time, during a treatment or after a treatment. The term also refers to a determination of chance of survival or recovery from the disease, as well as to a prediction of the expected survival time of a subject. A prognosis may, specifically, involve establishing the likelihood for survival of a subject during a period of time into the future, such as 6 months, 1 year, 2 years, 3 years, 5 years, 10 years or any other period of time.

The term "progression from a less progressed cancer stage to a more progressed cancer stage" as used herein relates to a switch between different stages of cancer development. Such a progression may be a development in small steps, e.g. from a certain stage to the next, or may alternatively be a development skipping one or more such steps, e.g. from stage I to stage III of the TNM classification. A progression from a less progressed cancer stage to a more progressed cancer stage may be considered as being detected or diagnosed if the expression level of a tumor marker or group of tumor marker according to the present invention is modified, e.g. increased or reduced or both by a value of between 3% to 50%, preferably by a value of 10%, 15%, 20% or 25% in a test sample in comparison to a previous test sample from the same individual. The modification may be detected over any period of time, preferably over 1, 2, 3, 4, 5, 6, 7, 8, 9, 10, 11, 12 months, 1.5, 2, 3, 4, 5, 6, 7, 8, 9, 10, 15 or 20 years, i.e. the value indicated above may be calculated by comparing the expression level of the tumor marker or group of tumor markers at a first point in time and at a second point in time after the above indicated period of time.

In a particularly preferred embodiment of the present invention the term "progression from a less progressed cancer stage to a more progressed cancer stage" relates to a switch from a healthy state or a benign prostate tumor state to a malignant prostate cancer state. Alternatively, for the comparison test samples from other individuals may be used, e.g. test samples of healthy individuals. Also envisaged is the use of available database information on the expression or the employment of cancer cell collection samples etc.

In a further embodiment the present invention relates to the diagnosis and detection of a predisposition for developing a more progressed cancer stage. A "predisposition for developing a more progressed cancer stage" in the context of the present invention is a state of risk of developing a more progressed cancer stage. Preferably a predisposition for developing a more progressed cancer stage may be present in cases in which the expression level of the tumor marker or group of tumor marker of the present invention as defined herein above is above a normal control level as defined herein above, i.e. a reference expression level derived from tissues or samples of a subject which are evidently healthy. The term "above" in this context relates to an expression level of the tumor marker of group of tumor markers which is increased by about 2% to 20% in comparison to such a control level, preferably increased by about 15%.

Alternatively, a predisposition for developing a more progressed cancer stage in the context of the present invention may be given in situations in which the expression level of the tumor marker or group of tumor markers as defined herein above is above a normal control level and in which further, alternative cancer markers, e.g. PSA, show no modification of expression level or the expression pattern in a less progressed cancer stage. Suitable further cancer markers are known to the person skilled in the art.

Thus, a predisposition for a more progressed cancer stage may be considered as being diagnosed or detected if one of the above depicted situations is observed.

The difference between the expression levels of a test biological sample and a control level or the expression level of a less progressed cancer stage can be normalized to the expression level of one or more control nucleic acids, e.g. housekeeping genes whose expression levels are known not to differ depending on the cancerous or non-cancerous state of the cell. Exemplary control genes include inter alia β-action, glycerinaldehyde 3-phosphate dehydrogenase (GAPDH), and ribosomal protein P1, 18s RNA, Ubiquitin C, Cytochrom C-1. Particularly preferred is the use of GAPDH.

In the context of the present invention, the terms "diagnosing" and "prognosticating" are also intended to encompass predictions and likelihood analyses. Tumor markers or groups of tumor markers may accordingly be used clinically in making decisions concerning treatment modalities, including therapeutic intervention or diagnostic criteria such as a surveillance for the disease. According to the present invention, an intermediate result for examining the condition of a subject may be provided. Such intermediate result may be combined with additional information to assist a doctor, nurse, or other practitioner to diagnose that a subject suffers from the disease. Alternatively, the present invention may be used to detect cancerous cells in a subject-derived tissue, and provide a doctor with useful information to diagnose that the subject suffers from the disease.

A subject or individual to be diagnosed, monitored or in which a more progressed cancer, a progression towards such cancer or a predisposition such cancer is to be detected or prognosticated according to the present invention is an animal, preferably a mammal, more preferably a human being.

Particularly preferred is the use of molecular imaging tools as known to the person skilled in the art, e.g. magnetic resonance imaging (MRI) and/or magnetic photon resonance imaging (MPI) technology in the context of using a tumor marker or group of tumor marker for diagnosing, detecting, monitoring or prognosticating a more progressed cancer of the progression towards such a cancer. For example, a tumor marker or group of tumor markers according to the present invention may be used as a marker for diagnosing, detecting, monitoring or prognosticating malignant, hormone-sensitive prostate cancer or the progression towards more progressed cancer states in approaches like MRI or MPI that allow for online detection of the diagnostic marker within a human subject.

In another aspect the present invention relates to a composition for diagnosing, detecting, graduating, monitoring or prognosticating a cancer disease associated with a progression from a less progressed cancer stage to a more progressed cancer stage, or the progression from a less progressed cancer stage to a more progressed cancer stage, comprising a nucleic acid affinity ligand and/or a peptide affinity ligand for the expression product(s) or protein(s) of said tumor marker or a group of tumor markers as defined above.

The term "nucleic acid affinity ligand for the expression product of a tumor marker or group of tumor markers" as used herein refers to a nucleic acid molecule being able to specifically bind to a transcript or a DNA molecule derived the nucleic acid molecules of said tumor marker or a group of tumor markers as defined above, preferably to the nucleotide sequences, (DNA sequence(s)) depicted in section D) of Table 1 or to the complementary nucleotide sequences (DNA sequence(s)) of the sequence(s) depicted in section D) of Table 1 or a corresponding RNA molecule. The nucleic acid affinity ligand may also be able to specifically bind to a DNA sequence being at least 75%, 80%, 85%, 90%, 91%, 92%, 93%, 94%, 95%, 96%, 97%, 98% or 99% identical to the sequence(s) as set forth in section D) of Table 1 or a DNA sequence encoding an amino acid sequence being at least 75%, 80%, 85%, 90%, 91%, 92%, 93%, 94%, 95%, 96%, 97%, 98% or 99% identical to the sequence(s) as set forth in section E) and section G) of Table 1 or to any fragments of said sequences.

The term "peptide affinity ligand for the protein of a tumor marker or a group of tumor markers" as used herein refers to a peptide molecule being able to specifically bind to the proteins of the tumor marker or group of tumor markers according to Table 1. The peptide molecule may preferably be able to specifically bind to a protein or polypeptide comprising the amino acid sequence(s) as set forth in section E) and section G) of Table 1. The peptide affinity ligand may also be able to specifically bind to a protein or polypeptide comprising an amino acid sequence encoded by a DNA sequence being at least 75%, 80%, 85%, 90%, 91%, 92%, 93%, 94%, 95%, 96%, 97%, 98% or 99% identical to the sequence as set forth in section E) and section G) of Table 1 or to a protein or polypeptide comprising an amino acid sequence being at least 75%, 80%, 85%, 90%, 91%, 92%, 93%, 94%, 95%, 96%, 97%, 98% or 99% identical to the sequence(s) as set forth in section E) and section G) of Table 1 or to any fragments of said sequences.

The term "peptide" in the context of the affinity ligand of the present invention refers to any type of amino acid sequence comprising more than 2 amino acids, e.g. polypeptide structures, protein structures or functional derivatives thereof. Furthermore, the peptide may be combined with further chemical moieties or functionalities.

The term "expression product" as used herein refers to a transcript or an mRNA molecule of the tumor marker or group of tumor markers according to Table 1 generated by the expression of the corresponding genomic sequence according to Table 1. More preferably, the term relates to a processed transcript of the tumor marker or group of tumor markers according to Table 1 as defined herein above, e.g. via the sequence(s) as set forth in sections D) and/or F) of Table 1. A person skilled in the art would know how to determine the identity, size, length and any other useful parameter of transcripts of tumor markers according to the present invention based on the information provided in said section F) of Table 1.

The term "protein of a tumor marker or group of tumor markers" as used herein also refers to any polypeptide, protein, in particular to the polypeptides or proteins as set forth in section E) and section G) of Table 1 or any domain, epitope, oligopeptide, or peptide derivable therefrom.

In a preferred embodiment of the present invention the composition of the present invention may comprise one or more, e.g. 2, 3, 4, 5, 6, 7, 8, 9, 10, 11, 12, 13, 14, 15, 16, 17, 18, 19, 20 or more nucleic acid and/or peptide affinity ligands selected from the group consisting of a set of oligonucleotides specific for the expression product of the tumor marker or group of tumor markers according to Table 1, a probe specific for the expression product of the tumor marker or group of tumor markers according to Table 1, an aptamer specific for the expression product or for the protein of the tumor marker or group of tumor markers according to Table 1, an antibody specific for the protein the tumor marker or group of tumor markers according to Table 1 and an antibody variant specific for the protein of the tumor marker or group of tumor markers according to Table 1. The composition of the present invention may, for example, comprise a set of oligonucleotides specific for the expression product the tumor marker or group of tumor markers according to Table 1 and/or a probe specific for the expression product of the tumor marker or group of tumor markers according to Table 1.

The term "oligonucleotide specific for the expression product the tumor marker or group of tumor markers according to Table 1" as used herein refers to a nucleotide sequence which is complementary to the sense- or antisense-strand of the tumor marker or group of tumor markers according to Table 1. Preferably, the oligonucleotide is complementary to the DNA sequence(s) shown in section D) of Table 1, or to the complementary DNA sequence of the sequence shown in section D) of Table 1. The oligonucleotide sequence may also be complementary to a DNA sequence being at least 75%, 80%, 85%, 90%, 91%, 92%, 93%, 94%, 95%, 96%, 97%, 98% or 99% identical to the sequence(s) as set forth in section D) of Table 1 or a DNA sequence encoding an amino acid sequence being at least 75%, 80%, 85%, 90%, 91%, 92%, 93%, 94%, 95%, 96%, 97%, 98% or 99% identical to the sequence as set forth in section E) and section G) of Table 1. The oligonucleotide may have any suitable length and sequence known to the person skilled in the art, as derivable from the sequence(s) shown in section D) of Table 1 or its complement. Typically, the oligonucleotide may have a length of between 8 and 60 nucleotides, preferably of between 10 and 35 nucleotides, more preferably a length of 11, 12, 13, 14, 15, 16, 17, 18, 19, 20, 21, 22, 23, 24, 25, 26, 27, 28, 29, 30, 31, 32 or 33 nucleotides. Oligonucleotide sequences specific for the expression product of the tumor marker or group of tumor markers according to Table 1 may be defined with the help of software tools known to the person skilled in the art.

In a further embodiment of the present invention the oligonucleotide sequences may be complementary to genomic sequences localized in (an) exon(s) of the gene(s) encoding for the tumor marker or group of tumor markers according to Table 1. Corresponding information on genomic loci is indicated in section F) of Table 1. An oligonucleotide usable as a forward primer may be localized at the boundary between exonic and intronic sequences. Such boundary positions may be determined with the help of any suitable tool, based on the information provided in section F) of Table 1.

The term "probe specific for the expression product of the tumor marker or group of tumor markers according to Table 1" as used herein means a nucleotide sequence which is complementary to the sense- or antisense-strand of the tumor marker or group of tumor markers according to Table 1. Preferably, the probe is complementary to the DNA sequence(s) depicted in section D) of Table 1 or to the complementary DNA sequence of the sequence(s) shown in section D) of Table 1. The probe sequence may also be complementary to a DNA sequence being at least 75%, 80%, 85%, 90%, 91%, 92%, 93%, 94%, 95%, 96%, 97%, 98% or 99% identical to the sequence(s) as set forth in the sequences indicated in section D) of Table 1 or a DNA sequence encoding an amino acid sequence being at least 75%, 80%, 85%, 90%, 91%, 92%, 93%, 94%, 95%, 96%, 97%, 98% or 99% identical to the sequence(s) as shown in section E) and section G) of Table 1. The probe may have any suitable length and sequence known to the person skilled in the, as derivable from the sequence(s) shown in section D) or its/their complement. Typically, the probe may have a length of between 6 and 300 nucleotides, preferably of between 15 and 60 nucleotides, more preferably a length of 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 nucleotides. Probe sequences specific for the expression product of the tumor marker or group of tumor markers according to Table 1 may be defined with the help of software tools known to the person skilled in the art. The composition of the present invention may additionally or alternatively comprise an aptamer specific for the expression product or protein of the tumor marker or group of tumor markers according to Table 1.

The term "aptamer specific for the expression product of the tumor marker or group of tumor markers according to Table 1" as used herein refers to a short nucleic acid molecule, e.g. RNA, DNA, PNA, CNA, HNA, LNA or ANA or any other suitable nucleic acid format known to the person skilled in the art, being capable of specifically binding to the tumor marker or group of tumor markers according to Table 1, preferably the DNA molecule with (a) sequence(s) as shown in section D) of Table 1. More preferably, the nucleic acid aptamer molecule may specifically bind to a DNA sequence(s) shown in section D) of Table 1 or a double stranded derivative thereof. The nucleic acid aptamer according to the present invention may also bind to an RNA molecule corresponding to the transcript(s) of the tumor marker or group of tumor markers according to Table 1, preferably an RNA molecule corresponding to the DNA sequence(s) as shown in section D) of Table 1. The nucleic acid aptamer may further be capable of specifically binding to a DNA sequence being at least 75%, 80%, 85%, 90%, 91%, 92%, 93%, 94%, 95%, 96%, 97%, 98% or 99% identical to the sequence(s) as shown in section D) of Table 1 or a DNA sequence encoding an amino acid sequence being at least 75%, 80%, 85%, 90%, 91%, 92%, 93%, 94%, 95%, 96%, 97%, 98% or 99% identical to the sequence(s) as shown in section D) of Table 1 or RNA molecules corresponding to these sequences. A nucleic acid aptamer according to the present invention may further be combined with additional moieties, e.g. with interacting portions like biotin or enzymatic functionalities like ribozyme elements.

The term "aptamer specific for the protein of the tumor marker or group of tumor markers according to Table 1" as used herein refers to (a) short peptide(s) capable of interacting and specifically binding the protein(s) of the tumor marker or group of tumor markers according to Table 1. The peptide aptamer(s) may preferably be able to specifically bind to (a) protein(s) or polypeptide(s) comprising (the) amino acid sequence(s) as shown in section E) and section G) of Table 1. The peptide aptamer(s) may also be able to specifically bind to (a) protein(s) or polypeptide(s) comprising (an) amino acid sequence(s) encoded by (a) DNA sequence(s) being at least 75%, 80%, 85%, 90%, 91%, 92%, 93%, 94%, 95%, 96%, 97%, 98% or 99% identical to the sequence(s) as shown in section D) of Table 1 or to a protein or polypeptide comprising an amino acid sequence being at least 75%, 80%, 85%, 90%, 91%, 92%, 93%, 94%, 95%, 96%, 97%, 98% or 99% identical to the sequence(s) as set forth in shown in section E) and section G) of Table 1. Typically, (a) peptide aptamer(s) is/are a variable peptide loop, comprising for example 10 to 20 amino acids. In the context of the present invention the peptide aptamer(s) may preferably be attached at one or both ends to a scaffold structure. The scaffold structure may be any molecule, preferably a protein, which has good solubility properties. Suitable scaffold molecules would be known to the person skilled in the art. A preferred scaffold molecule to be used in the context of the present invention is the bacterial protein thioredoxin-A. The aptamer peptide loop may preferably be inserted within a reducing active site of the scaffold molecule. Alternatively, staphylococcal protein A and domains thereof and derivatives of these domains, such as protein Z or lipocalins may be used as scaffold structures in the context of the present invention.

Peptide aptamers may be generated according to any suitable method known to the person skilled in the art, e.g. via yeast two-hybrid approaches.

In another preferred embodiment of the present invention the composition may comprise, or may additionally comprise, (an) antibody/antibodies, including auto-antibodies, specific for the protein(s) of the tumor marker according to Table 1, e.g. an antibody as defined herein below.

In another aspect of the invention relates to an antibody specific for the tumor marker according to Table 1. Preferably, such an antibody specifically binds to a protein or polypeptide having the amino acid shown in section E) and section G) of Table 1, or any derivative, fragement etc. thereof as defined herein above. Such antibodies are contemplated for any application, use, method, composition, immunoassay, screening method and pharmaceutical compositions as defined in the present application. The term "antibody" as used herein refers to immunoglobulin molecules and immunologically active portions of immunoglobulin molecules, i. e. molecules that contain an antigen binding site that immunospecifically binds an antigen. The immunoglobulin molecules of the invention can be of any type (e. g., IgG, IgE, IgM, IgD, IgA and IgY), class (e. g., IgG1, IgG2, IgG3, lgG4, IgA1 and IgA2) or subclass of immunoglobulin molecule.

Antibodies of the present invention may be described or specified in terms of the epitope(s) or portion(s) of a polypeptide of the present invention which they recognize or specifically bind. Preferred epitopes according to the present invention are amino acids 1-10, 11-20, 21-30, 31-40, 41-50, 51-60, 61-70, 71-80, 81-90, 91-100, 101-110, 111-120, 121-130, 131-140, 141-150, 151-160, 161-170, 171-180, 181-190, 191-200, 201-210 etc. or any other specific stretch of amino acids of a protein of the tumor markers of the present invention, preferably of the sequence as shown in section E) and section G) of Table 1. Further comprised are all other suitable epitopes, which can be recognized, determined, described and subsequently be employed according to methods known to the person skilled in the art.

The term "specific for the tumor marker according to Table 1" as used herein refers to the immunospecific detection and binding of an antibody to an antigenic epitope as defined herein above. The term "specifically binding" excludes non-specific binding but does not necessarily exclude cross-reactivity with other antigens, in particular with antigens comprising the same antigenic epitope detected by the present antibody.

In a preferred embodiment antibodies of the invention include human autoantibodies, polyclonal, monoclonal, multispecific, human, humanized or chimeric antibodies, single chain antibodies, Fab fragments, Fab' fragments, fragments produced by a Fab expression library, F(ab')2, Fv, disulfide linked Fv, minibodies, diabodies, scFv, sc(Fv)2, whole immunoglobulin molecules small modular immunopharmaceuticals (SMIP), binding-domain immunoglobulin fusion proteins, camelized antibodies, V_{HH} containing antibodies, anti-idiotypic (anti-Id) antibodies (including, e. g., anti-Id antibodies to antibodies of the invention) and epitope-binding fragments of any of the above.

Most preferably, the antibodies are human antigen-binding antibody fragments of the present invention and include Fab, Fab' and F (ab')2, Fv, single-chain Fvs (scFv), sc(Fv)2, single-chain antibodies, disulfide-linked Fvs (sdFv) and fragments comprising either a VL or VH domain.

The antibodies according to the invention may be from any animal origin including birds and mammals. Preferably, the antibodies are human, murine (e. g., mouse and rat), donkey, monkey, rabbit, goat, guinea pig, camel, horse, or chicken.

The antibodies according to the present invention may be monospecific, bispecific, trispecific or of greater multispecificity. Multispecific antibodies may be specific for different epitopes of a polypeptide of the present invention or may be specific for both a polypeptide of the present invention as well as for a heterologous epitope, such as a heterologous polypeptide or solid support material.

Antibodies of the present invention may also be described or specified in terms of their cross-reactivity. In a particularly preferred embodiment the present invention relates to antibodies that do not bind any other analog, ortholog, or homolog of a polypeptide of the present invention. However, also antibodies that bind polypeptides with at least 95%, at least 90%, at least 85%, at least 80%, at least 75%, at least 70%, at least 65%, at least 60%, at least 55%, and at least 50% identity (as calculated using methods known in the art and described herein) to a polypeptide of the present invention are included in the present invention.

In a further embodiment the antibodies of the invention include derivatives which are modified, for instance by the covalent attachment of any type of molecule to the antibody such that said covalent attachment does not prevent the antibody from specifically binding to the epitope or from generating an anti-idiotypic response. Typical examples of such modifications are glycosylation, acetylation, pegylation, phosphylation, amidation, derivatization by known protecting/blocking groups, proteolytic cleavage, linkage to a cellular ligand or other protein, etc. Chemical modifications may be carried out by known techniques, including specific chemical cleavage, acetylation, formylation etc. Additionally, the derivative may contain one or more non-classical amino acids.

Antibodies may be produced according to any suitable method known to the person skilled in the art. Monoclonal antibodies of defined specificity may be produced using, for instance, the hybridoma technology developed by Köhler and Milstein (Köhler and Milstein, 1976, Eur. J. Immunol., 6: 511-519).

In further embodiment of the present invention the antibody or fragment thereof as defined herein above may be biotinylated or labeled. In a particularly preferred embodiment said label is a radioactive label, an enzymatic label, a fluorescent label, a chemiluminescent or a bioluminescent label. Alternatively, antibodies may also be labeld or combined with fluorescent polypeptides, e.g. green fluoresenct protein (GFP) as well as derivates thereof known to the person skilled in the art.

Alternatively, a polynucleotide encoding an antibody may be generated from a nucleic acid from a suitable source.

In a further embodiment of the present invention a nucleic acid molecule encoding the antibody or fragment thereof as defined herein above may be used for recombinant antibody expression. Preferably, such expression vectors contain the antibody coding sequences and appropriate transcriptional and translational control signals. The vectors may either comprise coding sequences for the variable heavy chain or the variable light chain or for both. Such vectors may also include the nucleotide sequence encoding the constant regions of the antibody molecule. In a preferred embodiment of the present invention mammalian cells, more preferably Chinese hamster ovary cells (CHO), in conjunction with a vector such as the major intermediate early gene promoter element from human cytomegalovirus may be used as an effective expression system for antibodies.

In another aspect the present invention relates to a cell that produces the antibody or fragment thereof as defined herein above. Such a cell may be a hybridoma cell as defined herein above or a cell which expresses a nucleic acid molecule encoding an antibody according to the present invention. Particularly preferred are cells or cell lines which stably express the antibody molecule.

In addition, the antibodies of the present invention or fragments thereof can be fused to any heterologous polypeptide sequence, preferably to those defined herein above, e.g. in order to facilitate antibody purification or to provide target means for the antibody.

In a specific embodiment of the present invention commercially available antibodies against the tumor marker according to Table 1, in particular against a protein having an amino acid sequence as indicated in section E) and section G) of Table 1 may be comprised in the composition or may be used diagnostically.

An affinity ligand, as described herein above, may be labeled with various markers or may be detected by a secondary affinity ligand, labeled with various markers, to allow detection, visualization and/or quantification. This can be accomplished using any suitable labels, which can be conjugated to the affinity ligand capable of interaction with the expression product(s) of the tumor marker or group of tumor markers according to Table 1 or the corresponding protein(s) or to any secondary affinity ligand, using any suitable technique or methods known to the person skilled in the art.

The term "secondary affinity ligand" refers to a molecule which is capable of binding to the affinity ligand as defined herein above (i.e. a "primary affinity ligand" if used in the context of a system with two interacting affinity ligands). The binding interaction is preferably a specific binding.Examples of labels that can be conjugated to a primary and/or secondary affinity ligands include fluorescent dyes or metals (e.g. fluorescein, rhodamine, phycoerythrin, fluorescamine), chromophoric dyes (e.g. rhodopsin), chemiluminescent compounds (e.g. luminal, imidazole) and bioluminescent proteins (e.g. luciferin, luciferase), haptens (e.g. biotin).

In a particularly preferred embodiment an affinity ligand to be used as a probe, in particular a probe specific for the expression product(s) as defined herein above, may be labeled with a fluorescent label like 6-FAM, HEX, TET, ROX, Cy3, Cy5, Texas Red or Rhodamine, and/or at the same time with a quenching label like TAMRA, Dabcyl, Black Hole Quencher, BHQ-1 or BHQ-2. A variety of other useful fluorescents and chromophores are described in Stryer, 1968, Science, 162:526-533. Affinity ligands may also be labeled with enzymes (e.g. horseradish peroxidase, alkaline phosphatase, beta-lactamase), radioisotopes (e_{.}g_{.} ³H, ¹⁴C, ³²P, ³³P, ³⁵S, ¹²⁵I, ¹¹C, ¹³N, ¹⁵O, ¹⁸F, ⁶⁴Cu, ⁶²Cu, ¹²⁴I, ⁷⁶Br, ⁸²Rb, ⁶⁸Ga or ¹⁸F) or particles (e.g. gold).

The different types of labels may be conjugated to an affinity ligand using various chemistries, e.g. the amine reaction or the thiol reaction. However, other reactive groups than amines and thiols can also be used, e.g. aldehydes, carboxylic acids and glutamine.

In a further preferred embodiment of the present invention the nucleic acid affinity ligand or peptide affinity ligand of the present invention may be modified to function as a contrast agent, e.g. as an imaging contrast agent. The term "contrast agent" as used herein refers to a molecular compound that is capable of specifically interacting with the tumor marker or group of tumor markers as mentioned herein above or according to Table 1 and which can be detected by an apparatus positioned outside the human or animal body. Preferably, such contrast agents are suitable for use in magnetic resonance imaging (MRI) or magnetic photon imaging (MPI). The term "specifically interacting" refer to the property of a molecular compound to preferentially interact with the tumor marker or group of tumor markers according to Table 1 on the cell surface of cells being present within the human or animal body over other proteins that are expressed by such cells. Preferred contrast agents which may also be designated as contrast agent compositions will be capable of specifically detecting molecules having the nucleotide sequence(s) shown in section D) of Table 1 or the amino acid sequence(s) shown in section E) and section G) of Table 1 or derivatives or homologous variants thereof as defined herein above. Preferred contrast agents are aptamers specific for the expression product(s) of the tumor marker or group of tumor markers as mentioned herein above or according to Table 1 or for the protein(s) of the tumor marker or group of tumor markers as mentioned herein above or according to Table 1 as well as antibodies specific for the protein(s) of the tumor marker or group of tumor markers as mentioned herein above or according to Table 1. Contrast agents, aside from their property of being capable of specifically recognizing the tumor marker or group of tumor markers as mentioned herein above or according to Table 1 will in addition typically comprise a further molecule which is detectable by the specific detection technology used.

The term "modified to function" as used herein thus refers to any suitable modifications known to the person skilled in the art, which may be necessary in order to allow the use of the contrast agent in molecular imaging methods, in particular in MRI or MPI. For example, if fluorescent spectroscopy is used as a detection means, such molecules may comprise fluorophores as detectable marker molecules that can be excited at a specific wavelength.

Alternatively, a radioactive label, e.g. a radioisotope as described herein above may be employed. With respect to preferred contrast agents in accordance with the invention that are suitable for MRI, the contrast agents such as the above described antibodies may comprise a marker molecule which is detectable by MRI. Such detectable labels include e.g. USPIOS and 19Fluor.

In a specific embodiment of the present invention a composition may additionally comprise accessory ingredients like PCR buffers, dNTPs, a polymerase, ions like bivalent cations or monovalent cations, hybridization solutions, secondary affinity ligands like, e.g. secondary antibodies, detection dyes and any other suitable compound or liquid necessary for the performance of a detection based on any of the affinity ligands or contrast agents as defined herein above, which is known to the person skilled in the art.

In another aspect the present invention relates to the use of a nucleic acid or peptide affinity ligand for the expression product(s) or protein(s) of the tumor marker or group of tumor markers as mentioned herein above or according to Table 1, for the preparation of a composition for diagnosing, detecting, monitoring or prognosticating cancer disease associated with a progression from a less progressed cancer stage to a more progressed cancer stage or the progression from a less progressed cancer stage to a more progressed cancer stage or a predisposition for a more progressed cancer stage, as described herein above. The composition is preferably for diagnosing, detecting, monitoring or prognosticating cancer disease associated with a progression from a less progressed cancer stage to a more progressed cancer stage or the progression from a less progressed cancer stage to a more progressed cancer stage or a predisposition for a more progressed cancer stage in an individual, e.g. a patient or subject to be analyzed or examined.

In a preferred embodiment the present invention relates to the use of a set of oligonucleotides specific for the expression product(s) of the tumor marker or group of tumor markers as mentioned herein above or according to Table 1 and/or a probe specific for the expression product(s) the tumor marker or group of tumor markers as mentioned herein above or according to Table 1, preferably specific for a nucleic acid sequence having a sequence as indicated in section D) of Table 1 or being complementary to such a sequence, for the preparation of a composition for diagnosing, detecting, monitoring or prognosticating cancer disease associated with a progression from a less progressed cancer stage to a more progressed cancer stage or the progression from a less progressed cancer stage to a more progressed cancer stage or a predisposition for a more progressed cancer stage, as described herein above.

In another preferred embodiment the present invention relates to the use of an aptamer specific for the expression product(s) or protein(s) of the tumor marker or group of tumor markers as mentioned herein above or according to Table 1, preferably a protein having an amino acid sequence as shown in section E) and section G) of Table 1, for the preparation of a composition for diagnosing, detecting, monitoring or prognosticating cancer disease associated with a progression from a less progressed cancer stage to a more progressed cancer stage or the progression from a less progressed cancer stage to a more progressed cancer stage or a predisposition for a more progressed cancer stage, as described herein above. The set of oligonucleotides is preferably for diagnosing, detecting, monitoring or prognosticating cancer disease associated with a progression from a less progressed cancer stage to a more progressed cancer stage or the progression from a less progressed cancer stage to a more progressed cancer stage or a predisposition for a more progressed cancer stage in an individual, e.g. a patient or subject to be analyzed or examined.

In a further preferred embodiment the present invention relates to the use of an antibody specific for protein(s) of the tumor marker or group of tumor markers as mentioned herein above or according to Table 1 or an antibody variant specific for the protein(s) of the tumor marker or group of tumor markers as mentioned herein above or according to Table 1 for the preparation of a composition for diagnosing, detecting, monitoring or prognosticating cancer disease associated with a progression from a less progressed cancer stage to a more progressed cancer stage or the progression from a less progressed cancer stage to a more progressed cancer stage or a predisposition for a more progressed cancer stage as described herein above.

In a preferred embodiment of the present invention a composition as defined herein above is a diagnostic composition.

In another aspect the present invention relates to a diagnostic kit for diagnosing, detecting, monitoring or prognosticating cancer disease associated with a progression from a less progressed cancer stage to a more progressed cancer stage or the progression from a less progressed cancer stage to a more progressed cancer stage or a predisposition for a more progressed cancer stage, comprising a set of oligonucleotides specific for the expression product(s) of the tumor marker or group of tumor markers as mentioned herein above or according to Table 1, a probe specific for the expression product(s) of the tumor marker or group of tumor markers as mentioned herein above or according to Table 1 and/or an aptamer specific for the expression product(s) or protein(s) of the tumor marker or group of tumor markers as mentioned herein above or according to Table 1 and/or an antibody specific for the protein(s) of the tumor marker or group of tumor markers as mentioned herein above or according to Table 1 and an antibody variant specific for the protein(s) of the tumor marker or group of tumor markers as mentioned herein above or according to Table 1. Typically, the diagnostic kit of the present invention contains one or more agents allowing the specific detection of the tumor marker or group of tumor markers as mentioned herein above or according to Table 1. The agents or ingredients of a diagnostic kit may, according to the present invention, be comprised in one or more containers or separate entities. The nature of the agents is determined by the method of detection for which the kit is intended. Where detection at the mRNA expression level of the tumor marker or group of tumor markers as mentioned herein above or according to Table 1, i.e. via the expression product(s) of the tumor marker or group of tumor markers as mentioned herein above or according to Table 1, is intended, the agents to be comprised may be a set of oligonucleotides specific for the expression product(s) of said tumor marker or group of tumor markers and/or a probe specific for the expression product(s) of said tumor marker or group of tumor markers, which may be optionally labeled according to methods known in the art, e.g. with labels described herein above. In addition or alternatively, an aptamer specific for the expression product(s) of the tumor marker or group of tumor markers as mentioned herein above or according to Table 1 may be comprised. Where detection at the protein level of the tumor marker or group of tumor markers as mentioned herein above or according to Table 1 is intended, the agents to be comprised may be antibodies (including autoantibodies) or compounds containing an antigen-binding fragment of an antibody or antibody variants specific for the protein(s) of the tumor marker or group of tumor markers as mentioned herein above or according to Table 1, as described herein above. In addition or alternatively an aptamer specific for the protein(s) of the tumor marker or group of tumor markers as mentioned herein above or according to Table 1 may be comprised.

Alternatively, a diagnostic kit may comprise a contrast agent as defined herein above.

The presence of specific proteins may also be detected using other compounds that specifically interact with the tumor marker or group of tumor markers as mentioned herein above or according to Table 1, e.g. specific substrates or ligands. Preferably, a diagnostic kit of the present invention contains detection reagents for expression product(s) of the tumor marker or group of tumor markers as mentioned herein above or according to Table 1 or the protein(s) of the tumor marker or group of tumor markers as mentioned herein above or according to Table 1. Such detection reagents comprise, for example, buffer solutions, labels or washing liquids etc. Furthermore, the kit may comprise an amount of a known nucleic acid molecule or protein, which can be used for a calibration of the kit or as an internal control. Typically, a diagnostic kit for the detection of expression product(s) of the tumor marker or group of tumor markers as mentioned herein above or according to Table 1 may comprise accessory ingredients like a PCR buffers, dNTPs, a polymerase, ions like bivalent cations or monovalent cations, hybridization solutions etc. A diagnostic kit for the detection of protein(s) of the tumor marker or group of tumor markers as mentioned herein above or according to Table 1 may also comprise accessory ingredients like secondary affinity ligands, e.g. secondary antibodies, detection dyes and any other suitable compound or liquid necessary for the performance of a protein detection based known to the person skilled in the art. Such ingredients are known to the person skilled in the art and may vary depending on the detection method carried out.

Additionally, the kit may comprise an instruction leaflet and/or may provide information as to the relevance of the obtained results.

In another aspect the present invention relates to a method for detecting, diagnosing, monitoring or prognosticating a cancer disease associated with a progression from a less progressed cancer stage to a more progressed cancer stage, or the progression from a less progressed cancer stage to a more progressed cancer stage comprising at least the step of determining the level of a tumor marker or a group of tumor markers as defined above, in a sample.

The term "determining the level of a tumor marker or group of tumor markers" refers to the determination of the presence or amount of expression product(s) of the tumor marker or tumor marker or group of tumor markers as mentioned herein above or according to Table 1, e.g. (a) transcript(s) of the tumor marker or group of tumor markers according to Table 1, and/or the determination of the presence and/or amount of (a) protein(s) of the tumor marker or group of tumor markers according to Table 1.

The term "level of the tumor marker or group of tumor markers according to Table 1" thus means the presence or amount of (an) expression product(s) of the tumor marker or group of tumor markers according to Table 1, e.g. (a) transcript(s) of the tumor marker or group of tumor markers according to Table 1, and/or the determination of the presence or amount of (a) protein(s) of the tumor marker or group of tumor markers according to Table 1. The determination of the presence or amount of (an) expression product(s) of the tumor marker or group of tumor markers according to Table 1, e.g. (a) transcript(s) of the tumor marker or group of tumor markers according to Table 1 or (a) protein(s) of the tumor marker or group of tumor markers according to Table 1 may be accomplished by any means known in the art.

In a preferred embodiment of the present invention the determination of the presence or amount of the expression products of the tumor marker or group of tumor markers as mentioned herein above or according to Table 1, e.g. transcript(s) and/or of protein(s) of the markers mentioned in Table 1, comprising for instance sequences as depicted in sections D), E) and G) of Table 1, is accomplished by the measurement of nucleic acid or protein levels or by the determination of the biological activity of said tumor marker or group of tumor markers.

For example, the measurement of the nucleic acid level of the expression of the tumor marker or group of tumor markers as mentioned herein above or according to Table 1 may be assessed by separation of nucleic acid molecules (e.g. RNA or cDNA) obtained from the sample in agarose or polyacrylamide gels, followed by hybridization with tumor marker specific oligonucleotide probes as defined herein above, e.g. oligonucleotide probes comprising fragments of the sequences indicated in section D) of Table 1, or complementary sequences thereof. Alternatively, the expression level may be determined by the labeling of nucleic acid obtained from the sample followed by separation on a sequencing gel. Nucleic acid samples may be placed on the gel such that patient and control or standard nucleic acid are in adjacent lanes. Comparison of expression levels may be accomplished visually or by means of a densitometer. Methods for the detection of mRNA or expression products are known to the person skilled in the art. Typically, Northern blot analysis may be used for such a purpose.

Alternatively, the nucleic acid level of the expression of the tumor marker or group of tumor markers as mentioned herein above or according to Table 1 may be detected in a DNA array or microarray approach. Typically, sample nucleic acids derived from subjects to be tested are processed and labeled, preferably with a fluorescent label. Subsequently, such nucleic acid molecules may be used in a hybridization approach with immobilized capture probes corresponding to the tumor marker or group of tumor markers as mentioned herein above or according to Table 1 of the present invention or known biomarker or cancer marker genes. Suitable means for carrying out microarray analyses are known to the person skilled in the art.

In a standard setup a DNA array or microarray comprises immobilized high-density probes to detect a number of genes. The probes on the array are complementary to one or more parts of the sequence of the marker gene, or to the entire coding region of the marker gene. In the present invention, any type of tumor marker associated polynucleotide may be used as probe for the DNA array, as long as the polynucleotide allows for a specific distinction between the tumor marker expression and the expression of other genes. Typically, cDNAs, PCR products, and oligonucleotides are useful as probes. For example, a fragment comprising 5'- or 3'-portions of the tumor markers or group of tumor markers as mentioned herein above or according to Table 1, e.g. of the sequences indicated in section D) of Table 1 may be used as a probe. The DNA array or microarray may comprise probes of one or more of the tumor marker of Table 1, e.g. 2, 3, 4, 5, 6, 7, 8, 9, 10, 11, 12, 13, 14, 15 etc. or all of the tumor markers or any combination of said markers. Furthermore, any type of fragment or sub-portion of any of the markers sequences may be combined with any further fragment or sub-portion of any of the markers sequences of Table 1. In addition to the determination of the expression of tumor markers according to Table 1 also the determination of the expression of other genes, e.g. additional biomarker or cancer marker genes is envisaged by the present invention.

A DNA array- or microarray-based detection method typically comprises the following steps: (1) Isolating mRNA from a sample and optionally converting the mRNA to cDNA, and subsequently labeling this RNA or cDNA. Methods for isolating RNA, converting it into cDNA and for labeling nucleic acids are described in manuals for micro array technology. (2) Hybridizing the nucleic acids from step 1 with probes for the marker genes. The nucleic acids from a sample can be labeled with a dye, such as the fluorescent dyes Cy3 (red) or Cy5 (blue). Generally a control sample is labeled with a different dye. (3) Detecting the hybridization of the nucleic acids from the sample with the probes and determining at least qualitatively, and more particularly quantitatively, the amounts of mRNA in the sample for the tumor marker or group of tumor markers as mentioned herein above or according to Table 1 and/or additional marker genes investigated. The difference in the expression level between sample and control can be estimated based on a difference in the signal intensity. These can be measured and analyzed by appropriate software such as, but not limited to the software provided for example by Affymetrix.

There is no limitation on the number of probes corresponding to the marker genes used, which are spotted on a DNA array. Also, a marker gene can be represented by two or more probes, the probes hybridizing to different parts of a gene. Probes are designed for each selected marker gene. Such a probe is typically an oligonucleotide comprising 5-50 nucleotide residues. Longer DNAs can be synthesized by PCR or chemically. Methods for synthesizing such oligonucleotides and applying them on a substrate are well known in the field of micro-arrays. Genes other than the marker genes may be also spotted on the DNA array. For example, a probe for a gene whose expression level is not significantly altered may be spotted on the DNA array to normalize assay results or to compare assay results of multiple arrays or different assays. Alternatively, the nucleic acid level of expression of the tumor marker or group of tumor markers according to Table 1 may be detected in a quantitative RT-PCR approach, preferably in a real-time PCR approach following the reverse transcription of the mRNA transcript(s) of the tumor marker or group of tumor markers as mentioned herein above or according to Table 1.

Typically, as first step, a transcript is reverse transcribed into a cDNA molecule according to any suitable method known to the person skilled in the art. A quantitative or real-time PCR approach may subsequently be carried out based on a first DNA strand obtained as described above. Preferably, Taqman or Molecular Beacon probes as principal FRET-based probes of this type may be used for quantitative PCR detection. In both cases, the probes, preferably probes of the tumor marker or group of tumor markers according to Table 1 as defined herein above, serve as internal probes which are used in conjunction with a pair of opposing primers that flank the target region of interest, preferably a set of oligonucleotides specific for the tumor marker or group of tumor markers according to Table 1 as defined herein above. Upon amplification of a target segment, the probe may selectively bind to the products at an identifying sequence in between the primer sites, thereby causing increases in FRET signaling relative to increases in target frequency.

Preferably, a Taqman probe to be used for a quantitative PCR approach according to the present invention may comprise (a) oligonucleotide(s) derived from the tumor marker or group of tumor markers according to Table 1 as defined above of about 22 to 30 bases that is labeled on both ends with a FRET pair. Typically, the 5' end will have a shorter wavelength fluorophore such as fluorescein (e.g. FAM) and the 3' end is commonly labeled with a longer wavelength fluorescent quencher (e.g. TAMRA) or a non-fluorescent quencher compound (e.g. Black Hole Quencher).

It is preferred that the probes to be used for quantitative PCR, in particular the probes tumor marker or group of tumor markers as mentioned herein above or according to Table 1, have no guanine (G) at the 5' end adjacent to the reporter dye in order to avoid quenching of the reporter fluorescence after the probe is degraded. A Molecular Beacon probe to be used for a quantitative PCR approach according to the present invention preferably uses FRET interactions to detect and quantify a PCR product, with each probe having a 5' fluorescent-labeled end and a 3' quencher-labeled end. This hairpin or stem-loop configuration of the probe structure comprises preferably a stem with two short self-binding ends and a loop with a long internal target-specific region of about 20 to 30 bases.

Alternative detection mechanisms which may also be employed in the context of the present invention are directed to a probe fabricated with only a loop structure and without a short complementary stem region. An alternative FRET-based approach for quantitative PCR which may also be used in the context of the present invention is based on the use of two hybridization probes that bind to adjacent sites on the target wherein the first probe has a fluorescent donor label at the 3' end and the second probe has a fluorescent acceptor label at its 5' end.

The measurement of protein levels of the protein(s) of the tumor marker or group of tumor markers as mentioned herein above or according to Table 1 or of any fragments, homologues or derivates derived thereof may be carried out via any suitable detection technique known in the art. Preferably, the protein level of tumor marker or group of tumor markers as mentioned herein above or according to Table 1 and derivatives thereof may be determined immunologically, e.g. by using an antibody specific for the protein(s) of the tumor marker or group of tumor markers as mentioned herein above or according to Table 1, preferably an antibody as defined herein above. Alternatively, antibody variants or fragments as defined herein above may be used.

The present invention also envisages the use of peptide affinity ligands like aptamers specific for the protein(s) of the tumor marker or group of tumor markers according to Table 1 as defined herein above.

Determination of the protein levels of the protein(s) of the tumor marker or group of tumor markers as mentioned herein above or according to Table 1 can be accomplished, for example, by the separation of proteins from a sample on a polyacrylamide gel, followed by identification of the protein(s) of tumor marker or group of tumor markers as mentioned herein above or according to Table 1 using specifically binding antibodies in a Western blot analysis. Alternatively, proteins can be separated by two-dimensional gel electrophoresis systems. Two-dimensional gel electrophoresis is well known in the art and typically involves iso-electric focusing along a first dimension followed by SDS-PAGE electrophoresis along a second dimension. The analysis of 2D SDS-PAGE gels can be performed by determining the intensity of protein spots on the gel, or can be performed using immune detection.In other embodiments, protein samples are analyzed by mass spectroscopy.

Within the context of the present invention antibodies specific for (a) protein of a tumor marker or group of tumor markers as mentioned herein above or according to Table 1 may be placed on a support and be immobilized. Proteins derived from samples or tissues to be analyzed may subsequently be mixed with the antibodies. A detection reaction may then be carried out, e.g. with a second affinity ligand as defined herein above, preferably with a specific antibody.

Further, within the context of the present invention a tumor marker protein or a group of tumor marker proteins specific for (an) autoantibody or a group of autoantibodies as mentioned herein above or according to Table 1 (sections E) and/or section G)) may be placed on a support and be immobilized. Autoantibodies present in samples or tissues to be analyzed may subsequently be mixed with the immobilized proteins. A detection reaction may then be carried out, e.g. with a second affinity ligand as defined herein above, preferably with a specific antibody.

Immunological tests which may be used in the context of the present invention, in particular for the diagnostic purposes of the present invention, include, for example, competitive and non-competitive assay systems using techniques such as western blots, radioimmunoassay like RIA (radio-linked immunoassay), ELISA (enzyme linked immunosorbent assay), "sandwich" immunoassays, immunoprecipitation assays, precipitin reactions, gel diffusion precipitin reactions, immunodiffusion assays, agglutination assays, e.g. latex agglutination, complement-fixation assays, immunoradiometric assays, fluorescent immunoassays, e.g. FIA (fluorescence-linked immunoassay), chemiluminescence immunoassays, electrochemiluminescence immunoassay (ECLIA) and protein A immunoassays. Such assays are routine and well known to the person skilled in the art. Furthermore, the binding affinity of an antibody to an antigen and the off-rate of an antibody-antigen interaction may be determined by competitive binding assays.

In this context, the binding affinity of an antibody to an antigen and the off-rate of an antibody- antigen interaction may be determined by competitive binding assays. One example of a competitive binding assay is a radioimmunoassay comprising the incubation of labeled antigen (e.g., ³H or ¹²⁵I) with a suitable antibody in the presence of increasing amounts of unlabeled antigen, and the detection of the antibody bound to the labeled antigen. The affinity of the antibody of interest for a particular antigen and the binding off- rates may be determined from the data by any suitable analysis approach, e.g. by a scatchard plot analysis. Competition with a second antibody may also be determined using radioimmunoassays. In this case, the antigen may be incubated with a suitable antibody conjugated to a labeled compound (e.g., ³H or ¹²⁵I) in the presence of increasing amounts of an unlabeled second antibody.

In addition, aptamers specific for the protein(s) of the tumor marker or group of tumor markers as mentioned herein above or according to Table 1, may be used in a method of detecting proteins of the tumor marker or group of tumor markers as mentioned herein above or according to Table 1. Such aptamers may preferably be labeled in order to allow the detection of a protein-ligand interaction.

The determination of the biological activity of a tumor marker or group of tumor markers as mentioned herein above or according to Table 1 may be carried out by employing molecular or enzymatic assays specific to the corresponding function or functions of the tumor marker or group of tumor markers as mentioned herein above or according to Table 1, e.g. of a tumor marker protein having a sequence as indicted in section E) and section G) of Table 1. Suitable techniques would be known to the person skilled in the art. An inhibition of the activity may be carried out by any suitable means known to the person skilled in the art, preferably via the use of suitable antisense nucleotides, siRNA molecules or miRNA molecules, more preferably via specifically hybridizing antisense nucleotides, specific siRNA or miRNA molecules, e.g. as described herein below.

In a further preferred embodiment the biological activity of the tumor marker or group of tumor markers as mentioned herein above or according to Table 1 may be tested with the help of suitable enzymatic reactions or tests for the tumor marker or group of tumor markers as mentioned herein above or according to Table 1, known to the person skilled in the art, or by employing specific inhibitors of the tumor marker or group of tumor markers as mentioned herein above or according to Table 1. The use of such inhibitors may, for example, be combined with an enzymatic readout system. Typical inhibitors of the tumor marker or group of tumor markers as mentioned herein above or according to Table 1 to be used comprise antisense molecules, siRNA molecules or miRNA molecules.

The level of the tumor marker or group of tumor markers as mentioned herein above or according to Table 1 may also be detected in methods involving histological or cell-biological procedures. Typically, visual techniques, such as light microscopy or immunofluoresence microscopy, as well as flow cytometry or luminometry may be used.

The presence of (a) protein(s) of the tumor marker or group of tumor markers as mentioned herein above or according to Table 1 in a cell may, for instance, be detected or determined by removing cells to be tested from samples as defined herein above. Also tissue sections or biopsy samples may be used for these methods. Subsequently, affinity ligands for the tumor marker or group of tumor markers as mentioned herein above or according to Table 1 may be applied, preferably antibodies or aptamers. Typically, such affinity ligands are labeled, preferably with fluorescent labels as defined herein above. Such a procedure allows for the detection of the tumor marker or group of tumor markers according to Table 1, for its/their quantification and, in addition, allows to determine the distribution and relative level of the expression thereof.

Such procedures involve the use of visualization methods. Suitable visualization methods are known to the person skilled in the art. Typical methods to be used comprise fluorometric, luminometric and/or enzymatic techniques. Fluorescence is normally detected and/or quantified by exposing fluorescent labels to light of a specific wavelength and thereafter detecting and/or quantifying the emitted light of a specific wavelength. The presence of a luminescently tagged affinity ligand may be detected and/or quantified by luminescence developed during a chemical reaction. Detection of an enzymatic reaction is due to a color shift in the sample arising from chemical reaction.

In a further, preferred embodiment the level of the tumor marker or group of tumor markers according to Table 1 may be determined by suitable molecular imaging techniques, e.g. magnetic resonance imaging (MRI) or magnetic photon imaging (MPI), and/or by using suitable contrast agents, e.g. contrast agents as defined herein above.

In a further preferred embodiment of the present invention a method for detecting, diagnosing, monitoring or prognosticating a cancer disease associated with a progression from a less progressed cancer stage to a more progressed cancer stage, or the progression form a less progressed cancer stage to a more progressed cancer stage comprises the additional step of comparing the measured nucleic acid or protein level(s) or the measured biological activity to a control level, wherein said control level is the expression level of the tumor marker or the group of tumor markers in one or more samples of a less progressed stage of the same cancer.

The expression level of the tumor marker or the group of tumor markers of a less progressed stage of the same cancer may be an expression level which may be determined at the same time and/or under similar or comparable conditions as the test sample by using (a) sample(s) previously collected and stored from a subject/subjects whose disease state, is/are known or from the same subject at an earlier point in time or an expression level corresponding to a cancer stage or cancer form, whose disease state or stage is known including different cancerous proliferation/developmental stages or levels of tumor development in the organism , as described herein above.

The term "comparing" as used herein refers to any suitable method of assessing, calculating, evaluating or processing of data. In a preferred embodiment of the present invention the determination of the presence or amount of (an) expression product(s), e.g. (a) transcript(s) of the tumor marker or group of tumor markers as mentioned herein above or according to Table 1 and/or of (a) protein(s) of the tumor marker or group of tumor markers as mentioned herein above or according to Table 1, is accomplished by the measurement of nucleic acid or protein levels or by the determination of the biological activity of the tumor marker or group of tumor markers as mentioned herein above or according to Table 1. Thus, the expression level(s) of the tumor marker or group of tumor markers as mentioned herein above or according to Table 1 may be determined by a method involving the detection of an mRNA encoded by the genes encoding for the tumor marker or group of tumor markers as mentioned herein above or according to Table 1, or the according cDNA sequence, e.g. the sequences indicated in section D) of Table 1, the detection of the protein(s) encoded by the transcript of the tumor marker or group of tumor markers as mentioned herein above or according to Table 1 and/or the detection of the biological activity of the protein(s) of the tumor marker or group of tumor markers as mentioned herein above or according to Table 1, e.g. of the proteins having the sequences indicated in section E) and section G) of Table 1. For example, the measurement of the nucleic acid level of expression of the tumor marker or group of tumor markers as mentioned herein above or according to Table 1 may be assessed by separation of nucleic acid molecules as described herein above.

In yet another embodiment as a further, additional step a decision on the presence or stage of a more progressed cancer stage or the progression towards said stage may be based on the results of the comparison step. A more progressed cancer stage may be diagnosed or prognosticated or a progression towards said more progressed cancer stage may be diagnosed or prognosticated in said method according to the corresponding definitions provided herein above in the context of the tumor marker or group of tumor markers as mentioned herein above or according to Table 1.

In another embodiment of the present invention the above mentioned method is a method of graduating cancer, comprising the steps of
(a) determining the level of the tumor marker or group of tumor markers as mentioned herein above or according to Table 1 in a sample by the measurement of nucleic acid or protein level(s) or by the determination of the biological activity of said tumor marker or group of tumor markers,
(b) comparing the measured nucleic acid or protein level(s) or the measured biological activity to a control level, wherein said control level is the expression level of the tumor marker or the group of tumor markers in one or more samples of a less progressed stage of the same cancer; and
(c) deciding on the stage or developmental status of cancer based on the results obtained in step (b).

The determination of the level of a tumor marker or group of tumor markers as mentioned herein above or according to Table 1 may be carried out according to steps as defined herein above. Preferably, the determination may be carried out as measurement of a nucleic acid level or protein level according to the herein above described options for such measurements. In one embodiment, steps a), b), c) and/or d) of this method of diagnosis may be performed outside the human or animal body. A more progressed cancer stage may be graduated in said method according to the corresponding definitions provided herein above in the context of the tumor marker or group of tumor markers as mentioned herein above or according to Table 1.

In another aspect the present invention relates to a method of data acquisition comprising at least the steps of:
(a) testing in an individual for expression of the tumor marker or group of tumor markers as mentioned herein above or according to Table 1; and
(b) comparing the expression as determined in step (a) to a control level.

The testing for expression of a tumor marker or group of tumor markers as mentioned herein above or according to Table 1 may be carried out according to steps as defined herein above. Preferably, the testing may be carried out as measurement of protein levels of the tumor marker or group of tumor markers as mentioned herein above or according to Table 1, more preferably according to the herein above described options for such measurements. The term "control level" as used in the context of the method of data acquisition refers to the expression of the tumor marker or group of tumor markers as mentioned herein above or according to Table 1 or other suitable markers in a control or the expression level of said marker(s) in a less progressed cancer stage, as defined herein above. The status, nature, amount and condition of the control level may be adjusted according to the necessities.

A comparison of the expression to a control level may be carried out according to any suitable method of assessing, calculating, evaluating or processing of data and particularly aims at the detection of differences between two data sets. A statistical evaluation of the significance of the difference may further be carried out. Suitable statistical methods are known to the person skilled in the art. Obtained data and information may be stored, accumulated or processed by suitable informatics or computer methods or tools known to the person skilled in the art and/or be presented in an appropriate manner in order to allow the practitioner to use the data for one or more subsequent deduction or conclusion steps.

In another embodiment the present invention relates to a method of detecting, diagnosing, monitoring or prognosticating a cancer disease associated with a more progressed cancer stage or the progression from a less progressed cancer stage to a more progressed cancer stage comprising at least the steps of:
(a) testing in at least one sample obtained from at least one individual suspected to suffer from cancer for expression of the expression product(s) of the tumor marker or group of tumor markers as mentioned herein above or according to Table 1 or the protein(s) of the tumor marker or group of tumor markers as mentioned herein above or according to Table 1;
(b) testing in at least one control sample obtained from at least one individual not suffering from cancer or suffering form a less progressed cancer stage for the expression of the expression product(s) of the tumor marker or group of tumor markers as mentioned herein above or according to Table 1 or the protein(s) of the tumor marker or group of tumor markers as mentioned herein above or according to Table 1;
(c) determining the difference in the expression of steps (a) and (b); and
(d) deciding on the presence of cancer, in particular of a more progressed cancer stage or on the progression of cancer, in particular from a less progressed cancer stage to a more progressed cancer stage based on the results obtained in step (c).

In one embodiment, steps a), b), c) and/or d) of this method of diagnosis may be performed outside the human or animal body. The testing for expression of the tumor marker or group of tumor markers as mentioned herein above or according to Table 1 may be carried out according to steps as defined herein above. Preferably the testing may be carried out as measurement of nucleic acid or protein levels of the tumor marker or group of tumor markers as mentioned herein above or according to Table 1 or by determining the biological activity of said tumor markers, more preferably according to the herein above described options for such measurements. The testing may be carried out in an individual, i.e. in vivo, or outside the individual, i.e. ex vivo or in vitro.

In another aspect the present invention relates to an immunoassay for detecting, diagnosing, graduating, monitoring or prognosticating a cancer disease associated with a progression from a less progressed cancer stage to a more progressed cancer stage, or for detecting, diagnosing, monitoring or prognosticating the progression from a less progressed cancer stage to a more progressed cancer stage comprising at least the steps
(a) testing in a sample obtained from an individual for the expression of a tumor marker or group of tumor markers as mentioned herein above or according to Table 1;
(b) testing in a control sample for the expression of the same tumor marker or group of tumor markers as in (a);
(c) determining the difference in expression of the tumor marker or group of tumor markers of steps (a) and (b); and
(d) deciding on the presence, stage or risk of recurrence of cancer or the progression of cancer based on the results obtained in step (c),
wherein said testing steps are based on the use of an antibody specifically binding (a) protein(s) of a tumor marker or a group of tumor markers as defined above. The term "risk of recurrence" as used herein refers to a likelihood or probabilty assessment regarding the chances or the probability that a subject or individual may be afflicted with or may be developing a similar or the same neoplastic disease as defined herein above, preferably cancer, comparable to the one that the subject or individual has been treated for or diagnosed for, based on the expression modification of the tumor marker or a group of tumor markers as defined above. A risk of recurrence may, for example, be present when a tumor marker or group of turmor markers as defined herein above shows a modified expression level, e.g. an increased or decreased expression level in comparison to a less progressed stage, in particular a non-malignant stage, benign tumor stage or a healthy stage, although histological markers, cell shapes etc. or other tumor biomarker, e.g. PSA, show no modification, or a different modification, e.g. an opposite modification or a less pronounced increase or decrease of expression level(s).

The testing for expression of the tumor marker or group of tumor markers according to Table 1 may be carried out according to steps as defined herein above. Preferably, the testing may be carried out as measurement of protein levels of the tumor marker or group of tumor markers according to Table 1, more preferably according to the herein above described options for such measurements. As controls or control samples controls as defined herein above may be used.

In a particularly preferred embodiment the testing steps may be based on the use of an antibody specifically binding to a tumor marker according to Table 1 as laid out above, e.g. (a) commercially available antibody/antibodies against (a) protein(s) of the tumor marker or group of tumor markers as mentioned herein above or according to Table 1. A cancer, in particular a more progressed cancer stage, may be diagnosed or prognosticated or a progression of cancer, in particular form a less progressed to a more progressed cancer stage, may be diagnosed or prognosticated in said immunoassay according to the corresponding definitions provided herein above in the context of the tumor marker or group of tumor markers according to Table 1 as cancer marker(s).

In a preferred embodiment of the present invention the diagnosing, detecting, monitoring or prognosticating as mentioned above is to be carried out on a sample obtained from an individual.

The term "sample obtained from an individual" as used herein relates to any biological material obtained via suitable methods known to the person skilled in the art from an individual, as laid out above. The sample used in the context of the present invention should preferably be collected in a clinically acceptable manner, more preferably in a way that nucleic acids (in particular RNA) or proteins are preserved.

The biological samples may include body tissues and/or fluids, such as blood, or blood components like serum or plasma, sweat, sputum or saliva, semen and urine, as well as feces or stool samples. Furthermore, the biological sample may contain a cell extract derived from or a cell population including an epithelial cell, preferably a cancerous epithelial cell or an epithelial cell derived from tissue suspected to be cancerous. The sample used in the context of the present invention should preferably be collected in a clinically acceptable manner, more preferably in a way that nucleic acids (in particular RNA) or proteins are preserved.

Alternatively, the biological sample may contain a cell population derived from a glandular tissue, e.g. the sample may be derived from the prostate of a male individual. Additionally, cells may be purified from obtained body tissues and fluids if necessary, and then used as the biological sample.

In a specific embodiment of the present invention the content of a biological sample may also be submitted to an enrichment step. For instance, a sample may be contacted with ligands specific for the cell membrane or organelles of certain cell types, e.g. prostate cells, functionalized for example with magnetic particles. The material concentrated by the magnetic particles may subsequently be used for detection and analysis steps as described herein above or below.

In a specific embodiment of the invention, biopsy or resections samples may be obtained and/or used. Such samples may comprise cells or cell lysates. Furthermore, cells, e.g. tumor cells, may be enriched via filtration processes of fluid or liquid samples, e.g. blood, urine, sweat etc. Such filtration processes may also be combined with enrichment steps based on ligand specific interactions as described herein above.

In a particularly preferred embodiment of the present invention a sample may be a tissue sample, a urine sample, a biopsy sample, a urine sediment sample, a blood sample, a serum sample, a plasma sample, a saliva sample, a semen sample, or a sample comprising circulating tumor cells.

A subject or individual to be diagnosed, monitored or in which a cancer, a progression of cancer or predisposition for cancer is to be detected or prognosticated according to the present invention is an animal, preferably a mammal, more preferably a human being.

In a specific embodiment of the present invention the obtaining step of a sample may be included as a first or additional step in any of the herein mentioned methods, uses or approaches.

In another aspect the present invention relates to a pharmaceutical composition relates to a pharmaceutical composition comprising at least one element selected from the group of: (a) a compound directly inhibiting the activity of a tumor marker or group of tumor markers as mentioned herein above or according to Table 1, preferably an antagonist of said tumor marker enzymatic activity; (b) a compound indirectly inhibiting the activity of a tumor marker or group of tumor markers as mentioned herein above or according to Table 1; (c) a dominant negative form of a protein of a tumor marker or group of tumor markers as mentioned herein above or according to Table 1 or a biologically active equivalent thereof; (d) a nucleic acid encoding and expressing a dominant negative form of a protein of a tumor marker or group of tumor markers as mentioned herein above or according to Table 1; (e) a miRNA specific for a tumor marker or group of tumor markers as mentioned herein above or according to Table 1; (f) an antisense molecule of a tumor marker or group of tumor markers as mentioned herein above or according to Table 1; (g) a siRNA specific for a tumor marker or group of tumor markers as mentioned herein above or according to Table 1; (h) an aptamer specific for the expression product of a tumor marker or group of tumor markers as mentioned herein above or according to Table 1 or for the protein of a tumor marker or group of tumor markers as mentioned herein above or according to Table 1; (i) a small molecule or peptidomimetic capable of specifically binding to the protein of a tumor marker or group of tumor markers as mentioned herein above or according to Table 1; and (j) an antibody specific for the protein of a tumor marker or group of tumor markers as mentioned herein above or according to Table 1 and/or an antibody variant specific for the protein of a tumor marker or group of tumor markers as mentioned herein above or according to Table 1.

The term "a compound directly inhibiting the activity of a tumor marker" as used herein refers to a compound which is capable of decreasing the activity of a tumor marker according to Table 1. Such a compound may be any direct interactor of the tumor marker according to Table 1, which has negative influence on the catalytic activity of the tumor marker or group of tumor markers according to Table 1. Such a compound may preferably be an antagonist of the catalytic activity of the tumor marker according to Table 1, e.g. of a tumor marker protein having a sequence as indicated in section E) and section G) of Table 1.

The term "a compound indirectly inhibiting the activity of a tumor marker " as used herein refers to a compound which is capable of decreasing the activity of the tumor marker or group of tumor markers according to Table 1 by an interaction with a direct interactor of the tumor marker according to Table 1 ("indirect interactor") or via an indirect working pathway not involving an interaction with of the tumor marker according to Table 1. Such a compound may be any direct interactor of an interactor of the tumor marker according to Table 1, e.g. of a tumor marker protein having a sequence as indicated in section E) and section G) of Table 1. The effect conveyed by the direct interactor of an interactor of the tumor marker according to Table 1 may be either negative if the interactor of the tumor marker or group of tumor markers according to Table 1 itself has a negative effect on the activity of the tumor marker according to Table 1, or negative, if the interactor of the tumor marker according to Table 1 has a positive effect on the activity of said tumor marker.

Alternatively, such negatively working indirect integrators may provoke a modification of the binding behavior of directly binding proteins, leading to a decreased activity of the tumor marker or group of tumor markers as mentioned herein above or according to Table 1. Typically negatively working indirect interactors may have an inhibitory effect on activators of the tumor marker according to Table 1. Examples of such interactors are enzymatic activities degrading activators of the tumor marker according to Table 1, or proteins capable of binding and quenching activators of the tumor marker according to Table 1. Alternatively, such interactors may positively modulate activities leading to a degradation of the tumor marker according to Table 1, e.g. proteinases. Further examples and their implementation would be known to the person skilled in the art.

Alternatively, an indirect inhibition of the activity of the tumor marker according to Table 1 may be conveyed by compounds deactivating, interfering or disrupting the expression of the endogenous gene(s) of said tumor markers. Examples of such compounds are specific interactors of transcription factors of the tumor marker or group of tumor markers as mentioned herein above or according to Table 1 that inhibit and/or preclude binding of transcription factors and the basal transcription machinery to the promoters of the tumor marker according to Table 1, specific destabilizing activities of the mRNA(s) of the tumor marker according to Table 1 or factors inhibiting the splicing factors specific for the tumor marker according to Table 1. Further examples and their implementation would be known to the person skilled in the art.

The "nucleic acid encoding and expressing a dominant negative form of a protein of a tumor marker" as used herein refers to any nucleic acid capable of expressing a mutant form of a naturally occurring protein or polypeptide of the tumor marker or group of tumor markers as mentioned herein above or according to Table 1. Thus the term refers to a nucleic acid encoding (a) variant(s) of the tumor marker according to Table 1, which comprises an antimorphic modification, in particular which adversely affects the tumor marker according to Table 1 of the invention. Typically, such a behavior may occur if the antimorphic variant can interact with the tumor marker according to Table 1 but blocks some aspect of its function. Preferably, such variants may comprise or lack specific domains of the tumor marker according to Table 1, e.g. one or more protein-protein interacting or dimerization domains, complex assembly domains, one or more membrane-associated domains etc. This is particularly of importance in a protein that functions as a dimer or multimer. If, for example, one part of that protein complex is mutant in some functional aspect of the multimer but is still able to form the multimer it may have a dominant effect on the other wildtype portions of the complex, and a negative effect if the mutation prevents the complex from carrying out its normal function. Thus, especially in the case of tumor markers of the present invention which homomultimerize, such a dominant-negative form can specifically block the action of the wild-type tumor-marker from which it was derived. Tests to identify dominant negative variants include appropriate genetic screenings, for instance readout-systems based on the expression of nucleic acids comprising the nucleotide sequence as indicated in section D) of Table 1 and/or functional assays of the proteins and or polypeptides of the invention comprising the amino acid sequence as indicated in section E) and section G) of Table 1 or derivatives thereof.

The term "miRNA specific for the tumor marker or group of tumor markers as mentioned herein above or according to Table 1" refers to a short single-stranded RNA molecule of typically 18-27 nucleotides in length, which regulate gene expression of one or more of the tumor marker according to Table 1. miRNAs are encoded by genes from whose DNA they are transcribed but are not translated into a protein. In a natural context miRNAs are first transcribed as primary transcripts or pri-miRNA with a cap and poly-A tail and processed to short, 70-nucleotide stem-loop structures known as pre-miRNA in the cell nucleus. This processing is performed in animals by a protein complex known as the Microprocessor complex, consisting of the nuclease Drosha and the double-stranded RNA binding protein Pasha. These pre-miRNAs are then processed to mature miRNAs in the cytoplasm by interaction with the endonuclease Dicer, which also initiates the formation of the RNA-induced silencing complex (RISC). After integration into an active RISC complex, miRNAs may base pair with their complementary mRNA molecules and inhibit translation or may induce mRNA degradation by the catalytically active members of the RISC complex, e.g. argonaute proteins. Mature miRNA molecules are typically at least partially complementary to mRNA molecules corresponding to the expression product of the present invention, and fully or partially down-regulate gene expression. Preferably, miRNAs according to the present invention may be 100% complementary to their target sequences. Alternatively, they may have 1, 2 or 3 mismatches, e.g. at the terminal residues or in the central portion of the molecule. miRNA molecules according to the present invention may have a length of between about 18 to 27 nucleotides, e.g. 18, 19, 20, 21, 22, 23, 24, 25, 26 or 27 nucleotides. Preferred are 21 to 23 mers. miRNAs having 100% complementarity may preferably be used for the degradation of nucleic acids according to the present invention, whereas miRNAs showing less than 100% complementarity may preferably be used for the blocking of translational processes.

The term "antisense molecule of the tumor marker or group of tumor markers as mentioned herein above or according to Table 1" refers to nucleic acids corresponding to the sequences indicated in section D) of Table 1 or the complementary strand thereof. Preferably, the antisense molecule of the invention comprises a sequence complementary to at least a portion of a tumor marker expression product according to the present invention. While antisense molecules complementary to the coding region sequence of tumor marker expression products may be used, those complementary to the transcribed and untranslated region are preferred. Generally, antisense technology can be used to control, i.e. reduce or abolish gene expression through antisense DNA or RNA, or through triple-helix formation. In one embodiment, an antisense molecule may be generated internally by the organism, for example intracellularly by transcription from an exogenous sequence. A vector or a portion thereof may be transcribed, producing an antisense nucleic acid of the invention. Such a vector would contain a sequence encoding the antisense nucleic acid of the invention. Such a vector can remain episomal or become chromosomally integrated, as long as it can be transcribed to produce the desired antisense molecule. Corresponding vectors can be constructed by recombinant DNA technology methods known to the person skilled in the art. Vectors can be plasmid, viral, or others known in the art, used for replication and expression in vertebrate cells, e.g. vectors as defined herein above.

In another embodiment, the antisense molecule may be separately administered. As an example, the 5' coding portion of a nucleic acid according to the present invention, e.g. of the sequence indicated in section D) of Table 1 may be used to design an antisense RNA or DNA oligonucleotide of from about 6 to 50 nucleotides in length. Preferably, the oligonucleotide is at least 10 nucleotides, at least 17 nucleotides, at least 25 nucleotides or at least 50 nucleotides in length.

The antisense nucleic acids of the invention typically comprise a sequence complementary to at least a portion of an RNA transcript of a gene of interest. However, absolute complementarity, although preferred, is not required. A sequence "complementary to at least a portion of an RNA transcript" as referred to herein, means a sequence having sufficient complementarity to be able to hybridize with the RNA, forming a stable duplex or triplex formation in the case of double stranded antisense nucleic acids. The ability to hybridize will depend on both the degree of complementarity and the length of the antisense nucleic acid. Generally, the larger the hybridizing nucleic acid, the more base mismatches with a RNA sequence of the invention it may contain and still form a stable duplex or triplex. A person skilled in the art can ascertain a tolerable degree of mismatch by use of standard procedures to determine the melting point of the hybridized complex.

Preferably antisense molecules complementary to the 5' end of the transcript, e.g., the 5' untranslated sequence up to and including the AUG initiation codon may be used in for the inhibition of transloation. In a further preferred embodiment, sequences complementary to the 3' untranslated sequences of mRNAs may also be used.

An antisense molecule according to the present invention may be DNA or RNA or chimeric mixtures or derivatives or modified versions thereof, single-stranded or double-stranded. An antisense molecule, preferably an antisense olignucleotide or any further antisense nucleic acid molecule according to the present invention or a siRNA molecule according to the present invention or any other ncRNA molecule according to the present invention as defined herein above can be modified at the base moiety, sugar moiety, or phosphate backbone, for example, to improve stability of the molecule, hybridization, etc. The molecule may include other appended groups such as peptides (e. g., for targeting host cell receptors in vivo), or agents facilitating transport across the cell membrane or the blood-brain barrier hybridization triggered cleavage agents or intercalating agents. The molecule may accordingly be conjugated to another molecule, e. g., a peptide, hybridization triggered cross-linking agent, transport agent, hybridization-triggered cleavage agent, etc.

The antisense molecule or antisense oligonucleotide, miRNA- or siRNA molecule, may comprise at least one modified base moiety which is selected from the group including 5-fluorouracil, 5-bromouracil, 5-chlorouracil, 5-iodouracil, hypoxanthine, xantine, 4-acetylcytosine, 5- (carboxyhydroxylmethyl) uracil, 5-carboxymethyl-aminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methyl guanine, 3-methyl cytosine, 5-methylcytosine, N6-adenine, 7-methyl guanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6isopentenyladenine, uracil-5-oxyacetic acid, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid, 5-methyl-2-thiouracil, 3-(3-amino-3-N-2-carboxypropyl) uracil, and 2,6-diaminopurine. The molecule may also comprise at least one modified sugar moiety selected from the group including, but not limited to, arabinose, 2-fluoroarabinose, xylulose, and hexose. In another embodiment, the molecule comprises alternatively or additionally at least one modified phosphate backbone, e.g. a phosphorothioate, a phosphorodithioate, a phosphoramidothioate, a phosphoramidate, a phosphordiamidate, a methylphosphonate, an alkyl phosphotriester, and a formacetal or analog thereof.

In another embodiment, the antisense molecule, e.g. the antisense oligonucleotide may be an alpha-anomeric oligonucleotide, i.e. an oligonucleotide which forms specific double-stranded hybrids with complementary RNA in which the strands run parallel to each other.

The term "siRNA specific for the tumor marker or group of tumor markers as mentioned herein above or according to Table 1" refers to a particular type of antisense-molecules, namely small inhibitory RNA duplexes that induce the RNA interference (RNAi) pathway to negatively regulate gene expression of the tumor marker or group of tumor markers as mentioned herein above or according to Table 1. These siRNA molecules can vary in length and may be between about 18-28 nucleotides in length, e.g. have a length of 18, 19, 20, 21, 22, 23, 24, 25, 26, 27 or 28 nucleotides. Preferably, the molecule has a length of 21, 22 or 23 nucleotides. The siRNA molecule according to the present invention may contain varying degrees of complementarity to their target mRNA, preferably in the antisense strand. siRNAs may have unpaired overhanging bases on the 5' or 3' end of the sense strand and/or the antisense strand. The term "siRNA" includes duplexes of two separate strands, as well as single strands that can form hairpin structures comprising a duplex region. Preferably the siRNA may be double-stranded wherein the double-stranded siRNA molecule comprises a first and a second strand, each strand of the siRNA molecule is about 18 to about 23 nucleotides in length, the first strand of the siRNA molecule comprises nucleotide sequence having sufficient complementarity to the target RNA via RNA interference, and the second strand of said siRNA molecule comprises nucleotide sequence that is complementary to the first strand.

Methods for designing suitable siRNAs directed to a given target nucleic acid are known to person skilled in the art. Furthermore, antagonistic siRNA molecules may be obtained according to methods of identifying antagonists as described herein.

The term "aptamer specific for the expression product or specific for the protein of the tumor marker or group of tumor markers as mentioned herein above or according to Table 1" as used herein refers to (a) short peptide(s) capable of interacting and specifically binding the protein(s) of the tumor marker according to Table 1. The peptide aptamer(s) may preferably be able to specifically bind to (a) protein(s) or polypeptide(s) comprising (the) amino acid sequence(s) as indicated in section E) and section G) of Table 1. The peptide aptamer(s) may also be able to specifically bind to (a) protein(s) or polypeptide(s) comprising (an) amino acid sequence(s) encoded by (a) DNA sequence(s) being at least 75%, 80%, 85%, 90%, 91%, 92%, 93%, 94%, 95%, 96%, 97%, 98% or 99% identical to the sequence(s) as indicated in section D) of Table 1, or to a protein or polypeptide comprising an amino acid sequence being at least 75%, 80%, 85%, 90%, 91%, 92%, 93%, 94%, 95%, 96%, 97%, 98% or 99% identical to the sequence(s) as indicated in section E) and section G) of Table 1. Typically, (a) peptide aptamer(s) is/are a variable peptide loop, comprising for example, 10 to 20 amino acids. In the context of the present invention the peptide aptamer(s) may preferably be attached at one or both ends to a scaffold structure. The scaffold structure may be any molecule, preferably a protein, which has good solubility properties. Suitable scaffold molecules would be known to the person skilled in the art. A preferred scaffold molecule to be used in the context of the present invention is the bacterial protein thioredoxin-A. The aptamer peptide loop may preferably be inserted within a reducing active site of the scaffold molecule. Alternatively, staphylococcal protein A and domains thereof and derivatives of these domains, such as protein Z or lipocalins may be used as scaffold structures in the context of the present invention. Peptide aptamers may be generated according to any suitable method known to the person skilled in the art, e.g. via yeast two-hybrid approaches.

In a preferred embodiment the above mentioned peptide aptamer is capable to bind to a protein or polypeptide of the invention corresponding to the sequences indicated in section E) and section G) of Table 1 and to reduce the biological activity and/or the enzymatic activity of these/this protein(s) by at least 10%, 20%, 30%, 40%, 50%, 60%, 70%, 80%, 90%, 95% or by at least 98% or 99% when compared to a control level obtained from an untreated sample. A "small molecule capable of specifically binding to the protein of a tumor marker or group of tumor markers as mentioned herein above or according to Table 1" as used herein refers to a small organic compound that is preferably biologically active, i.e. a biomolecule, but is preferably not a polymer. Such an organic compound may have any suitable form or chemical property. The compound may be a natural compound, e.g. a secondary metabolite or an artificial compound, which has been designed and generated de novo. In an embodiment of the present invention a small molecule is capable of blocking the interaction between the tumor marker protein(s) and its interactor(s). Methods and techniques for the identification and preparation of small molecules as well as assays for the testing of small molecules are known to the person skilled in the art.

The term "peptidomimetic capable of specifically binding to the protein of a tumor marker or group of tumor markers as mentioned herein above or according to Table 1" in the context of the present invention refers to a small protein-like chain designed to mimic a peptide and capable of binding (a) protein(s) of a tumor marker or group of tumor markers as mentioned herein above or according to Table 1. Such a peptidomimetic may arise from a modification of an existing peptide, e.g. a peptide or peptide aptamer as defined herein above, in order to alter the molecule's properties. A peptidomimetic may arise from a modification which changes the molecule's stability or binding capability. These modifications typically involve changes to the peptide that will not occur naturally. For example, a peptidomimetic according to the present invention may have altered peptide backbones or may comprise non-natural amino acids. Methods and techniques for the preparation of peptidomimetics as well as assays for the testing of peptidomimetics are known to the person skilled in the art.

A pharmaceutical composition according to the present invention may also comprise an antibody specific for the protein(s) of a tumor marker or group of tumor markers as mentioned herein above or according to Table 1 and/or an antibody variant specific for said protein, e.g. an antibody or antibody variant as defined herein above.

In a preferred embodiment such an antibody or antibody fragment may be capable of inhibiting the biological activity and/or enzymatic activity of a tumor marker or group of tumor markers as mentioned herein above or according to Table 1.

The skilled person would also be aware of the possibility to target and destroy cancer cells and tissue by virtue of conjugated antibodies specific for the tumor markers. Thus, in a specific embodiment of the present invention the antibody or fragment thereof as defined herein above may be conjugated to a therapeutic or cytotoxic agent. The term "therapeutic agent" refers to any compound, drug, small molecule or medicament, which is able to confer a therapeutic effect to a cell, a tissue or the entire organism. Examples of such agents are known to the person skilled in the art. The term "cytotoxic agent" refers to any compound, drug, small molecule which is able to confer a toxic effect to a cell or a tissue. Such agents may, for example, comprise compounds which activate endogenous cytotoxic effector systems, as well as radioisotopes, holotoxins, modified toxins, catalytic subunits of toxins, or any molecules or enzymes not normally present in or on the surface of a cell that under defined conditions cause the cell's death. The term may also include radioisotopes known in the art, additional antibodies (or complement fixing containing portions thereof) that bind an inherent or induced endogenous cytotoxic effector system, thymidine kinase, endonuclease, RNAse, alpha toxin, ricin, abrin, Pseudomonas exotoxin A, diphtheria toxin, saporin, momordin, gelonin, pokeweed antiviral protein, alpha-sarcin and cholera toxin. The term also refers to cytotoxic produgs. By "cytotoxic prodrug" is meant a non-toxic compound that is converted by an enzyme, normally present in the cell, into a cytotoxic compound. Cytotoxic prodrugs that may be used according to the invention include glutamyl derivatives of benzoic acid mustard alkylating agent, phosphate derivatives of etoposide or mitomycin C, cytosine arabinoside, daunorubicin, and phenoxyacetamide derivatives of doxorubicin.

In a another aspect the present invention relates to a pharmaceutical composition for the treatment or prevention of a cancer disease associated with a progression from a less progressed cancer stage to a more progressed cancer stage, wherein said cancer disease implies the increased (up-regulated) expression of a tumor marker or group of tumor markers as defined above, comprising at least one element selected from the group of: (a) a compound directly inhibiting the activity of a tumor marker or group of tumor markers as mentioned herein above or according to Table 1, preferably an antagonist of said tumor marker enzymatic activity; (b) a compound indirectly inhibiting the activity of a tumor marker or group of tumor markers as mentioned herein above or according to Table 1; (c) a dominant negative form of a protein of a tumor marker or group of tumor markers as mentioned herein above or according to Table 1 or a biologically active equivalent thereof; (d) a nucleic acid encoding and expressing a dominant negative form of a protein of a tumor marker or group of tumor markers as mentioned herein above or according to Table 1; (e) a miRNA specific for a tumor marker or group of tumor markers as mentioned herein above or according to Table 1; (f) an antisense molecule of a tumor marker or group of tumor markers as mentioned herein above or according to Table 1; (g) a siRNA specific for a tumor marker or group of tumor markers as mentioned herein above or according to Table 1; (h) an aptamer specific for the expression product of a tumor marker or group of tumor markers as mentioned herein above or according to Table 1 or for the protein of a tumor marker or group of tumor markers as mentioned herein above or according to Table 1; (i) a small molecule or peptidomimetic capable of specifically binding to the protein of a tumor marker or group of tumor markers as mentioned herein above or according to Table 1; and (j) an antibody specific for the protein of a tumor marker or group of tumor markers as mentioned herein above or according to Table 1 and/or an antibody variant specific for the protein of a tumor marker or group of tumor markers as mentioned herein above or according to Table 1. Such pharmaceutical compostion preferably comprises elements as defined herein above.

In another aspect of the present invention relates to a pharmaceutical comprising at least one element selected from the group of: (a) a compound directly stimulating or modulating the activity of a tumor marker or group of tumor markers as mentioned herein above or according to Table 1, preferably an agonist of said tumor marker enzymatic activity;(b) a compound indirectly stimulating or modulating the activity of a tumor marker or group of tumor markers as mentioned herein above or according to Table 1; (c) a protein of a tumor marker or group of tumor markers as mentioned herein above or according to Table 1 or a biologically active equivalent thereof; (d) a nucleic acid encoding and expressing a protein of a tumor marker or group of tumor markers as mentioned herein above or according to Table 1 and (e) a miRNA inhibitor specific for a miRNA of a tumor marker or group of tumor markers as mentioned herein above or according to Table 1.

The term "a compound directly stimulating or modulating the activity of the tumor marker or group of tumor markers as mentioned herein above or according to Table 1" as used herein refers to a compound which is capable of increasing the activity of one or more of the tumor marker according to Table 1. Such a compound may be any direct interactor of the tumor marker according to Table 1, which has positive influence on the catalytic activity of the tumor marker according to Table 1. Such a compound may preferably be an agonist of the catalytic activity of the tumor marker according to Table 1.

The term "a compound indirectly stimulating or modulating the activity of the tumor marker or group of tumor markers as mentioned herein above or according to Table 1" as used herein refers to a compound which is capable of increasing the activity of the tumor marker according to Table 1 by an interaction with a direct interactor of the tumor marker according to Table 1 ("indirect interactor") or via an indirect working pathway not involving an interaction with of the tumor marker according to Table 1. Such a compound may be any direct interactor of an interactor of the tumor marker according to Table 1, e.g. of the protein(s) of a tumor marker or group of tumor markers according to section E) and section G) of Table 1. The effect conveyed by the direct interactor of an interactor of the tumor marker according to Table 1 may be either positive if the interactor of the tumor marker according to Table 1 itself has a positive effect on the activity of the tumor marker according to Table 1, or negative, if the interactor of the tumor marker according to Table 1 has a negative effect on the activity of the tumor marker according to Table 1.

Alternatively, such positively working indirect integrators may provoke a modification of the binding behavior of directly binding proteins, leading to an increased activity of the tumor marker according to Table 1. Typically negatively working indirect interactors may have an inhibitory effect on inhibitors of the tumor marker according to Table 1. Examples of such interactors are enzymatic activities degrading inhibitors of the tumor marker according to Table 1, or proteins capable of binding and quenching inhibitors of the tumor marker according to Table 1. Alternatively, such interactors may inhibit activities leading to a degradation of the tumor marker according to Table 1, e.g. proteinase inhibitors. Further examples and their implementation would be known to the person skilled in the art.

Alternatively, an indirect stimulation of the activity of a tumor marker or group of tumor markers as mentioned herein above or according to Table 1 may be conveyed by compounds activating, protecting or sustaining the expression of the endogenous gene(s) of the tumor marker according to Table 1. Examples of such compounds are specific transcription factors of the tumor marker or group of tumor markers as mentioned herein above or according to Table 1, specific stabilizing activities of the mRNA(s) of the the tumor marker or group of tumor markers as mentioned herein above or according to Table 1 or splice factors specific for the the tumor marker or group of tumor markers as mentioned herein above or according to Table 1. Further examples and their implementation would be known to the person skilled in the art.

The "protein of a tumor marker" comprised in the pharmaceutical composition may be a protein of the tumor marker according to Table 1 as defined herein above. In particular, it may be a protein being encoded by splice variant of the tumor marker according to Table 1. More preferably it may have the amino acid sequence as set forth in section E) and section G) of Table 1. The "protein of a tumor marker" as used in this context also comprises amino acid sequences being at least 60%, 70%, 75%, 80%, 85%, 90%, 91%, 92%, 93%, 94%, 95%, 96%, 97%, 98% or 99% identical to the sequences as set forth in section E) and section G) of Table 1 and amino acid sequences being encoded by nucleotide sequences being at least 60%, 70%, 75%, 80%, 85%, 90%, 91%, 92%, 93%, 94%, 95%, 96%, 97%, 98% or 99% identical to the sequence as indicated in section D) of Table 1. In a further embodiment of the invention the homologous variants of the tumor marker according to Table 1 may additionally or alternatively have a similar or identical localization pattern as the tumor marker according to Table 1 within a cell or within a tissue type.

The term "biologically active equivalent of a tumor marker" as used herein refers to a protein of the tumor marker according to Table 1 which is capable of performing all or a majority of the individual functions of the tumor marker according to Table 1. In a further embodiment of the invention the biologically active equivalents of the tumor marker according to Table 1 may additionally or alternatively have a similar or identical localization pattern as the tumor marker according to Table 1 within a cell or within a tissue type. Biologically active equivalents of the tumor marker according to Table 1 may also comprise variants of the tumor marker according to Table 1 as defined herein above.

The proteins of the tumor marker according to Table 1 or biologically active equivalents of the tumor marker according to Table 1 according to the present invention may be produced recombinantly by any suitable method known to the person skilled in the art. The present invention, thus, also encompasses methods for the production of the tumor marker proteins according to Table 1 or biologically active equivalents of the tumor markers according to Table 1.

Accordingly, the present invention contemplates vectors containing the polynucleotides encoding the tumor markers according to Table 1 or biologically active equivalents of the tumor markers according to Table 1 as defined herein above, host cells, and the production of the tumor markers according to Table 1 or biologically active equivalents of the tumor markers according to Table 1 by recombinant techniques.

A suitable vector may be, for example, a phage, plasmid, viral, or retroviral vector. Retroviral vectors may be replication competent or replication defective. In the latter case, viral propagation generally will occur only in complementing host cells. The vectors may preferably comprise one or more of the nucleotide sequences indicated in section D) of Table 1.

Polynucleotides encoding the tumor markers according to Table 1 or biologically active equivalents of the tumor markers according to Table 1 may be joined to a vector or carrier containing a selectable marker for propagation in a host. A corresponding polynucleotide insert may be operatively linked to an appropriate promoter, such as the phage lambda PL promoter, the E. coli lac, trp, phoA and tac promoters, the SV40 early and late promoters and promoters of retroviral LTRs, or the PSA promoter. Other suitable promoters are known to the person skilled in the art. The expression constructs may further contain sites for transcription initiation, termination, and, in the transcribed region, a ribosome binding site for translation. The coding portion of the transcripts expressed by the constructs will preferably include a translation initiating codon at the beginning and a termination codon (UAA, UGA or UAG) appropriately positioned at the end of the polypeptide to be translated.

The polypeptides or proteins may be glycosylated or may be non-glycosylated or may otherwise by modified. In addition, polypeptides or proteins may also include an initial modified methionine residue, in some cases as a result of host-mediated processes. Furthermore, the polypeptide, protein or peptide may be modified by acetylation, pegylation, hesylation, formylation, phosphorylation, amidation, derivatization by known protecting/blocking groups, specific chemical cleavage, proteolytic cleavage, a linkage to a cellular ligand or other protein or hapylation, i.e. a fusion with a glycine-rich homo-aminoacid polymer (HAP), etc. Such modifications may be carried out by suitable techniques known to the person skilled in the art. Additionally, the polypeptide, peptide or variant may contain one or more non-classical amino acids.

In addition, the tumor marker proteins according to Table 1 or biologically active equivalents of the tumor markers according to Table 1 of the invention can be chemically synthesized using techniques known in the art, e.g. by using a peptide synthesizer.

The "nucleic acid encoding and expressing the tumor marker or group of tumor markers as mentioned herein above or according to Table 1" comprised in the pharmaceutical composition as defined herein above refers to any suitable carrier element, e.g. as described herein above, comprising an expressable gene of a tumor marker or group of tumor markers as mentioned herein above or according to Table 1. Preferably, such a carrier element may comprise the sequence(s) as indicated in section E) and section G) of Table 1. Such a carrier element may also comprises nucleotide sequences showing a high degree of homology to the tumor markers according to Table 1, e.g. nucleic acid sequences being at least 60%, 70%, 75%, 80%, 85%, 90%, 91%, 92%, 93%, 94%, 95%, 96%, 97%, 98% or 99% identical to the sequence(s) as indicated in section D) of Table 1 or nucleic acid sequences encoding amino acid sequences being at least 60%, 70%, 75%, 80%, 85%, 90%, 91%, 92%, 93%, 94%, 95%, 96%, 97%, 98% or 99% identical to the sequence(s) as indicated in section E) and section G) of Table 1. Alternatively, the carrier may comprise the genomic sequence of the tumor marker according to Table 1.

Furthermore, biologically active equivalents of the tumor markers according to Table 1 as defined herein above may be comprised in a carrier of the present invention.

The polynucleotide encoding the tumor marker according to Table 1 may preferably be joined to a vector containing a selectable marker for propagation in a human cell. In a preferred embodiment the polynucleotide insert may be operatively linked to a PSA promoter.

In one embodiment of the present invention nucleic acids encoding and expressing the tumor marker or group of tumor markers as mentioned herein above or according to Table 1 may be provided via living therapeutics. The term "living therapeutic" means that the tumor markers according to Table 1 or biologically active equivalents of the tumor markers according to Table 1 as defined herein above are expressed in any suitable live carrier. Accordingly, the present invention relates to corresponding polynucleotides which are suitable for expression in a living cell. The present invention also relates to vectors containing such polynucleotides, appropriate host cells, and the production of polypeptides by recombinant techniques in said host cells.

The term "live carrier" relates to any appropriate living host cell or virus known to the person skilled in the art. Representative examples of appropriate hosts include, but are not limited to, bacterial cells such as Escherichia coli or Lactobacillus, fungal cells, such as yeast cells, protozoa, insect cells, or animal cells. Preferably, the term relates to attenuated bacteria, attenuated fungal cells or attenuated protozoa. Representative examples of appropriate viruses include viruses of the group of adenoviruses, retrovirues or lentiviruses, preferably attenuated viruses of the group of adenoviruses, retroviruses or lentiviruses. In a preferred embodiment, probiotic bacterial cells, in particular probiotic Escherichia coli or Lactobacillus cells may be used. More preferably, cells of Escherichia coli Nissle 1973 and even more preferably cells of Lactobacillus casei or Lactobacillus zeae 393 may be used.

The "miRNA inhibitor specific for miRNA of a tumor marker or group of tumor markers as mentioned herein above or according to Table 1" comprised in the pharmaceutical composition as defined herein above refers to a nucleic acid molecule encoding a nucleic acid sequence complementary to a miRNA or microRNA molecule of the tumor marker or group of tumor markers as mentioned herein above or according to Table 1. The term "complementary" as used herein refers to a perfect complementary between the miRNA inhibitor nucleic acid (sense molecule) and the miRNA (antisense molecule) without any mismatch, as well as situations in which the nucleic acid contains any base mismatches and/or additional or missing nucleotides in comparison to the miRNA molecule. In other embodiments, the two molecules comprise one or more base mismatches or differ in their total numbers of nucleotides (due to additions or deletions). In further embodiments, the "complementary" miRNA inhibitor nucleic acid molecule comprises at least ten contiguous nucleotides showing perfect complementarity with a sequence comprised in the miRNA molecule.

Typically miRNA inhibitor nucleic acid molecules are naturally occurring DNA- or RNA-molecules or synthetic nucleic acid molecules comprising in their sequence one or more modified nucleotides which may be of the same type or of one or more different types.

It is, for example, envisaged by the present invention that such a miRNA inhibitor nucleic acid molecule comprises at least one ribonucleotide backbone unit and at least one deoxyribonucleotide backbone unit. Furthermore, the miRNA inhibitor nucleic acid molecule may contain one or more modifications of the RNA backbone into 2'-O-methyl group or 2'-O-methoxyethyl group (also referred to as "2'-O-methylation"), which prevented nuclease degradation in the culture media and, importantly, also prevented endonucleolytic cleavage by the RNA-induced silencing complex nuclease, leading to irreversible inhibition of the miRNA. Another possible modification, which is functionally equivalent to 2'-O-methylation, involves locked nucleic acids (LNAs) representing nucleic acid analogs containing one or more LNA nucleotide monomers, as defined herein above.

Another class of silencers of miRNA expression to be used in the context of the present invention comprises chemically engineered oligonucleotides named "antagomirs", which represent single-stranded RNA molecules conjugated to cholesterol. The molecules may comprise between 19 and 25 nucleotides. Preferably, the molecule comprises 20, 21, 22, 23 or 24 nucleotides. More preferably, the molecule comprises 23 nucleotides.

In another embodiment of the present invention miRNA inhibitors as defined herein above may be provided in the form of expression vectors to be introduced into tissue or cells. Alternatively, such vectors may also be introduced in living therapeutics as defined herein above.

Typically, RNAs may be produced from transgenes provided in the form of tranfection or transient expression vectors or carriers. For instance, competitive miRNA inhibitors may be provided as transcripts expressed from strong promoters, containing more than one, preferably multiple, tandem binding sites to a microRNA of interest.

In a specific embodiment of the present invention a demethylation agent may be comprised in the pharmaceutical composition according to the present invention. The term "demethylation agent" as used herein refers to an agent capable of demethylating chromatine structures, preferably promoter regions, more preferably the promoter(s) of the tumor marker according to Table 1. Examples of demethylation agents to be used in the context of the present invention are 5-aza-2'-deoxycytidine and 5-azacytidine, which reactivate genes inappropriately silenced by structural chromatin changes that involve DNA methylation and which can reverse these changes and, therefore, restore principal cellular pathways. This typically results in gene re-expression and reversion of some aspects of the transformed state. 5-azacytidine and 5-aza-2'-deoxycytidine typically inactivate DNA cytosine C5-methyltransferases through the formation of stable complexes between the 5-aza-2'-deoxycytidine residues in DNA and the enzyme, thereby mimicking a stable transition state intermediate when bound to the methyltransferase enzyme.

A further agent, which may be comprised in a pharmaceutical composition according to the present invention, either per se or in combination with 5-aza-2'-deoxycytidine and/or 5-azacytidine, is trichostatin A (TSA).

In a another aspect the present invention relates to a pharmaceutical composition for the treatment or prevention of a cancer disease associated with a progression from a less progressed cancer stage to a more progressed cancer stage, wherein said cancer disease implies the decreased (down-regulated) expression of a tumor marker or group of tumor markers as defined above, comprising at least one element selected from the group of: (a) a compound directly stimulating or modulating the activity of a tumor marker or group of tumor markers as mentioned herein above or according to Table 1, preferably an agonist of said tumor marker enzymatic activity;(b) a compound indirectly stimulating or modulating the activity of a tumor marker or group of tumor markers as mentioned herein above or according to Table 1; (c) a protein of a tumor marker or group of tumor markers as mentioned herein above or according to Table 1 or a biologically active equivalent thereof; (d) a nucleic acid encoding and expressing a protein of a tumor marker or group of tumor markers as mentioned herein above or according to Table 1 and (e) a miRNA inhibitor specific for a miRNA of a tumor marker or group of tumor markers as mentioned herein above or according to Table 1. Such pharmaceutical compostion preferably comprises elements as defined herein above.

In an preferred embodiment of the invention a pharmaceutical composition according to the present invention, e.g. as defined herein above, may further comprise additional compounds being active against cancer cells. Furthermore, in another embodiment of the invention the pharmaceutical composition may further comprise hormone-inhibitors, preferably anti-androgens or androgen antagonists like spironolactone, cyproterone acetate, flutamide, nilutamide, bicalutamide, ketoconazole, finasteride or dutasteride.

The skilled person is aware of the fact that also a situation in which a given tumor marker of the invention is up-regulated in a cancer diasease while in parallel another tumor marker of the invention is down-regulated. It is thus an aim of the present invention to provide also pharmaceutical compositions wherein the pharmaceutical composition contains any combination of such elements as laid out above, e.g. the compounds, proteins, dominant negative proteins, nucleic acids, miRNAs, siRNAs, antisense RNAs, aptamers, antibodies, peptidomimetics and small molecules, and wherein the composition contains said elements being capable of down-regulating at least one tumor marker according to Table 1 and up-regulating at least one other tumor marker according to Table 1. It is preferred to avoid conflicting and/or opposite functionalities or overlapping functional spectra of the elements of the pharmaceutical compositions as defined herein above, e.g. if the tumor markers have similar functionalities. Due to the different identity of the tumor marker, in such situations the use of highly specific elements like antibodies, siRNAs etc. is envisaged.

In another preferred embodiment of the invention the pharmaceutical relates to the pharmaceutical compositions as laid out in the present description for the treatment of cancer, particularly prostate cancer.

In a particularly preferred embodiment of the present invention the above described pharmaceutical compositions is for the manufacture of a medicament for the treatment of cancer, particularly prostate cancer.

In a most preferred embodiment of the present invention the pharmaceutical compositions and medicaments of the present invention are capable of reducing the tumor volume of a given prostate carcinoma by at least 5%, 10%, 20%, 30%, 40%, 50%, 60%, 70%, 80%, 90%, or at least 95% when compared to an untreated control.

In a further embodiment the present invention also envisages screening procedures and methods for the identification of an aptamer specific for the expression product(s) or protein(s) of the tumor marker or group of tumor markers as mentioned herein above or according to Table 1, a compound directly stimulating or modulating the activity of the tumor marker according to Table 1, an agonist of the tumor marker or group of tumor markers as mentioned herein above or according to Table 1 enzymatic activity, a miRNA inhibitor specific for miRNA(s) of the tumor marker or group of tumor markers as mentioned herein above or according to Table 1, an antagomir, a demethylation agent specific for the tumor marker or group of tumor markers as mentioned herein above or according to Table 1, or a peptidomimetic specific for the tumor marker or group of tumor markers as mentioned herein above or according to Table 1. Such screening procedures may comprise the steps of (a) producing cells which express the tumor marker or group of tumor markers according to Table 1 as a polypeptide either as secreted protein or on the cell membrane or as intracellular component, (b) contacting the polypeptide produced in step (a) with a test sample potentially containing an interacting molecule, e.g. an aptamer specific for the protein(s) of the tumor marker or group of tumor markers as mentioned herein above or according to Table 1, a compound directly stimulating or modulating the activity of the tumor marker or group of tumor markers as mentioned herein above or according to Table 1, a compound directly stimulating or modulating the activity of the tumor marker or group of tumor markers as mentioned herein above or according to Table 1, an agonist of enzymatic activity of the tumor marker or group of tumor markers as mentioned herein above or according to Table 1, a peptidomimetic specific for the tumor marker or group of tumor markers as mentioned herein above or according to Table 1, and (c) indentifying an interacting molecule by observing binding and/or inhibition or modulation of the activity of the tumor marker or group of tumor markers as mentioned herein above or according to Table 1.

Alternatively, such screening procedures may comprise the steps of (a) contacting a test sample potentially containing a directly or indirectly interacting molecule, e.g. an aptamer specific for the transcript(s) of the tumor marker or group of tumor markers as mentioned herein above or according to Table 1, a miRNA inhibitor specific for miRNA(s) of the tumor marker or group of tumor markers as mentioned herein above or according to Table 1, an antagomir, a demethylation agent specific for the tumor marker or group of tumor markers as mentioned herein above or according to Table 1, a peptidomimetic specific for the tumor marker or group of tumor markers as mentioned herein above or according to Table 1 with one or more cells which express the tumor marker according to Table 1 as (a) transcript(s), (b) detecting the expression level of said sequence; and (c) indentifying an interacting molecule by observing binding or a modulation or reduction of the expression level of the tumor marker or group of tumor markers as mentioned herein above or according to Table 1.

The present invention also encompasses an aptamer specific for the expression product(s) or protein(s) of the tumor marker or group of tumor markers as mentioned herein above or according to Table 1, a compound directly stimulating or modulating the activity of the tumor marker or group of tumor markers as mentioned herein above or according to Table 1, an agonist of the enzymatic activity of the tumor marker or group of tumor markers as mentioned herein above or according to Table 1, a miRNA inhibitor specific for miRNA(s) of the tumor marker or group of tumor markers as mentioned herein above or according to Table 1, an antagomir, a demethylation agent specific for of the tumor marker or group of tumor markers as mentioned herein above or according to Table 1 and a peptidomimetic specific for the tumor marker or group of tumor markers as mentioned herein above or according to Table 1, obtainable or obtained by a screening procedure or method as described herein above.

In a further aspect the present invention relates to a pharmaceutical composition as defined herein above for the treatment or prevention of cancer.

Further, in yet another aspect, the present invention relates to the use of (a) a compound directly inhibiting the activity of a tumor marker or group of tumor markers as mentioned herein above or according to Table 1, preferably an antagonist of said tumor marker enzymatic activity; (b) a compound indirectly inhibiting the activity of a tumor marker or group of tumor markers as mentioned herein above or according to Table 1; (c) a dominant negative form of a protein of a tumor marker or group of tumor markers as mentioned herein above or according to Table 1 or a biologically active equivalent thereof; (d) a nucleic acid encoding and expressing a dominant negative form of a protein of a tumor marker or group of tumor markers as mentioned herein above or according to Table 1; (e) a miRNA specific for a tumor marker or group of tumor markers as mentioned herein above or according to Table 1; (f) an antisense molecule of a tumor marker or group of tumor markers as mentioned herein above or according to Table 1; (g) a siRNA specific for a tumor marker or group of tumor markers as mentioned herein above or according to Table 1; (h) an aptamer specific for the expression product of a tumor marker or group of tumor markers as mentioned herein above or according to Table 1 or for the protein of a tumor marker or group of tumor markers as mentioned herein above or according to Table 1; (i) a small molecule or peptidomimetic capable of specifically binding to the protein of a tumor marker or group of tumor markers as mentioned herein above or according to Table 1; and/or (j) an antibody specific for the protein of a tumor marker or group of tumor markers as mentioned herein above or according to Table 1 and/or an antibody variant specific for the protein of a tumor marker or group of tumor markers as mentioned herein above or according to Table 1 for the preparation of a pharmaceutical composition for the treatment or prevention of cancer, preferably for the treatment or prevention of a cancer disease associated with a progression from a less progressed cancer stage to a more progressed cancer stage.

Further, in yet another aspect, the present invention relates to the use of (a) a compound directly stimulating or modulating the activity of a tumor marker or group of tumor markers as mentioned herein above or according to Table 1, preferably an agonist of said tumor marker enzymatic activity;(b) a compound indirectly stimulating or modulating the activity of a tumor marker or group of tumor markers as mentioned herein above or according to Table 1; (c) a protein of a tumor marker or group of tumor markers as mentioned herein above or according to Table 1 or a biologically active equivalent thereof; (d) a nucleic acid encoding and expressing a protein of a tumor marker or group of tumor markers as mentioned herein above or according to Table 1 and (e) a miRNA inhibitor specific for a miRNA of a tumor marker or group of tumor markers as mentioned herein above or according to Table 1 for the preparation of a pharmaceutical composition for the treatment or prevention of cancer, preferably for the treatment or prevention of a cancer disease associated with a progression from a less progressed cancer stage to a more progressed cancer stage.

In another aspect the present invention relates to a method of treatment or prevention of cancer, in particular the treatment or prevention of a cancer disease associated with a progression from a less progressed cancer stage to a more progressed cancer stage, comprising the administration of (a) a compound directly inhibiting the activity of a tumor marker or group of tumor markers as mentioned herein above or according to Table 1, preferably an antagonist of said tumor marker enzymatic activity; (b) a compound indirectly inhibiting the activity of a tumor marker or group of tumor markers as mentioned herein above or according to Table 1; (c) a dominant negative form of a protein of a tumor marker or group of tumor markers as mentioned herein above or according to Table 1 or a biologically active equivalent thereof; (d) a nucleic acid encoding and expressing a dominant negative form of a protein of a tumor marker or group of tumor markers as mentioned herein above or according to Table 1; (e) a miRNA specific for a tumor marker or group of tumor markers as mentioned herein above or according to Table 1; (f) an antisense molecule of a tumor marker or group of tumor markers as mentioned herein above or according to Table 1; (g) a siRNA specific for a tumor marker or group of tumor markers as mentioned herein above or according to Table 1; (h) an aptamer specific for the expression product of a tumor marker or group of tumor markers as mentioned herein above or according to Table 1 or for the protein of a tumor marker or group of tumor markers as mentioned herein above or according to Table 1; (i) a small molecule or peptidomimetic capable of specifically binding to the protein of a tumor marker or group of tumor markers as mentioned herein above or according to Table 1; and/or (j) an antibody specific for the protein of a tumor marker or group of tumor markers as mentioned herein above or according to Table 1 and/or an antibody variant specific for the protein of a tumor marker or group of tumor markers as mentioned herein above or according to Table 1 to an individual, in particular to an individual suffering from cancer or being prognosticated to develop cancer.

In another aspect the present invention relates to a method of treatment or prevention of cancer, in particular the treatment or prevention of a cancer disease associated with a progression from a less progressed cancer stage to a more progressed cancer stage, comprising the administration of (a) a compound directly stimulating or modulating the activity of a tumor marker or group of tumor markers as mentioned herein above or according to Table 1, preferably an agonist of said tumor marker enzymatic activity;(b) a compound indirectly stimulating or modulating the activity of a tumor marker or group of tumor markers as mentioned herein above or according to Table 1; (c) a protein of a tumor marker or group of tumor markers as mentioned herein above or according to Table 1 or a biologically active equivalent thereof; (d) a nucleic acid encoding and expressing a protein of a tumor marker or group of tumor markers as mentioned herein above or according to Table 1 and (e) a miRNA inhibitor specific for a miRNA of a tumor marker or group of tumor markers as mentioned herein above or according to Table 1 to an individual, in particular to an individual suffering from cancer or being prognosticated to develop cancer.

A pharmaceutical composition according to the present invention may be administered to a patient, subject or individual with the help of various delivery systems known to the person skilled in the art, e.g., via encapsulation in liposomes, microparticles, microcapsules, recombinant cells capable of expressing the compound, receptor-mediated endocytosis, construction of a nucleic acid as part of a retroviral or other vector, etc. Methods of introduction may be topical, enteral or parenteral and may include intradermal, intramuscular, intraperitoneal, intravenous, subcutaneous, intranasal, inhalational, epidural, and oral routes. The composition may be administered by any convenient route, for example by infusion or bolus injection, by absorption through epithelial or mucocutaneous linings (e. g., oral mucosa, rectal and intestinal mucosa, etc.) or by inhalation and may be administered together with other biologically active agents. Administration can be systemic or local. A preferred method of local administration is by direct injection.

In another embodiment the pharmaceutical composition may be delivered directly to internal organs, body cavities and the like by use of imaging devices used to guide an injecting needle directly to the site of interest. The pharmaceutical composition may also be administered to disease sites at the time of surgical intervention. In yet another embodiment, the composition can be delivered in a controlled release system.

Preferably the pharmaceutical composition is in a form, which is suitable for oral, local or systemic administration. In a preferred embodiment the pharmaceutical composition is administered locally, orally or systemically.

In a further embodiment the pharmaceutical composition comprises a therapeutically effective amount of the ingredients of the pharmaceutical composition of the present invention as defined herein above and a pharmaceutically acceptable carrier. The term "pharmaceutically acceptable" means approved by a regulatory agency or other generally recognized pharmacopeia for use in animals, and more particularly in humans. The term "carrier" refers to a diluent, adjuvant, excipient, or vehicle with which the therapeutic is administered. Such a carrier is pharmaceutically acceptable, i.e. is non-toxic to a recipient at the dosage and concentration employed.

Generally, the ingredients are supplied either separately or mixed together in unit dosage form, for example, as a dry lyophilised powder or water free concentrate in a hermetically sealed container such as an ampoule or sachette indicating the quantity of active agent.

The pharmaceutical composition of the invention can be formulated as neutral or salt forms.

Preferably, the pharmaceutical composition may be administered directly or in combination with any suitable adjuvant known to the person skilled in the art. The composition of the present invention can be administered to an animal, preferably to a mammal. "Mammal" as used herein is intended to have the same meaning as commonly understood by one of ordinary skill in the art. Particularly, "mammal" encompasses human beings.

The term "administered" means administration of a therapeutically effective dose of the aforementioned composition. By "therapeutically effective amount" is meant a dose that produces the effects for which it is administered, preferably this effect is induction and enhancement of the tumor marker or group of tumor markers as mentioned herein above or according to Table 1. The exact dose will depend on the purpose of the treatment, and will be ascertainable by one skilled in the art using known techniques. As is known in the art and described above, adjustments for systemic versus localized delivery, age, body weight, general health, sex, diet, time of administration, drug interaction and the severity of the condition may be necessary, and will be ascertainable with routine experimentation by those skilled in the art.

The concentration of the active ingredients or compounds of a pharmaceutical composition according to the present invention may be further adjusted to the intended dosage regimen, the intended usage duration, the exact amount and ratio of all ingredients of the composition and further factors and parameter known to the person skilled in the art.

The active agents or compounds according to the present invention may be administered alone or in combination with other treatments. In a preferred embodiment the pharmaceutical composition of the present invention may be administered in combination with an anti-hormone treatment, e.g. an anti-androgen treatment.

The pharmaceutical composition of the present invention can also comprise any suitable preservative known to the person skilled in the art.

Furthermore, the preparations according to the invention may also comprise compounds, which have an antioxidative, free-radical scavenger, antierythematous, antiinflammatory or antiallergic action, in order to supplement or enhance their action.

In another preferred embodiment of the present invention active components of the pharmaceutical composition as defined herein above may be fused to a suitable carrier protein, e.g. to Ig Fc receptor proteins or polymeric Ig receptors. Preferably the protein(s) of the tumor marker or group of tumor markers as mentioned herein above or according to Table 1 or biologically active equivalents thereof as defined herein above may be provided as fusion proteins. The fusion partner may be provided at the N- or C-terminus.

If the pharmaceutical composition according to the present invention is to be administered in the form of a live cell or living therapeutic as defined herein above, transformed and prepared cells may be administered to a patient in any suitable form known to the person skilled in the art. Preferably living therapeutics may be administered in the form of a composition comprising a microorganism, e.g. a Lactobacillus as described above, in an amount between 10² to 10¹² cells, preferably 10³ to 10⁸ cells.

In a further preferred embodiment of the present invention the ratio between two or more ingredients in the pharmaceutical composition or medicament may be suitably adjusted according to the skilled person's knowledge.

Suitable assays may optionally be employed to help identify optimal ratios and/or dosage ranges for ingredients of pharmaceutical compositions of the present invention. The precise dose and the ratio between the ingredients of the pharmaceutical composition as defined herein above to be employed in the formulation will, inter alia, depend on the route of administration, and the exact type of disease or disorder, and should be decided according to the judgment of the practitioner and each patient's circumstances. Effective doses or ingredient ratios may be extrapolated from dose-response curves derived from in vitro or (animal) model test systems.

A typical dose can be, for example, in the range of 0.001 to 1000 µg; however, doses below or above this exemplary range are envisioned, especially considering the aforementioned factors.

In another aspect the present invention relates to a medical kit for the treatment or prevention of cancer, comprising at least one element selected from the group consisting of (a) a compound directly inhibiting the activity of a tumor marker or group of tumor markers as mentioned herein above or according to Table 1, preferably an antagonist of said tumor marker enzymatic activity; (b) a compound indirectly inhibiting the activity of a tumor marker or group of tumor markers as mentioned herein above or according to Table 1; (c) a dominant negative form of a protein of a tumor marker or group of tumor markers as mentioned herein above or according to Table 1 or a biologically active equivalent thereof; (d) a nucleic acid encoding and expressing a dominant negative form of a protein of a tumor marker or group of tumor markers as mentioned herein above or according to Table 1; (e) a miRNA specific for a tumor marker or group of tumor markers as mentioned herein above or according to Table 1; (f) an antisense molecule of a tumor marker or group of tumor markers as mentioned herein above or according to Table 1; (g) a siRNA specific for a tumor marker or group of tumor markers as mentioned herein above or according to Table 1; (h) an aptamer specific for the expression product of a tumor marker or group of tumor markers as mentioned herein above or according to Table 1 or for the protein of a tumor marker or group of tumor markers as mentioned herein above or according to Table 1; (i) a small molecule or peptidomimetic capable of specifically binding to the protein of a tumor marker or group of tumor markers as mentioned herein above or according to Table 1; and (j) an antibody specific for the protein of a tumor marker or group of tumor markers as mentioned herein above or according to Table 1 and/or an antibody variant specific for the protein of a tumor marker or group of tumor markers as mentioned herein above or according to Table 1; or at least one element selected from the group consisting of (a) a compound directly stimulating or modulating the activity of a tumor marker or group of tumor markers as mentioned herein above or according to Table 1, preferably an agonist of said tumor marker enzymatic activity;(b) a compound indirectly stimulating or modulating the activity of a tumor marker or group of tumor markers as mentioned herein above or according to Table 1; (c) a protein of a tumor marker or group of tumor markers as mentioned herein above or according to Table 1 or a biologically active equivalent thereof; (d) a nucleic acid encoding and expressing a protein of a tumor marker or group of tumor markers as mentioned herein above or according to Table 1 and (e) a miRNA inhibitor specific for a miRNA of a tumor marker or group of tumor markers as mentioned herein above or according to Table 1.

A medical kit that can be used in the context of the administration of the pharmaceutical composition as defined herein above. In particular, a kit according to the present invention may be used for the treatment or prevention of cancer.

The ingredients of a medical kit may, according to the present invention, be comprised in one or more containers or separate entities. They may preferably be formulated as pharmaceutical compositions or medicaments, more preferably they may be formulated as has been described herein above in the context of the pharmaceutical compositions of the present invention, e.g. they may comprise suitable pharmaceutical carriers etc. Particularly preferred are formulations for topical administration as mentioned herein above in the context of pharmaceutical compositions of the invention. The medical kit according to the present invention may optionally also comprise a documentation which indicates the use or employment of the medical kit and its components. Preferably, instructions comprised in the medical kit of the present invention may comprise recommended treatment options, dosage regimens etc. The medical kit may also comprise an instruction leaflet and/or may provide additional information on the use, dosage etc.

The medical kit of the present invention may be administered to a patient according to any suitable dosage regimen known to the person skilled in the art. The medical kit or kit components may preferably be given once a week, more preferably 2 times, 3 times, 4 times, 5 times or 6 times a week and most preferably daily and or 2 times a day or more often, unless otherwise indicated. During progression of the treatment the dosages may be given in much longer time intervals and in need can be given in much shorter time intervals, e.g., several times a day. In a preferred case a response to the treatment may be monitored using herein described methods and further methods known to those skilled in the art and dosages may accordingly be optimized, e.g., in time, amount and/or composition. Progress can be monitored by periodic assessment. It is also envisaged that the medical kit is employed in co-therapy approaches, i.e. in co-administration with other medicaments or drugs, for example antibiotics, antiviral medicaments or IgG or IgA immunoglobulins, anticancer medicaments and, preferably, anti-hormone medicaments, more preferably anti-androgens as mentioned herein above.

An especially preferred embodiment the present invention relates to any of the abovementioned tumor marker or group of tumor markers, the abovementioned compositions, the abovementioned methods, the abovementioned use, the abovementioned immunoassay, kits or the abovementioned pharmaceutical compositions etc., wherein said cancer is prostate cancer.

The term "prostate cancer" relates to a cancer of the prostate gland in the male reproductive system, which occurs when cells of the prostate mutate and begin to multiply out of control. A "prostate cancer" as used herein denotes a prostate cancer which can be classified according to the TNM classification by the International Union Against Cancer (UICC) into stages I to IV. Preferably, the term relates to the classification of prostate cancer pursuing the following T - Primary Prostate Tumor classification schedule:
TX. Primary tumor cannot be assessed
T0. No evidence of primary tumor
T1. Clinically inapparent tumor not palpable or visible by imaging
T1a. Tumor incidental histological finding in 5% or less of tissue resected
T1b. Tumor incidental histological finding in more than 5% of tissue resected
T1c. Tumor identified by needle biopsy (e.g., because of elevated PSA)
T2. Tumor confined within prostate (Tumor found in one or both lobes by needle biopsy, but not palpable or visible by imaging, is classified as T1c)
T2a. Tumor involves one half of one lobe or less
T2b. Tumor involves more than half of one lobe, but not both lobes
T2c. Tumor involves both lobes
T3. Tumor extends through the prostatic capsule (Invasion into the prostatic apex, or into (but not beyond) the prostate capsule, is not classified as T3, but as T2)
T3a. Extracapsular extension (unilateral or bilateral)
T3b. Tumor invades seminal vesicle(s)
T4. Tumor is fixed or invades adjacent structures other than seminal vesicles: bladder neck, external sphincter, rectum, levator muscles, or pelvic wall N1. Tumor invades regional lymph node(s)
M1a. Tumor invades non-regional lymph node(s)
M1b. Tumor invades bone(s)
M1c. Tumor invades other site(s)
G Histopathological Grading
GX. Grade cannot be assessed
G1. Well differentiated (slight anaplasia) (Gleason 2-4)
G2. Moderately differentiated (moderate anaplasia) (Gleason 5-6)
G3-4. Poorly differentiated/undifferentiated (marked anaplasia) (Gleason 7-10),
wherein T categories are physical examination, imaging, endoscopy, biopsy, and biochemical tests , N categories are physical examination and imaging tests and M categories are physical examination, imaging, skeletal studies, and biochemical tests, and wherein stages I to IV of prostate cancer correspond to the following scheme:

| | |
|---|---|
| Stage I: | T1a; N0; M0; G1 |
| Stage II: | T1a; N0; M0; G2, 3-4, or T1b. c; N0; M0; any G, or T1. T2; N0; M0; any G |
| Stage III | T3; N0; M0; any G |
| Stage IV | T4; N0; M0; any G, or Any T; N1; M0; any G, or Any T; Any N; M1; any G. |

A "prostate cancer" as used herein may further have one of the following grade of Gleason score: Grade 1 (the cancerous prostate closely resembles normal prostate tissue. The glands are small, well-formed, and closely packed), Grade 2 (the tissue still has well-formed glands, but they are larger and have more tissue between them), Grade 3 (the tissue still has recognizable glands, but the cells are darker. At high magnification some of these cells have left the glands and are beginning to invade the surrounding tissue), Grade 4 (the tissue has few recognizable glands. Many cells are invading the surrounding tissue), Grade 5 the tissue does not have recognizable glands. There are often just sheets of cells throughout the surrounding tissue). The grading typically follows the Gleason grading as established by the ASCP.

Typically, prostate cancer is further linked to an elevated level of prostate-specific antigen (PSA). In one embodiment of the present invention the term "prostate cancer" relates to a cancer showing PSA levels above 4.0. In another embodiment the term relates to cancer showing PSA levels above 2.0. The term "PSA level" refers to the concentration of PSA in the blood in ng/ml.

In a further preferred embodiment the present invention relates to any of the above mentioned tumor marker or group of tumor markers, the abovementioned compositions, the above mentioned methods, the above mentioned use, the above mentioned immunoassay, kits or the above mentioned pharmaceutical compositions etc., wherein said less progressed stage of prostate cancer is a local prostate cancer with insignificant disease parameters. The term "insignificant disease parameters", as used herein, refers to a clinical tumor stage ≤ T2 and a Gleason Score ≤ 6 and a Prostate Cancer Volume ≤ 0.5 ml,

The term "Prostate Cancer Volume" as used herein refers to a calculated or measured volume of a prostate tumor or prostate swelling, which may be based on the assessment of the extension of the tumor/swelling in three dimensions and a subsequent determination of the volume. For example, such an assessment may employ suitable morphometric reconstruction techniques, as known to the person skilled in the art. Preferably, such a measurement follows any suitable method of cancer volumetric calculations known to the person skilled in the art.

In a further preferred embodiment the present invention relates to any of the abovementioned tumor marker or group of tumor markers, the above mentioned compositions, the above mentioned methods, the above mentioned use, the above mentioned immunoassay, kits or the above mentioned pharmaceutical compositions etc., wherein said more progressed stage of prostate cancer is a local prostate cancer with significant disease parameters. The term "significant disease parameters", as used herein, refers to a clinical tumor stage > T2 or a Gleason Score > 6 or a Prostate Cancer Volume > 0.5 ml, alternatively to a clinical tumor stage > T2 and/or a Gleason Score > 6 and a Prostate Cancer Volume > 0.5 ml, alternatively to a clinical tumor stage > T2 and a Gleason Score > 6 and/or a Prostate Cancer Volume > 0.5 ml, alternatively also to a clinical tumor stage > T2 and a Prostate Cancer Volume > 0.5 ml and a Gleason Score > 6.

In a further preferred embodiment the present invention relates to any of the above mentioned tumor marker or group of tumor markers, the above mentioned compositions, the above mentioned methods, the above mentioned use, the above mentioned immunoassay, kits or the above mentioned pharmaceutical compositions etc., wherein said less progressed stage of prostate cancer is a local prostate cancer with insignificant disease parameters, as defined herein above and wherein said more progressed stage of prostate cancer is a local prostate cancer with significant disease parameters, as defined herein above.

In a further preferred embodiment the present invention relates to any of the above mentioned tumor marker or group of tumor markers, the abovementioned compositions, the above mentioned methods, the above mentioned use, the above mentioned immunoassay, kits or the above mentioned pharmaceutical compositions etc., wherein said less progressed stage of prostate cancer is a local prostate cancer with insignificant disease parameters and wherein said more progressed stage of prostate cancer is a local prostate cancer with significant disease parameters.

A further, particularly preferred embodiment the present invention relates to any of the abovementioned tumor marker or group of tumor markers, the abovementioned compositions, the abovementioned methods, the abovementioned use, the abovementioned immunoassay, kits or the abovementioned pharmaceutical compositions etc., wherein said less progressed stage of prostate cancer has a Gleason score ≤6.

A further, particularly preferred embodiment the present invention relates to any of the abovementioned tumor marker or group of tumor markers, the abovementioned compositions, the abovementioned methods, the abovementioned use, the abovementioned immunoassay, kits or the abovementioned pharmaceutical compositions etc., wherein said less progressed stage of prostate cancer is of stage ≤ T2 (UICC 2002 classification).

A further, particularly preferred embodiment the present invention relates to any of the above mentioned tumor marker or group of tumor markers, the abovementioned compositions, the above mentioned methods, the above mentioned use, the above mentioned immunoassay, kits or the above mentioned pharmaceutical compositions etc., wherein said less progressed stage of prostate cancer is of stage ≤ T2 (UICC 2002 classification) and has a Gleason score ≤ 6.

A further, particularly preferred embodiment the present invention relates to any of the above mentioned tumor marker or group of tumor markers, the above mentioned compositions, the above mentioned methods, the abovementioned use, the abovementioned immunoassay, kits or the abovementioned pharmaceutical compositions etc., wherein said more progressed stage of prostate cancer has a Gleason score ≥7.

A further, particularly preferred embodiment the present invention relates to any of the above mentioned tumor marker or group of tumor markers, the abovementioned compositions, the above mentioned methods, the abovementioned use, the above mentioned immunoassay, kits or the above mentioned pharmaceutical compositions etc., wherein said more progressed stage of prostate cancer is of stage > T2 (UICC 2002 classification) as defined herein above.

In specific embodiments of the present invention the less progressed stage of prostate cancer may imply a Gleason score of 1, 2, 3, 4, 5, or 6, preferably 6. In further specific embodiments of the present invention the less progressed stage of prostate cancer may imply a stage of T1, T1a, T1b, T1c, T2, T2, T2a, T2b or T2c, preferably T1c or T2 (UICC 2002 classification).

In further specific embodiments of the present invention the less progressed stage of prostate cancer may imply a Gleason score of 1, 2, 3, 4, 5, or 6, preferably 6 and a stage of T1, T1a, T1b, T1c, T2, T2, T2a, T2b or T2c, preferably T1c or T2 (UICC 2002 classification).

In further specific embodiments of the present invention the more progressed stage of prostate cancer may imply a Gleason score of 7, 8, 9 or 10, preferably 7. In further specific embodiments of the present invention the more progressed stage of prostate cancer may imply a stage of T3, T3a, T3b or T4, preferably T3 (UICC 2002 classification).

In specific embodiments of the present invention the more progressed stage of prostate cancer may imply a Gleason score of 7, 8, 9 or 10, preferably 7 and a stage of T2, T2a, T2b, T2c, preferably T2 (UICC 2002 classification).

In specific embodiments of the present invention the more progressed stage of prostate cancer may imply a Gleason score of 5 or 6, preferably 6 and a stage of T3, T3a, T3b, T3c, preferably T3 (UICC 2002 classification).

The most preferred embodiment of the present invention relates to any of the above mentioned tumor marker or group of tumor markers, the above mentioned compositions, the above mentioned methods, the abovementioned use, the abovementioned immunoassay, kits or the above mentioned pharmaceutical compositions etc, wherein said less progressed stage of prostate cancer is of stage ≤ T2 (UICC 2002 classification) as defined herein above and has a Gleason score ≤6, and said more progressed stage of prostate cancer is of stage >T2 (UICC 2002 classification), as defined herein above and/or has a Gleason score ≥6.

Another most preferred embodiment of the present invention relates to any of the above mentioned tumor marker or group of tumor markers, the above mentioned compositions, the above mentioned methods, the abovementioned use, the abovementioned immunoassay, kits or the above mentioned pharmaceutical compositions etc, wherein said less progressed stage of prostate cancer is of stage ≤ T2 (UICC 2002 classification) as defined herein above and has a Gleason score ≤6, and has a tumor volume < 0.5 ml and said more progressed stage of prostate cancer is of stage >T2 (UICC 2002 classification), as defined herein above and/or has a Gleason score ≥6 and/or has a tumor volume of > 0.5 ml.

The following examples and figures are provided for illustrative purposes. It is thus understood that the example and figures are not to be construed as limiting. The skilled person in the art will clearly be able to envisage further modifications of the principles laid out herein.

### EXAMPLES

### Example 1 -Preparation of samples

### Clinical human plasma samples

The clinical samples were human plasma drawn and prepared under standardized conditions. Prepared plasma samples were stored in aliquots at -80°C until use.

The characteristics of the clinical samples were chosen such that they would support as much as possible the identification of diagnostic and prognostic biomarkers.

Four groups of unselected men between ages 50-70 had been selected: 1) controls (healthy), 2) men with a benign prostate disease, 3&4) men with localised prostate cancers, and 5) men with advanced prostate cancers. Group 1 men were selected based on very low PSA values (<0.5 ng/ml), presumably healthy (i.e., no cancer). Group 2 men were selected based on PSA levels >3 ng/ml, but negative biopsies ((i.e., presumably men with a benign prostate disease). Group 3 men were selected based on PSA levels >3 ng/ml and cancer positive biopsy and pre- and post-treatment stages T2 or less, and Gleason scores 6 or less. Group 4 men were selected based on PSA levels >3 ng/ml and cancer positive biopsy and pre-treatment stages T2 or less, post-treatment stages ≥T2, Gleason scores ≥7. Group 5 men were selected on signs of clinical advanced cancer disease (clinical stage >T3).

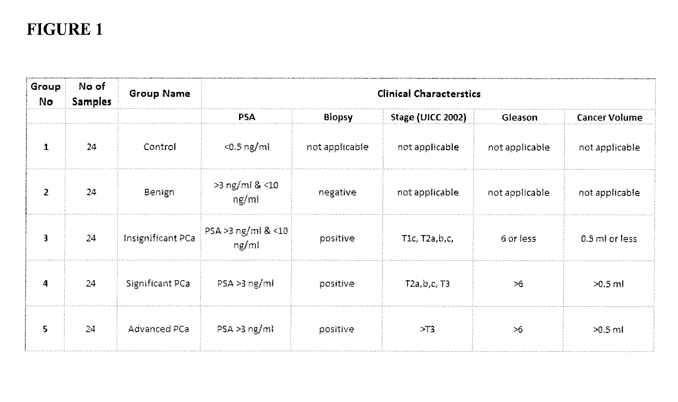

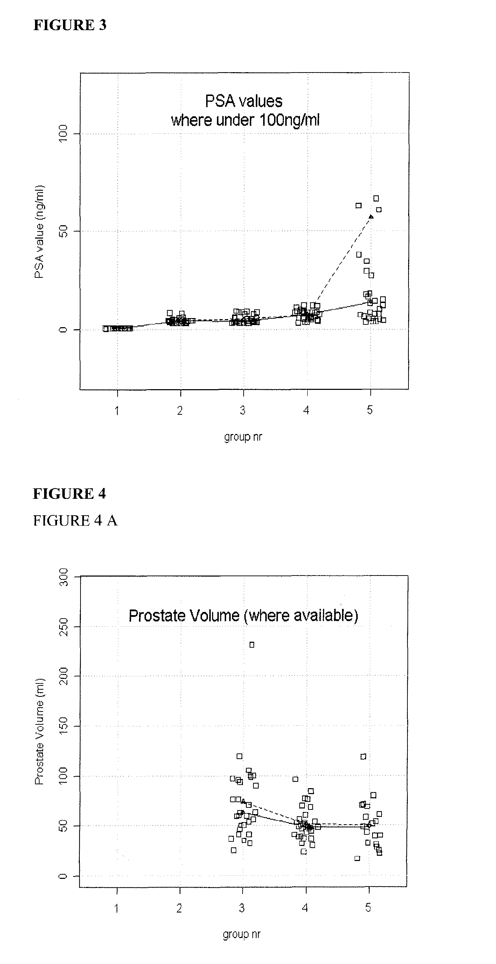

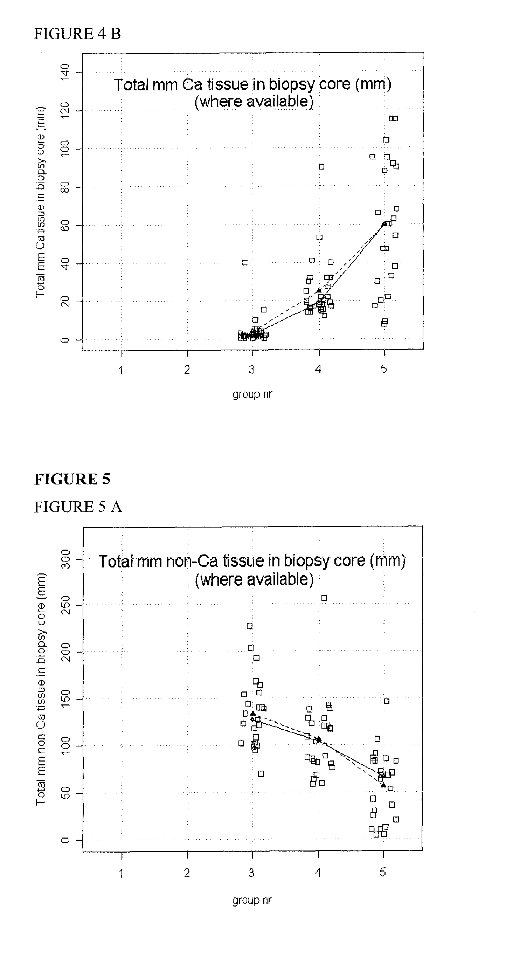

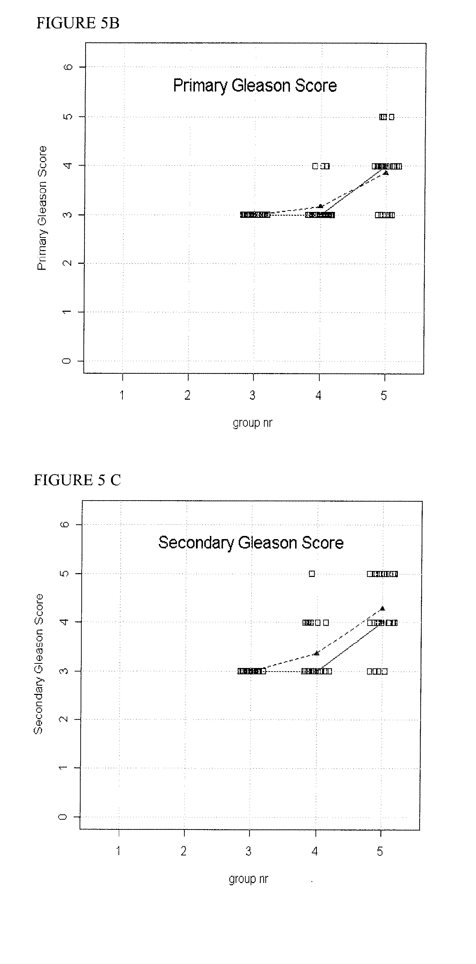

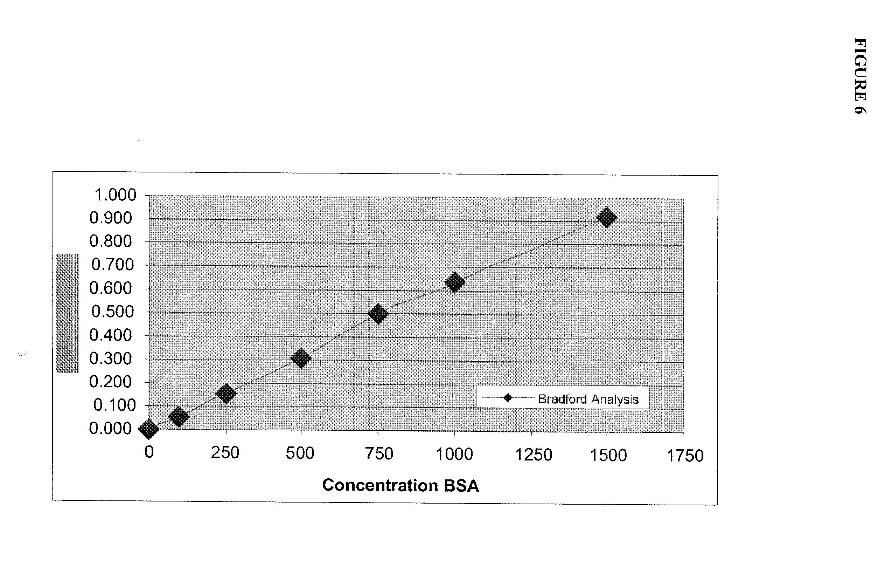

The Figures 1 to 6 summarize the characteristics of clinical and diagnostic parameters of the plasma samples

### Example 2 - Quantifying samples

Samples were retrieved from the -80°C freezer, thawed quickly and put on ice. 2 µl of sample were diluted with 98 µl of Milli-Q (dilution factor 50). The sample dilution was mixed and centrifuged. 300 µl of Bradford Reagent was pipetted into a new vial. 10 µl of the sample dilution were added to the 300 µl of Bradford reagent. After20 minutes the samples were measured. The protein concentration was approximately 1.5 mg/ml, so samples needed to be diluted 70 times. The samples were pooled by adding 68 µl of each of the 15 samples to one tube. The samples were mixed and spun (end volume was 1020 µl) and this procedure was repeated for pools 2-8. Finally, samples were stored at -80°C.

### Bradford Analysis

A 9000 µg/ml BSA Stock Solution was obtained by dissolving 10.87 mg of BSA (Bovine Serum Albumin) in 1.20777 ml H₂O.

The following standards were prepared from this BSA stock solution:

| Conc.BSA | Dilution | Amount of the BSA | Amount of | Total Volume |
|---|---|---|---|---|
| (µg/ml) | factor | Stock Solution (µl) | H₂O (µl) | (µl) |
| 100 | 90 | 10 | 890 | 900 |
| 250 | 36 | 25 | 875 | 900 |
| 500 | 18 | 50 | 850 | 900 |
| 750 | 12 | 75 | 825 | 900 |
| 1000 | 9 | 100 | 800 | 900 |
| 1500 | 6 | 150 | 750 | 900 |

| | | | | |
|---|---|---|---|---|
| 10 µl of Standard were mixed with 300 µl of Bradford Reagent. | | | | |

The standards were measured after 20 minutes of incubation.

| Concentration BSA (µg/ml) Absorbance at 595 nm rc = 0.9972 | |
|---|---|
| 0 | 0.000 |
| 100 | 0.057 |
| 250 | 0.154 |
| 500 | 0.306 |
| 750 | 0.501 |
| 1000 | 0.640 |
| 1500 | 0.914 |

All samples from all pools were measured individually.

### Example 3 - Proteomics Workflows

A total of one hundred and twenty (120) serum samples from 5 groups (healthy controls, benign disease, low-grade cancer, locally advanced cancer, and metastatic cancer, see above) were profiled on ProtoArray® Human Protein Microarrays v4.1 (Invitrogen Inc) containing more than 8,000 human proteins. Sera were profiled at a 1:500 dilution, utilizing one ProtoArray® Human Protein Microarray per sample. Two lots of arrays were used in this assay, and a negative control assay for each of the two lots was run in parallel with the samples as described below. Samples from each of the five groups were divided evenly between the two lots of arrays used in this study (lots HA20174 and HA20179).

### Assay Controls

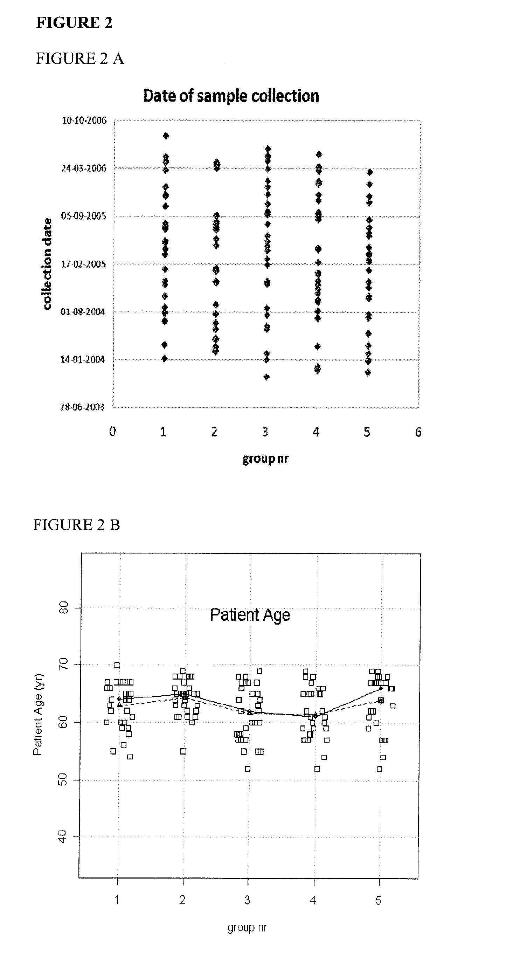

In the negative control assays, a ProtoArray® Human Protein Microarray from each of the two lots used in this service was treated in an identical manner to the experimental assays, except that it was incubated with buffer containing no serum prior to incubation with the Alexa Fluor®647-anti-human IgG detection reagent. A small percentage (less than 0.5%) of the proteins exhibited significant signals in the negative control assay due to interaction with the detection reagent and were eliminated from the analysis. Figure 2 shows a representative negative control image displaying the regular pattern of control proteins spotted in one subarray. The purpose of these control proteins is to provide reference points for data acquisition and analysis. The Alexa Fluor® conjugated antibody (boxed in white) allows proper alignment of the spotfinding software for data acquisition. The anti-biotin antibody (boxed in green) serves as a control for another ProtoArray® application, but is recognized by the anti-human IgG antibody used for detection in this assay. Each ProtoArray® subarray contains a gradient of human IgG (boxed in red), which serves as a control for proper performance of the detection reagent. Anti-human IgG antibody is also spotted as a gradient in every subarray. This antibody binds to IgG present in the serum sample and serves as a control for proper assay performance.

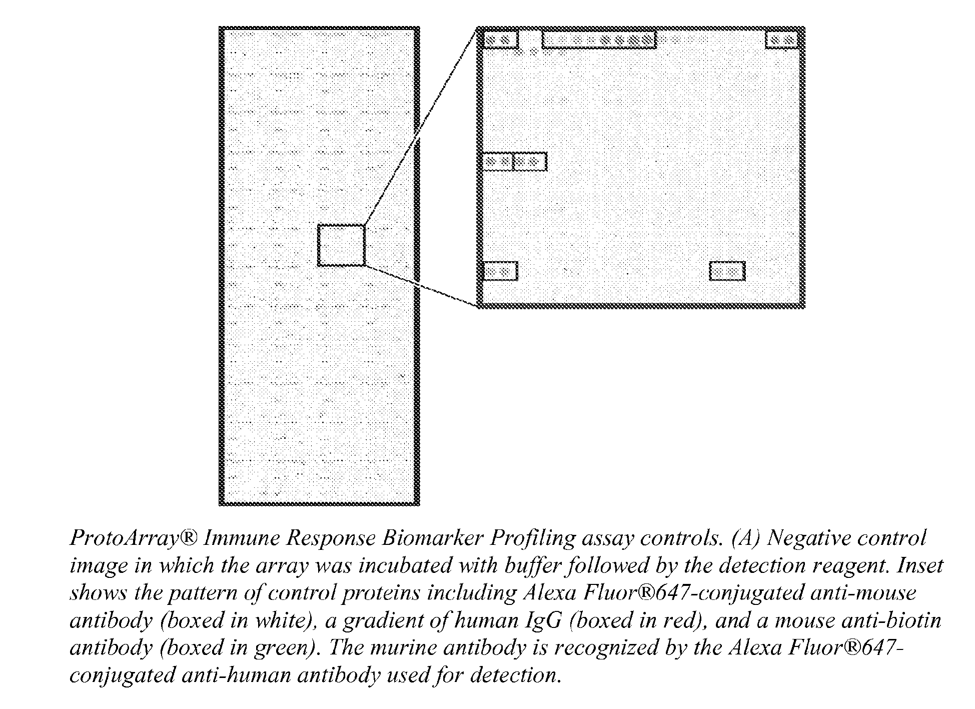

ProtoArray® Immune Response Biomarker Profiling assay controls. (A) Negative control
image in which the array was incubated with buffer followed by the detection reagent. Inset
shows the pattern of control proteins including Alexa Fluor®647-conjugated anti-mouse
antibody (boxed in white), a gradient of human IgG (boxed in red), and a mouse anti-biotin
antibody (boxed in green). The murine antibody is recognized by the Alexa Fluor®647-
conjugated anti-human antibody used for detection.

### Assay Performance

In this study, a total of one hundred and twenty (120) serum samples divided into five groups were profiled on ProtoArray® Human Protein Microarrays v4.1. Array images showed good signal-to background levels in all samples tested, although elevated background was seen for samples 3, 27, and 77. As shown above, the control features on the array serve as an indicator of assay performance; however, data quality can also be influenced by the scanning and data acquisition steps. To optimize this parameter, scanner settings were selected such that maximal signals on the array were sub-saturated, thus ensuring that the full dynamic range of the scanner was utilized. Maximizing the dynamic range of the scanner without increasing background to unacceptable levels is also critical. As illustrated in Table 1, the maximum group average background signal observed across the five groups was 437 relative fluorescence units (RFU) in group 2, with the cumulative average background across all samples equal to 419. Maximal signals were observed at >65,000 RFU for all of the samples, indicating a dynamic range of greater than 2 logs. Together, these data indicate that the experiments and data acquisition were performed under optimal conditions.

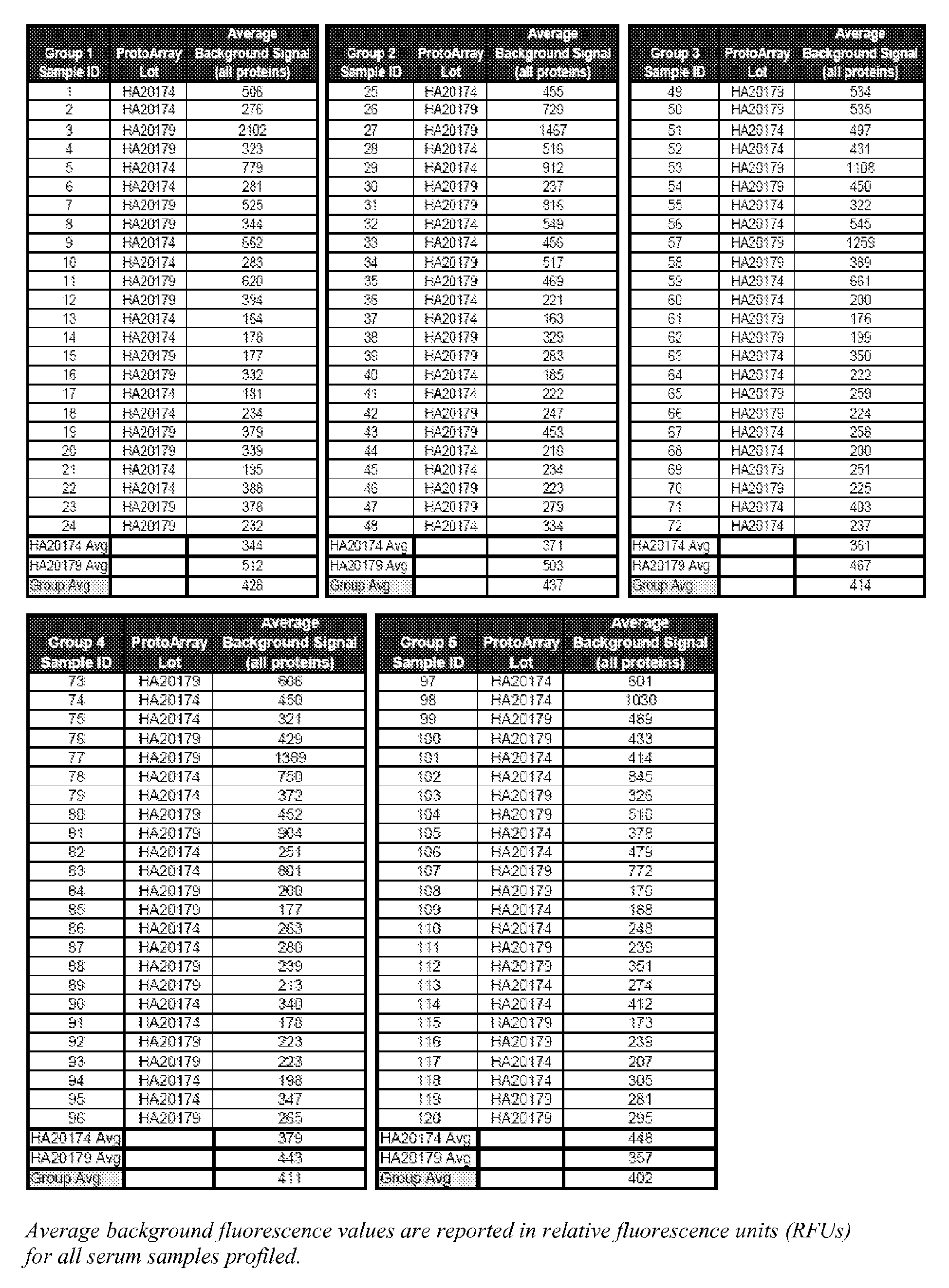

Some samples profiled in this study exhibited globally low signal intensity across the array. One of the control protein features present on the array is anti-human IgG, printed in every subarray as a gradient. Signals arising from the anti-human IgG gradient are used to identify proper scanning parameters, such that the signals at the highest concentration of anti-human IgG are just below saturation. Table 2 shows the mean signals for the spots corresponding to the highest concentration of anti-human IgG on each array. Arrays profiled with samples 46, 60, 61, 62, 84, 108, and 109 had mean signal intensities that were greater than two standard deviations below the mean for all arrays (highlighted red). The low signals may be the result of lower IgG levels or differences in immunoglobulin binding proteins in the corresponding samples. To ensure the use of the highest quality data set, those samples highlighted red in Table 2 were excluded from the analysis and candidate autoantigen tables in this report.

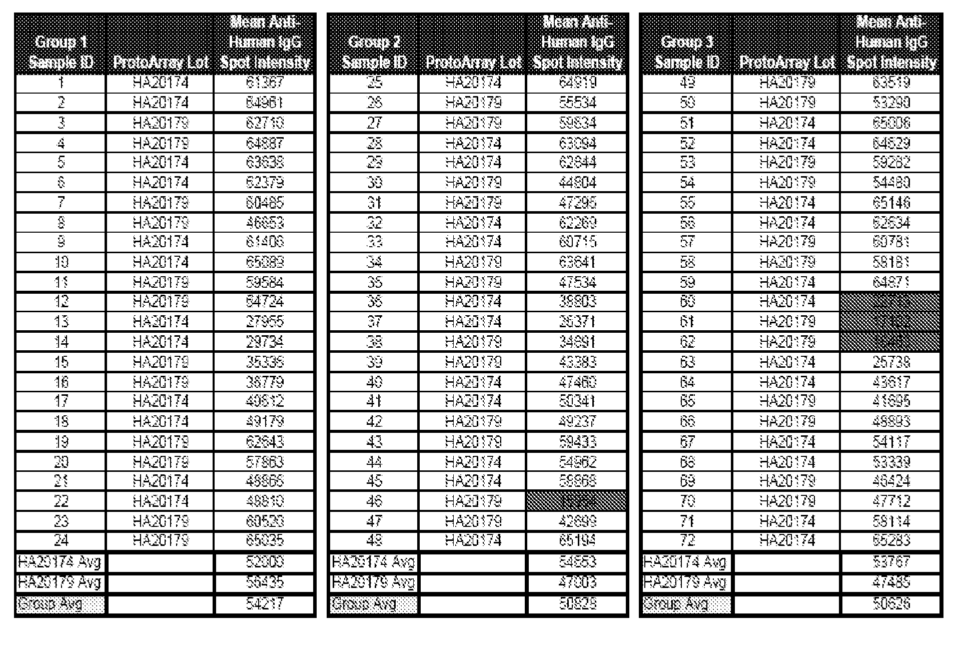

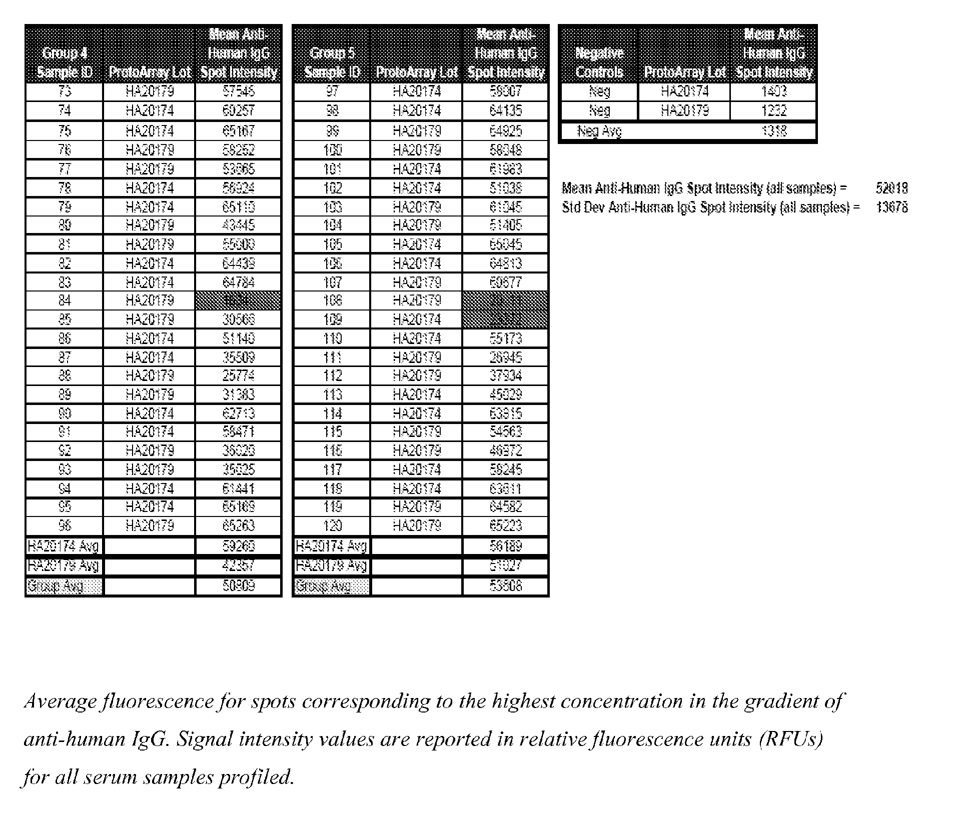

### Example 4 - Data analysis and scoring

### Data analysis

The array data were analyzed as follows. The raw results (fluorescence from duplex, neighboring spots) were converted to semi-ready data by averaging the intensities of the two spots. The resulting file contained 8302 rows for the different spotted proteins and 113 columns for the samples that had passed the quality control (described before) . As a final step, all arrays were normalized to have the same median intensity. We found this an essential step as the median intensities of the arrays varied by more than a factor of two in either direction before normalization.

In order to find the most relevant proteins with respect to the clinical question, the proteins were ranked according to their p-value from a one-sided Wilcoxon test comparing groups 3 and 4 (signal in 4 greater than in 3).. For visual inspection, we plotted for each protein the normalized data of all patients, ordered by group. See fig. XX; the blue and green line indicate the mean and median normalized intensity per patient group. The top-left number indicates the rank of the protein according to the Wilcoxon test, with number one the protein with the lowest P-value. The PSA values measured clinically for each patient were added as a separate row to the data as a benchmark, and we see PSA (gene name KLK3) appear with rank 42.

To investigate which marker proteins are statistically significant, we compared the distribution of the P-values for the individual proteins with the average distribution of 1000 randomized sets. See the figure below, we find experimentally (black) more low P-values than in the average randomized set (blue). The number we find is larger than the largest number we find in 90% of the random sets (red line, 90% quantile). We see that roughly the top 500 proteins seem significant at the 90% level. The calculation was done in R with 1000 randomized sets (samples from groups 3 and 4 shuffled), and took around 10 hours to run on a Dell dual-core workstation (1.5 GB RAM, 2.4 GHz clock frequency).

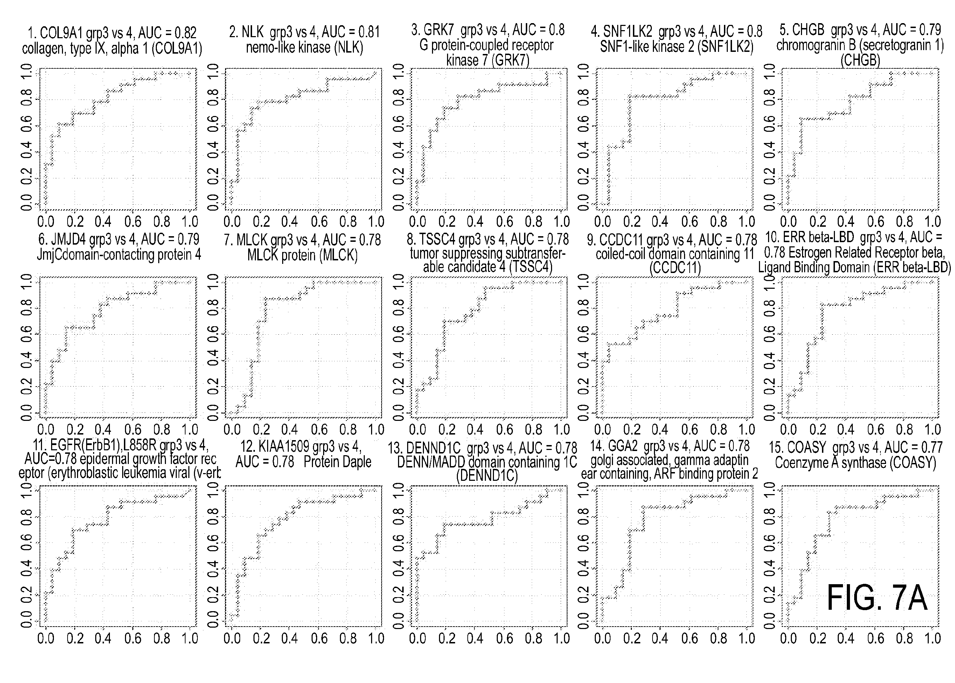

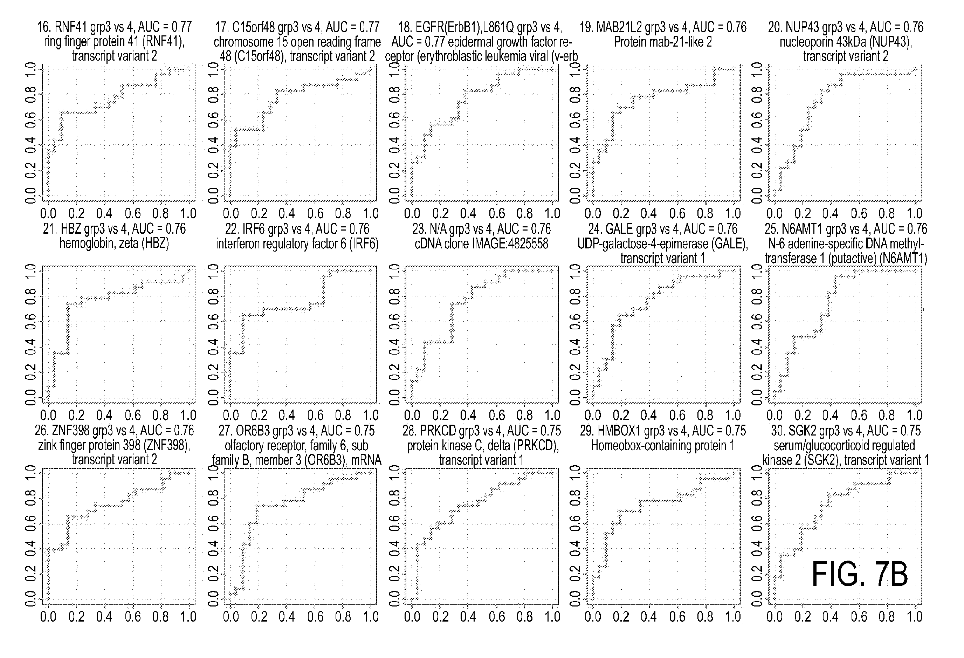

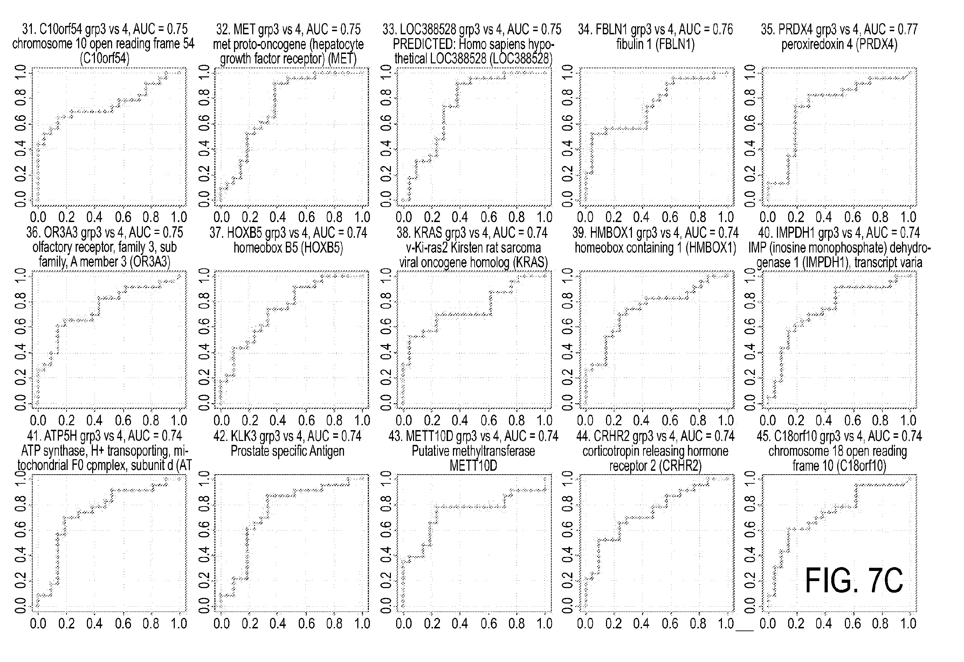

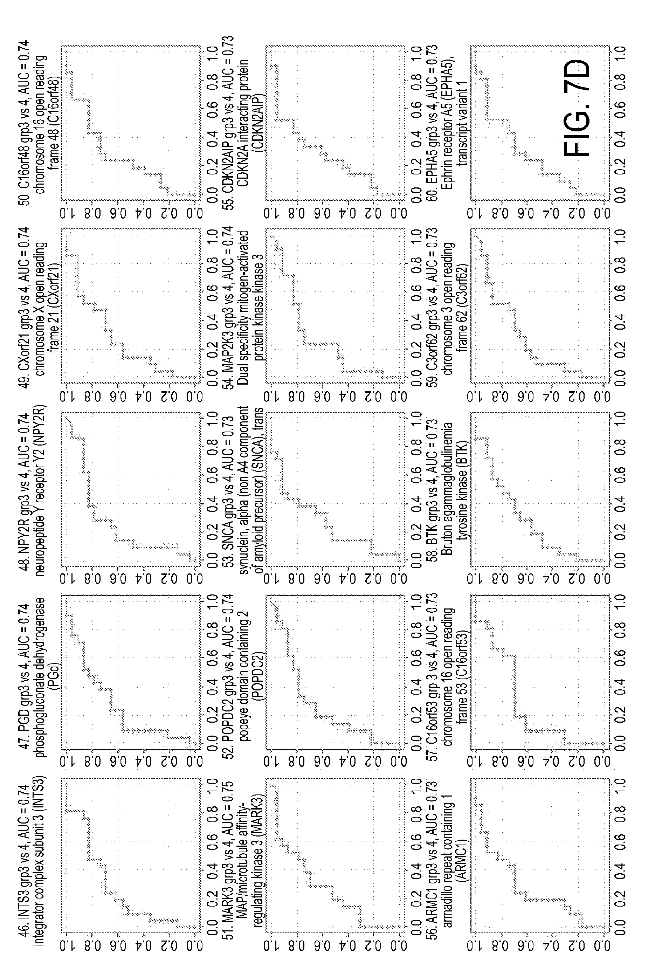

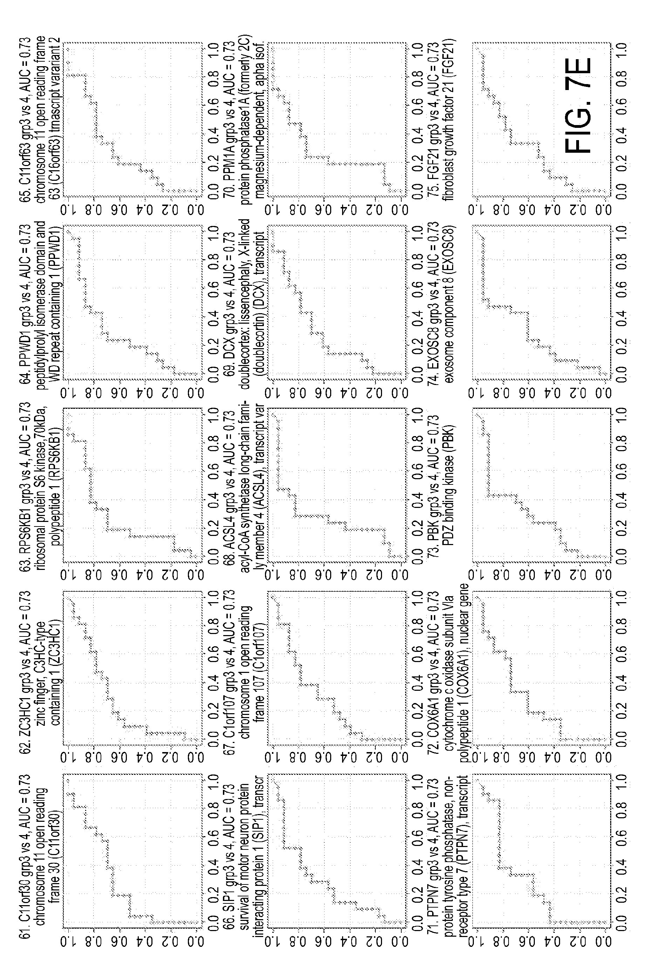

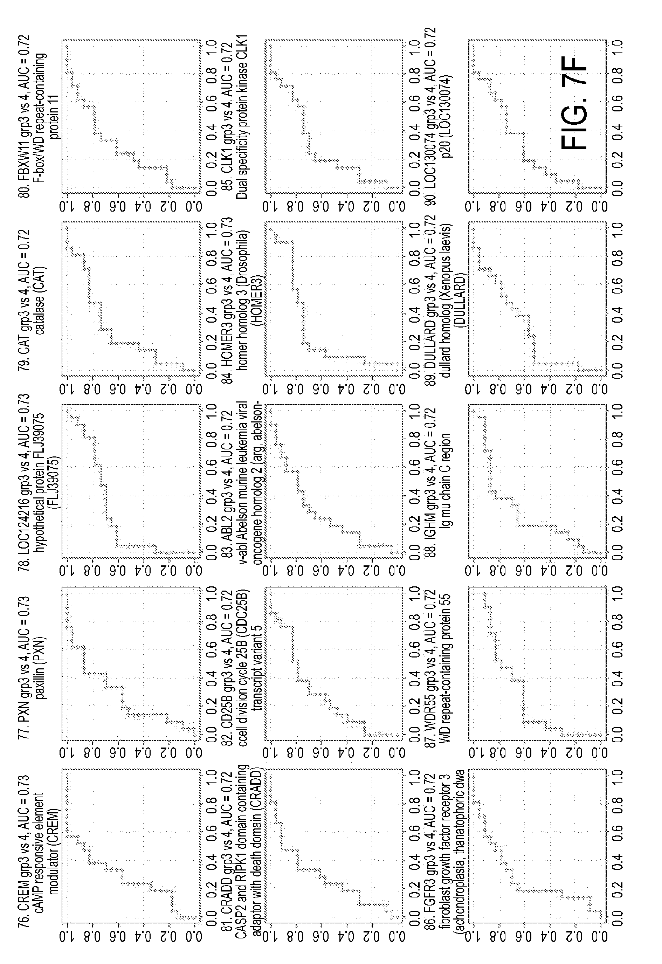

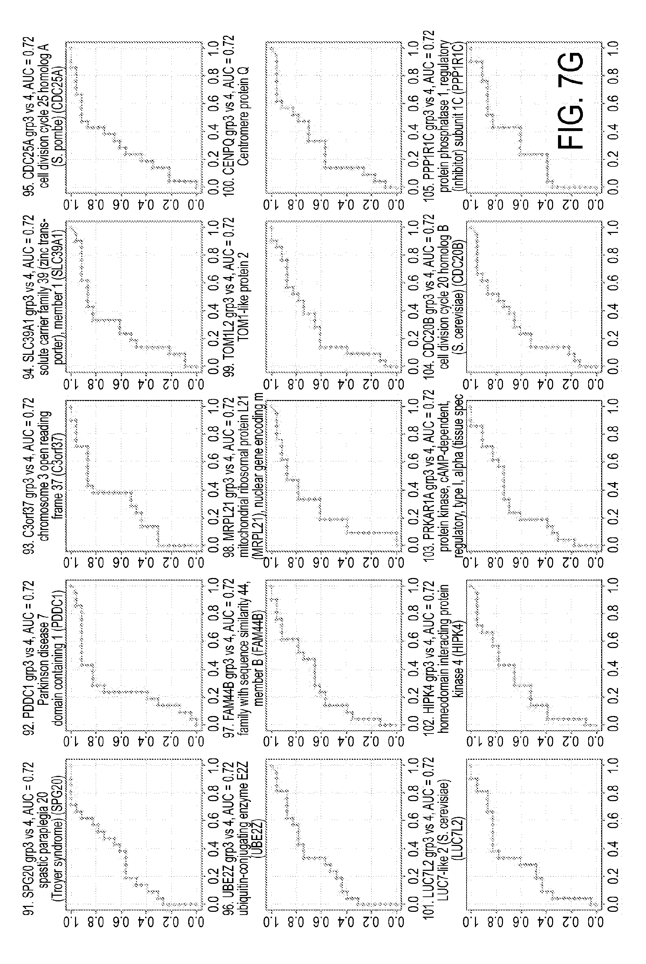

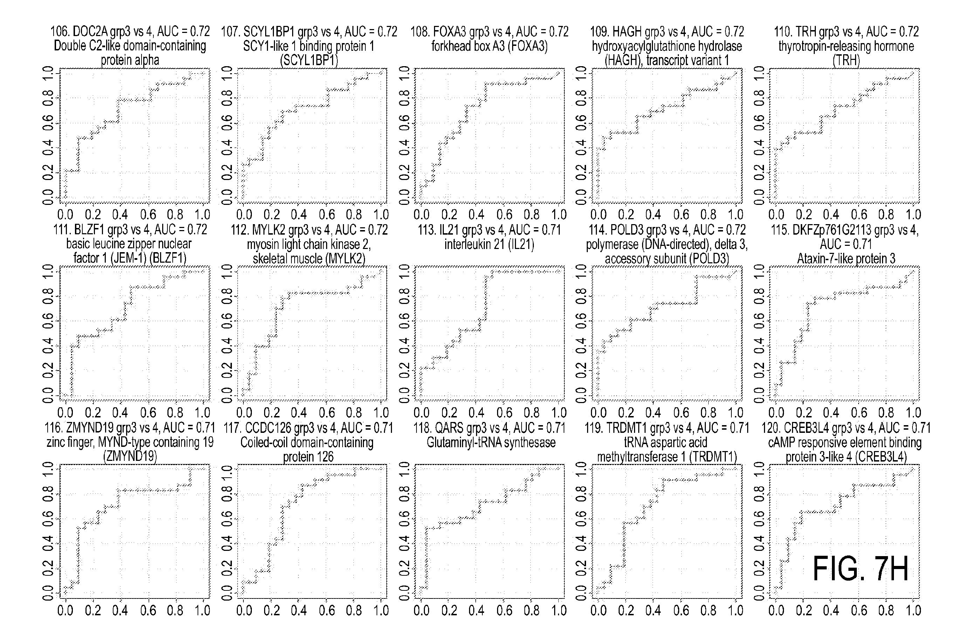

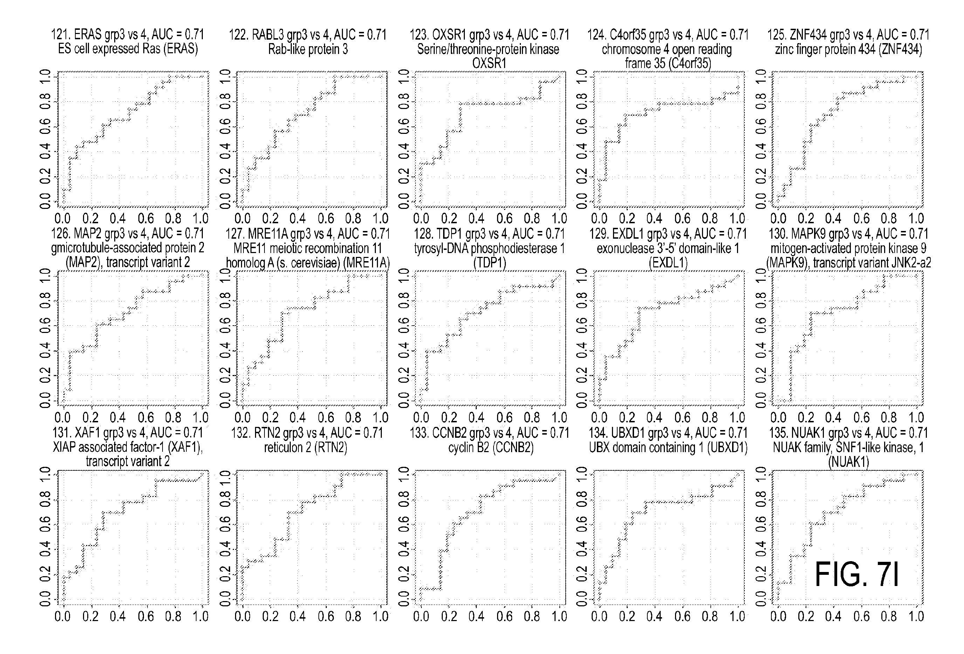

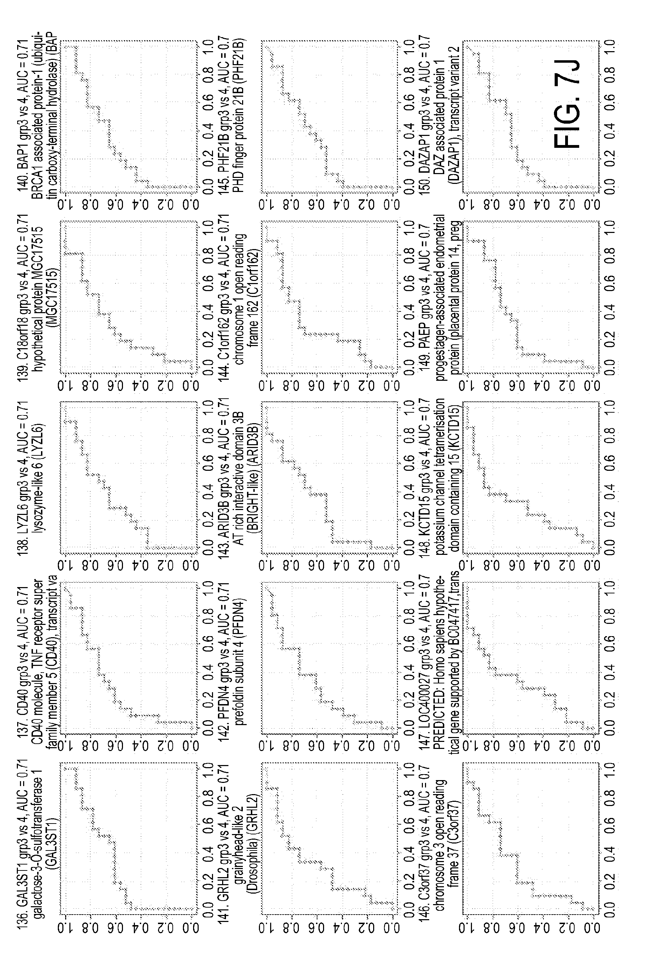

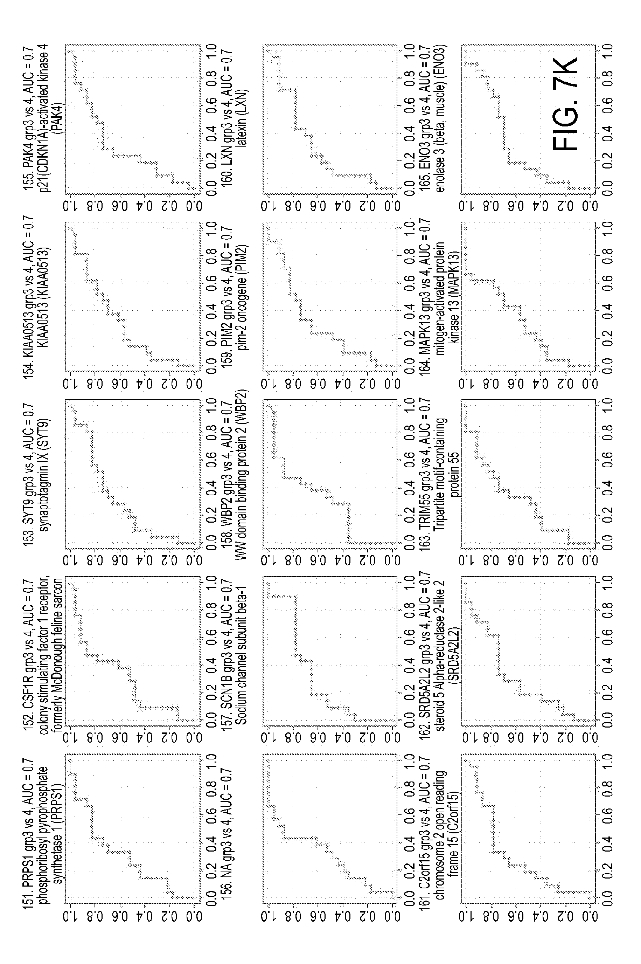

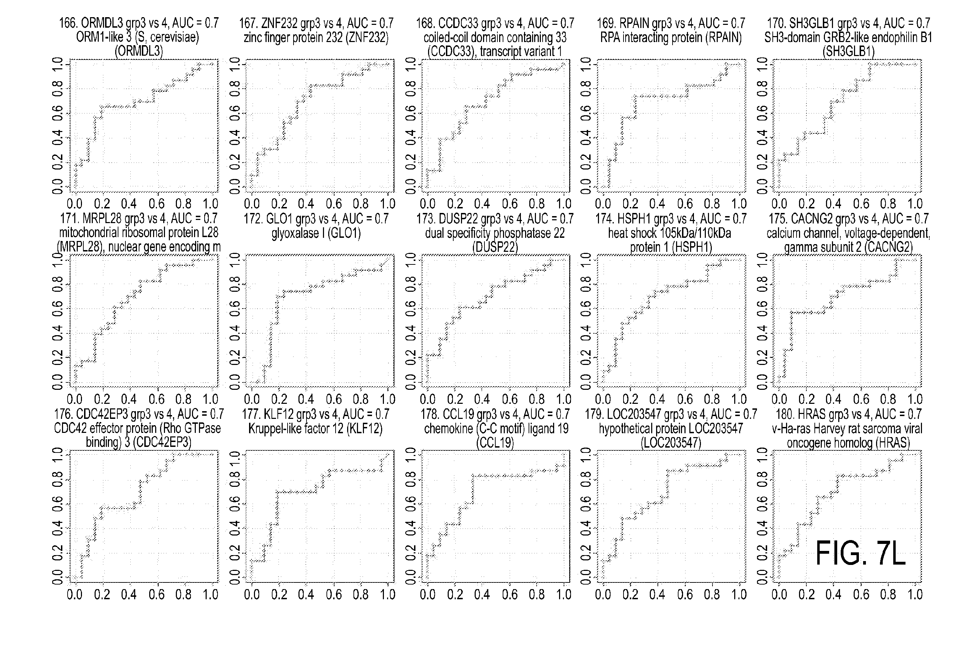

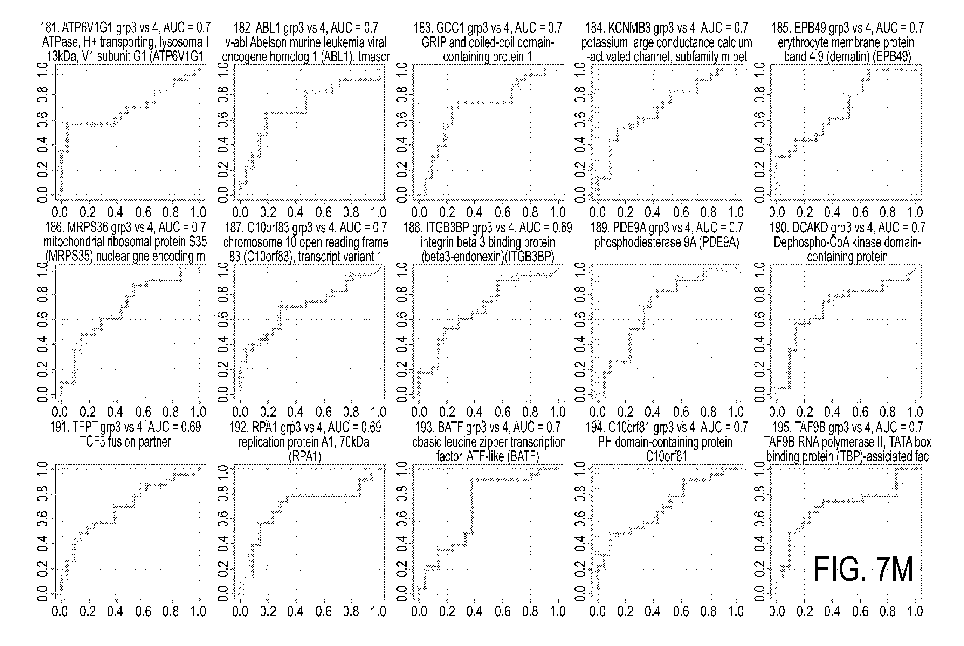

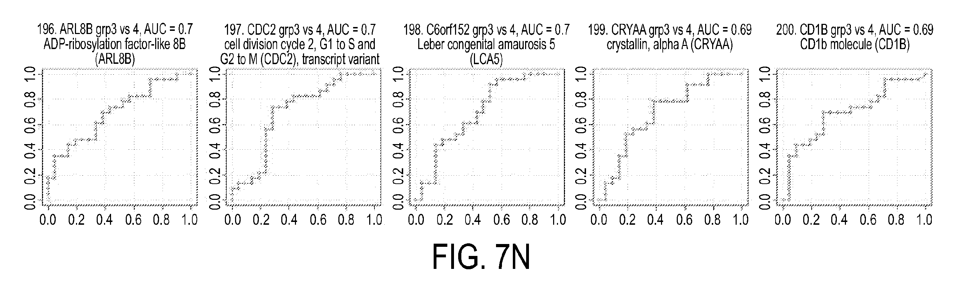

The quality of a clinical test is commonly described in terms of sensitivity and specificity; these depend on the threshold chosen. As the threshold goes down, the true positive rate increases, but so does the false positive rate. This is typically shown by means of a Receiver-Operating-Curve (ROC). The area under the curve (AUC) represents the accuracy of the test, averaged over all thresholds. We calculated the ROC's and their AUC's for all proteins, for discrimination between groups 3 and 4. Fig 7 shows the ROC's for the proteins with rank 1 to 200. With increasing rank, the AUC drops as expected.

### Example 5 - Identification of men with indolent, insignificant prostate cancer for stratification towards active surveillance regimes

Screening has resulted in a marked increase in the number of newly diagnosed prostate cancers, while it is unclear whether the early detection of these tumors reduces the prostate cancer mortality.

Up to 80% of men with PSA screen-detected prostate cancer are overdiagnosed, that is, their cancer would never have caused any symptoms. Overdiagnosis would matter less if treatment had no adverse effects.

It would be more acceptable to treat all cases, including those destined never to cause symptoms, if treatment was problem-free. However, while radical treatment for prostate cancer may or may not improve a man's longevity, it can certainly have a big impact on his lifestyle. Ideally, such intervention should be restricted to those who need it. Active surveillance aims to individualize the management of early prostate cancer by selecting only those men with significant cancers for curative treatment.

Today's criteria to select patients for active surveillance regimes are based on parameters that can be assessed on biopsy material like Gleason score as well as clinical staging information. Nevertheless, ca 30% of men in active surveillance programs will have progressive disease over time and need to be treated by traditional techniques.

The measurement of a marker, or a combination of markers from table 1 in a patient serum sample is used to select patients for active surveillance. The measurement of the marker, or the combinations of markers above a given threshold hereby indicates a >95%, >90%, >85%, >80%, >75%, >70%, >65%, or >60% chance that the identified prostate cancer is indolent, insignificant disease and is not going to progress over time. The measurement of the marker, or a combination of markers from table 1 is further used to monitor the potential progression of the prostate cancer. The marker(s) measurement is performed every 3, or every 6, or every 12 months after the patient has been moved into an avtive surveillance regime. If the marker, or marker combination measurement is going to pass a given threshold by 5%, 10%, 15%, 20%, 25%, or 30% further investigations or treatment will be started. In such a way active surveillance regimes will become much more effective as compared to today.

### Example 6 - Identification of men with significant. progressive prostate cancer for stratification towards radical treatment

Up to 80% of men with PSA screen-detected prostate cancer are overdiagnosed, that is, their cancer would never have caused any symptoms. Overdiagnosis would matter less if treatment had no adverse effects.

It would be more acceptable to treat all cases, including those destined never to cause symptoms, if treatment was problem-free. However, while radical treatment for prostate cancer may or may not improve a man's longevity, it can certainly have a big impact on his lifestyle. Ideally, such intervention should be restricted to those who need it.

Furthermore, by selecting men with insignificant disease for active surveillance (i.e., no primary radical intervention) typically around 30% of these patients will opt voluntarily for radical treatments to remove their prostate cancer as they do not want to continue the surveillance regimes often for psychological reasons although there are no obvious signs of disease progression. These men will suffer from treatment side effects most likely unnecessarily due to the fact that the prostate cancer has been detected by e.g. a screening approach in the first place. If those cancers would have been never identified many (f not all) of them would never had caused a matter of concern for the patient. Therefore, it would be preferred to only detect those cancers that are of significant nature whereas indolent, insignificant cancers would stay undetected.

The measurement of a marker, or a combination of markers from table 1 in a serum sample of a patient before a biopsy is perfromed is used to identify prostate cancers that have a very high probability to progress over time and therefore require radical intervention. The measurement of the marker, or the combinations of markers above a given threshold hereby indicates a >95%, >90%, >85%, >80%, >75%, >70%, >65%, or >60% chance that the identified prostate cancer is significant disease and is going to progress in the future. The measurement of the marker, or the combinations of markers below a given threshold indicates a >95%, >90%, >85%, >80%, >75%, >70%, >65%, or >60% chance that in case a prostate cancer is present it is insignificant disease and is not going to progress in the future.

The measurement of the marker, or a combination of markers from table 1 in a serum sample of a patient is further used to monitor the progression of a potential prostate cancer in case the initial measurement of the markers, or combination of markers indiciated the absence of significant disease. The marker(s) measurement is performed every 3, or every 6, or every 12 months after the patient has been moved into an avtive surveillance regime. If the marker, or marker combination measurement is going to pass a given threshold by 5%, 10%, 15%, 20%, 25%, or 30% further investigations or treatment will be started.

This workflow provides a very effective way of prostate cancer patient management towards treatment of selectively those patients that need intervention to save their live.

## Claims

1. A tumor marker or group of tumor markers associated with the progression of a cancer disease from a less progressed stage to a more progressed stage, wherein the expression of the tumor marker or group of tumor markers is modified when comparing the expression of the tumor marker or group of tumor markers in the less progressed stage to the expression in the more progressed stage, wherein said tumor marker or group of tumor markers comprises at least one tumor marker selected from Table 1.

2. The tumor marker or group of tumor markers of claim 1, wherein the expression of the marker(s) is increased (up-regulated) when comparing the expression in the more progressed stage to the expression in the less progressed stage, as indicated in section I) of Table 1.

3. The group of tumor markers of any one of claims 1 to 4, wherein said group comprises at least 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195 or all of the tumor markers of Table 1.

4. The group of tumor markers of any one of claims 1 to 3, wherein the p-value of the expression modification is 0.014 or lower.

5. The group of tumor markers of any one of claims 1 to 4, wherein the group comprises at least 5 tumor markers corresponding to tumor marker #1 to #5 of Table 1, at least 10 tumor markers corresponding to tumor marker #1 to #10 of Table 1, at least 15 tumor markers corresponding to tumor marker #1 to #15 of Table 1, at least 20 tumor markers corresponding to tumor marker #1 to #20 of Table 1, at least 25 tumor markers corresponding to tumor marker #1 to #25 of Table 1, at least 30 tumor markers corresponding to tumor marker #1 to #30 of Table 1, at least 35 tumor markers corresponding to tumor marker #1 to #35 of Table 1, at least 40 tumor markers corresponding to tumor marker #1 to #40 of Table 1, at least 45 tumor markers corresponding to tumor marker #1 to #45 of Table 1 or at least 50 tumor markers corresponding to tumor marker #1 to #50 of Table 1.

6. A composition for diagnosing, detecting, graduating, monitoring or prognosticating a cancer disease associated with a progression from a less progressed cancer stage to a more progressed cancer stage, or the progression from a less progressed cancer stage to a more progressed cancer stage, comprising a nucleic acid affinity ligand and/or a peptide affinity ligand for the expression product(s) or protein(s) of a tumor marker or a group of tumor markers as defined in any one of claims 1 to 5.

7. The composition of claim 6, wherein said nucleic acid affinity ligand or peptide affinity ligand is modified to function as an imaging contrast agent.

8. A method for detecting, diagnosing, monitoring or prognosticating a cancer disease associated with a progression from a less progressed cancer stage (e.g., indolent, insignificant disease) to a more progressed cancer stage (e.g., significant disease), or the progression from a less progressed cancer stage (e.g., indolent, insignificant disease) to a more progressed cancer stage (e.g., significant disease), comprising at least the step of determining the level of a tumor marker or a group of tumor markers as defined in any one of claims 1 to 5 in a sample.

9. The method of claim 8, wherein the determining step is accomplished by the measurement of nucleic acid or protein level(s) or by the determination of the biological activity of said tumor marker or group of tumor markers.

10. The method of claim 9, wherein said method comprises the additional step of comparing the measured nucleic acid or protein level(s) or the measured biological activity to a control level, wherein said control level is the expression level of the tumor marker or the group of tumor markers in one or more samples of a less progressed stage of the same cancer.

11. The method of claim 10, wherein said method is a method of graduating cancer, comprising the steps of
(a) determining the level of a tumor marker or a group of tumor markers as defined in any one of claims 1 to 5 in a sample by the measurement of nucleic acid or protein level(s) or by the determination of the biological activity of said tumor marker or group of tumor markers,
(b) comparing the measured nucleic acid or protein level(s) or the measured biological activity to a control level, wherein said control level is the expression level of the tumor marker or the group of tumor markers in one or more samples of a less progressed stage of the same cancer; and
(c) deciding on the stage or developmental status of cancer based on the results obtained in step (b).

12. Use of a tumor marker or a group of tumor markers as defined in any one of claims 1 to 5 as a marker for detecting, diagnosing, graduating, monitoring or prognosticating a cancer disease associated with a progression from a less progressed cancer stage to a more progressed cancer stage, or the progression from a less progressed cancer stage to a more progressed cancer stage.

13. An immunoassay for detecting, diagnosing, graduating, monitoring or prognosticating a cancer disease associated with a progression from a less progressed cancer stage (e.g., indolent, insignificant disease) to a more progressed cancer stage (e.g., significant disease), or for detecting, diagnosing, monitoring or prognosticating the progression from a less progressed cancer stage (e.g., indolent, insignificant disease) to a more progressed cancer stage (e.g., significant disease) comprising at least the steps
(a) testing in a sample obtained from an individual for the expression of a tumor marker or a group of tumor markers according to any one of claims 1 to 5;
(b) testing in a control sample for the expression of the same tumor marker or group of tumor markers as in (a);
(c) determining the difference in expression of the tumor marker or group of tumor markers of steps (a) and (b); and
(d) deciding on the presence, stage or risk of recurrence of cancer or the progression of cancer based on the results obtained in step (c),
wherein said testing steps are based on the use of an antibody specifically binding (a) protein(s) of a tumor marker or a group of tumor markers as defined in any one of claims 1 to 5.

14. A pharmaceutical composition for the treatment or prevention of a cancer disease associated with a progression from a less progressed cancer stage (e.g., indolent, insignificant disease) to a more progressed cancer stage (e.g., significant disease), wherein said cancer disease implies the increased (upregulated) expression of a tumor marker or group of tumor markers as defined in claim 2, comprising at least one element selected from the group of:
(a) a compound directly inhibiting the activity of a tumor marker as defined in claim 2, preferably an antagonist of said tumor marker enzymatic activity;
(b) a compound indirectly inhibiting the activity of a tumor marker as defined in claim 2;
(c) a dominant negative form of a protein of a tumor marker as defined in claim 2 or a biologically active equivalent thereof;
(d) a nucleic acid encoding and expressing a dominant negative form of a protein of a tumor marker as defined in claim 2;
(e) a miRNA specific for a tumor marker as defined in claim 2;
(f) an antisense molecule of a tumor marker as defined in claim 2;
(g) a siRNA specific for a tumor marker as defined in claim 2;
(h) an aptamer specific for the expression product of a tumor marker as defined in claim 2 or for the protein of a tumor marker as defined in claim 2;
(i) a small molecule or peptidomimetic capable of specifically binding to the protein of a tumor marker as defined in claim 2; and
(j) an antibody specific for the protein of a tumor marker as defined in claim 2 and/or an antibody variant specific for the protein of a tumor marker as defined in claim 2.

15. The tumor marker or group of tumor markers of any one of claims 1 to 5, the composition of claim 6 or 7, the method of any one of claims claim 8 to 11, the use of claim 12, the immunoassay of claim 13, or the pharmaceutical composition of claim 14, wherein said cancer is prostate cancer.

16. The tumor marker or group of tumor markers, composition, method, use, immunoassay or pharmaceutical composition of claim 15, wherein said less progressed stage of prostate cancer is of stage ≤ T2 (UICC 2002 classification), and Gleason score ≤ 6, and said more progressed stage of prostate cancer is of stage >T2 (UICC 2002 classification), and/or Gleason score >6.
